# EUROPEAN PATENT APPLICATION

(11) **EP 4 431 526 A1**
(43) Date of publication of application: **18.09.2024**
(21) Application number: 23162489.1
(22) Date of filing: 16.03.2023
(51) Int. Cl.: C07K 16/28, A61K 39/395

(54) **ANTI-GPVI ANTIBODIES AND FUNCTIONAL FRAGMENTS THEREOF**

(71) Applicant: Emfret Analytics GmbH & Co. KG, 97246 Eibelstadt (DE)
(72) Inventor: NIESWANDT, Bernhard, 97246 Eibelstadt (DE)
(74) Representative: Kraus & Lederer PartGmbB

(57) **Abstract**

The present invention relates to antibody molecules and functional fragments thereof, capable of binding to human glycoprotein VI (GPVI), to processes for their production, and to their therapeutic uses.

## Description

### FIELD OF THE INVENTION

The present invention relates to antibody molecules and functional fragments thereof, capable of binding to human glycoprotein VI (GPVI), to processes for their production, and to their therapeutic uses.

### BACKGROUND

Platelet adhesion and aggregation at sites of vascular injury is essential for normal hemostasis and maintenance of vascular integrity.¹⁻³ The pathological deviation of hemostasis is thrombosis, which is associated with cardiovascular diseases (e.g. myocardial infarction, stroke, lower limb ischemia, venous thromboembolism) and with numerous pathologies, such as cancer, infections or inflammatory diseases, and which represents the leading causes of global death and severe disability and remain a major global health burden.

Thrombosis is the formation of intravascular clots in an uncontrolled manner, e.g. at sites of atherosclerotic plaque rupture. This can result in vascular occlusion and cause life-threatening cardiovascular disease states like myocardial infarction or ischemic stroke.^{4, 5} Therefore, anti-platelet drugs such as acetyl salicylic acid, P2Y₁₂ ADP receptor-blockers or glycoprotein (GP) Ilb/Illa inhibitors have become indispensable therapeutics to efficiently prevent or treat arterial thrombosis, However, these drugs target mechanisms also common to hemostasis, and so their use leads to an inherent antihemostatic effects and an increased risk of uncontrolled bleeding complications that correlates with their antithrombotic efficacy and restricts their use, most obviously in multimorbid patients requiring dual platelet inhibition or combined anticoagulation .^{2, 6, 7, 26} Thus, there is a need for new safe and efficient treatments without systemic side-effects such as hemorrhage. The development of new antithrombotic treatment regimens with a focus on a reduced risk of bleeding without losing drug efficacy, however, remains challenging.

Glycoprotein VI (GPVI) is an activating platelet receptor for collagen/fibrin(ogen) and an important antithrombotic target as its absence as well as its functional inhibition prevent experimental thrombosis and thrombo-inflammatory disease states without impairing hemostasis. In particular, both the absence and functional inhibition of GPVI provides protection from pathological thrombus formation and thrombo-inflammatory disease mechanisms such as (hyper-)acute ischemic stroke⁸ in several mammalian model organisms without causing major bleeding complications.⁹⁻¹³ Thus the functional inhibition of GPVI is a promising and suitable strategy to treat thrombosis and associated diseases.

GPVI is a ~65 kDa transmembrane signaling receptor for collagen and fibrin that is exclusively expressed on platelets and megakaryocytes. It is non-covalently associated with the immunoreceptor tyrosine-based activation motif (ITAM)-containing Fc receptor common γ-subunit (FcRy-chain) which serves as the signal transducing subunit of the receptor complex.^{14, 15} The ectodomain of GPVI is composed of two Ig-like domains namely D1, and D2, followed by a putative intrinsically disordered region proximal to the transmembrane region.¹⁶ Based on X-ray crystallography collagen binding was attributed to D1,¹⁷ whereas GPVI dimerization is largely mediated by D2 and parts of the following disordered region.¹⁸

Different approaches to pharmacologically target GPVI-mediated platelet activation have been reported, including antibody (IgG)-mediated GPVI immunodepletion,^{9, 19, 20} the competitive inhibitor Revacept (dimeric GPVI-Fc fusion protein),^{21, 22} and GPVI blockade by monovalent IgG-derived Fab fragments^{20, 23, 24, 25, 54} and these studies showed a significant benefit of these different anti-GPVI strategies in experimental models of thrombosis and thrombo-inflammation.

Until now, several antibodies and antibody fragments have been reported and their GPVI binding sites have been mapped to various different interfaces within residues 58 to 187, but not beyond.³¹ Few of these antibodies and antibody fragments are capable of blocking GPVI-collagen interaction, some induce GPVI shedding, and they generally suffer from a weak affinity for GPVI, making them unsuitable for clinical investigation.²⁶ The first reported function blocking anti-GPVI antibody was JAQ1 (rat IgG), raised against mouse GPVI,³³ that also recognizes human GPVI but does not block its function.^{32, 55, 56} However, *in vivo* administration of JAQ1 IgG in mice induced GPVI depletion through a Fc-dependent mechanism¹⁹ resulting in a sustained GPVI-knockout like phenotype and long-term antithrombotic protection.⁹ Further studies later suggested that similar mechanisms of GPVI depletion may also occur in humans.^{14, 48, 20} GPVI depletion is undesirable since it cannot be controlled, and is irreversible (i.e. it lasts the lifetime of platelets, or even longer due to GPVI depletion on megakaryocytes). In the art, other anti-GPVI antibodies inducing a GPVI depletion phenotype were described.^{57, 58, 59} Generally, whole bivalent IgG could potentially bridge platelet membrane GPVI with the low affinity FcyRIIA receptor, leading to platelet activation and possibly induce GPVI deficiency because of internalization or shedding.²⁶

One antibody fragment with a slightly higher but still moderate binding affinity for GPVI is the humanized monovalent GPVI-blocking Fab (ACT017, glenzocimab) with a KD of 4.1 nM, which is based on the parent mouse antibody fragment, 9O12^{Fab} with a K_{D} of 17 nM.^{26,60, 61} Mapping of ACT017 binding site to GPVI showed recognition of a discontinuous epitope composed of two stretches 114 to 142 and 165 to 187 of hGPVI (numbering according to Q9HCN6) located in the D2 domain.³¹

In initial studies with the monovalent parent mouse antibody fragment of ACT017, 9012^{Fab},²⁶ it efficiently inhibited GPVI ex *vivo* in non-human primates²⁹ and in a mouse line expressing human GPVI instead of mouse GPVI.²³ First data from a clinical phase II study with the humanized GPVI-blocking Fab (ACT017, glenzocimab) indicate that treatment with anti-GPVI Fab fragment may be effective as an add-on therapy in the acute phase of ischemic stroke,^{26, 27} and in reducing the thrombo-inflammatory response that drives infarct progression. In comparison, the other GPVI inhibitor that recently entered the clinical stage, Revacept, failed to reduce the incidence of myocardial injury in patients with stable ischemic heart disease²² and different groups also reported that this indirect approach was less effective compared to direct functional inhibition of GPVI by antibodies in mouse and human platelets.^{46, 47}

The *in vivo* half-life of 9012^{Fab} in mice (~2.5 hours at a dose of 4 mg/kg intravenously^{23,29}) as well as that of ACT017 (glenzocimab) in humans (~10 hours at a dose of >16 mg/kg intravenously³⁰), however, are relatively short, which, at least in part, may be explained by the rather moderate binding affinity of both 9012^{Fab} and ACT017 to GPVI (K_{D}: 17.08 nM and 4.1 nM, respectively).^{26,29, 30} The moderate binding affinity and relatively short plasma half-life are paralleled in mice by a complete clearance of the antibody fragment at 24 hours after injection,²³ and in primates by a rapid decline in detection of 9O12^{Fab} binding to circulating platelets, with a reduction of 9O12^{Fab} on circulating platelets to < 50% of peak level as well as more than a halving of the 9O12^{Fab}-positive circulating platelets within 24 hours after injection.²⁹

There are other GPVI antibodies described in the prior art, including four mouse monoclonal anti-GPVI antibodies OM1, OM2, OM3 and OM4, that were found to inhibit GPVI binding to collagen, collagen-induced secretion and thromboxane A2 (TxA2) formation *in vitro, ex vivo* collagen-induced platelet aggregation after intravenous injection to *Cynomolgus* monkeys, and OM4 also appeared to inhibit thrombus formation in a rat thrombosis model.⁶² These antibodies are reported to have a smaller K_{D} value than for example ACT017, which is however greater than 0.7 nM. Moreover, the respective patent document reports that the K_{D} measurements were carried out using bivalent IgG antibodies and that monovalent fragments generally have somewhat reduced affinities compared with their corresponding intact IgG.⁶²

There is thus an ongoing need for an improved inhibitor of human GPVI as an anti-platelet agent to efficiently prevent or treat arterial or venous thrombosis and thrombo-inflammatory disease states in humans at risk, while having a low hemorrhagic risk. The inhibitor of GPVI should at least have a high affinity for human GPVI (e.g. at least a K_{D} < 700 pM) and preferably a long plasma half-life. Ideally such an inhibitor of GPVI is characterized by a prolonged persistence on circulating platelets and is effective in inhibiting thrombus formation as assessed *in vitro* and/or *in vivo.*

### SUMMARY OF THE INVENTION

The inventors of the present application found certain anti-GPVI antibodies and functional fragments thereof with extremely high binding affinity for human GPVI with dissociation equilibrium constant (K_{D}) of less than 700 pM, and as low as 195 pM (thus ~21-fold lower than ACT017). Moreover, the new anti-GPVI antibodies and functional fragments (i) unexpectedly bind a novel epitope on human GPVI, which includes the proposed GPVI dimerization residues; (ii) have a long plasma half-life and potently inhibit GPVI function in human and *hGP6*^{*tg*/*tg*} mouse platelets of mice humanized for *GPVI* (*hGP6*^{*tg*/*tg*} mice) *in vitro;* (iii) and *in vivo* provide sustained GPVI blockade (e.g. with >50% receptor occupancy at 48 h after injection and > 35% at 72 h after injection on circulating platelets) and profound thrombosis protection (e.g. are effective in inhibiting thrombus formation as assessed *in vitro* and/or *in vivo*), while having no significant effect on tail bleeding times in the *hGP6*^{*tg*/*tg*} mouse model. Based on these results, it is reasonable to expect that the anti-GPVI antibodies and functional fragments of the present invention achieve a desired level of GPVI inhibition in humans at comparably lower doses and/or for longer time periods compared to for example ACT017. The antibodies and functional fragments thereof in monovalent form exclude Fc-dependent thrombocytopenia and/or GPVI depletion, and as Fab fragment circumvent possible Fc-mediated effects of full-length IgG through the platelet expressed Fc gamma receptor IIA (FcyRIIA, CD32). Thus, the present invention provides anti-GPVI antibodies and functional fragments with very high affinity for human GPVI, allowing sustained and safe inhibition of human GPVI function on circulating platelets, presumably through a non-canonical mechanism of action, at very low hemorrhagic risk.

The invention provides antibody molecules and functional fragments thereof capable of binding to human GPVI with very high affinity and blocking its function *in vitro* and *in vivo* at low hemorrhagic risk. The present invention therefore relates to the subject matter defined in the following items (1) to (133):
(1) An antibody or a functional fragment thereof capable of binding to human glycoprotein VI (GPVI), wherein the antibody or functional fragment comprises (i) a V_{L} domain comprising a CDR1 region having an amino acid sequence in accordance with the amino acid sequence as shown in SEQ ID NO:1, a CDR2 region having an amino acid sequence in accordance with the amino acid sequence as shown in SEQ ID NO:2 or SEQ ID NO:9, and a CDR3 region having an amino acid sequence in accordance with the amino acid sequence as shown in SEQ ID NO:3, and (ii) a V_{H} domain comprising a CDR1 region having an amino acid sequence in accordance with the amino acid sequence as shown in SEQ ID NO:4, a CDR2 region having an amino acid sequence in accordance with the amino acid sequence as shown in SEQ ID NO:5 or SEQ ID NO:13, and a CDR3 region having an amino acid sequence in accordance with the amino acid sequence as shown in SEQ ID NO:6.
(2) The antibody or functional fragment thereof of item (1), wherein the antibody or functional fragment comprises (i) a V_{L} domain comprising a CDR1 region having an amino acid sequence in accordance with the amino acid sequence as shown in SEQ ID NO:1, a CDR2 region having an amino acid sequence in accordance with the amino acid sequence as shown in SEQ ID NO:9, and a CDR3 region having an amino acid sequence in accordance with the amino acid sequence as shown in SEQ ID NO:3, and (ii) a V_{H} domain comprising a CDR1 region having an amino acid sequence in accordance with the amino acid sequence as shown in SEQ ID NO:4, a CDR2 region having an amino acid sequence in accordance with the amino acid sequence as shown in SEQ ID NO:5, and a CDR3 region having an amino acid sequence in accordance with the amino acid sequence as shown in SEQ ID NO:6.
(3) The antibody or functional fragment thereof of item (1) or (2), wherein in the amino acid sequence SEQ ID NO:9 the residue X is A, R, N, D, Q, E, G, H, I, L, K, M, F, S, T, W, Y, or V.
(4) The antibody or functional fragment thereof of any one of items (1)-(3), wherein in the amino acid sequence SEQ ID NO:9 the residue X is A.
(5) The antibody or functional fragment thereof of any one of items (1)-(3), wherein in the amino acid sequence SEQ ID NO:9 the residue X is R.
(6) The antibody or functional fragment thereof of any one of items (1)-(3), wherein in the amino acid sequence SEQ ID NO:9 the residue X is N.
(7) The antibody or functional fragment thereof of any one of items (1)-(3), wherein in the amino acid sequence SEQ ID NO:9 the residue X is D.
(8) The antibody or functional fragment thereof of any one of items (1)-(3), wherein in the amino acid sequence SEQ ID NO:9 the residue X is Q.
(9) The antibody or functional fragment thereof of any one of items (1)-(3), wherein in the amino acid sequence SEQ ID NO:9 the residue X is E.
(10) The antibody or functional fragment thereof of any one of items (1)-(3), wherein in the amino acid sequence SEQ ID NO:9 the residue X is G.
(11) The antibody or functional fragment thereof of any one of items (1)-(3), wherein in the amino acid sequence SEQ ID NO:9 the residue X is H.
(12) The antibody or functional fragment thereof of any one of items (1)-(3), wherein in the amino acid sequence SEQ ID NO:9 the residue X is I.
(13) The antibody or functional fragment thereof of any one of items (1)-(3), wherein in the amino acid sequence SEQ ID NO:9 the residue X is L.
(14) The antibody or functional fragment thereof of any one of items (1)-(3), wherein in the amino acid sequence SEQ ID NO:9 the residue X is K.
(15) The antibody or functional fragment thereof of any one of items (1)-(3), wherein in the amino acid sequence SEQ ID NO:9 the residue X is M.
(16) The antibody or functional fragment thereof of any one of items (1)-(3), wherein in the amino acid sequence SEQ ID NO:9 the residue X is F.
(17) The antibody or functional fragment thereof of any one of items (1)-(3), wherein in the amino acid sequence SEQ ID NO:9 the residue X is S.
(18) The antibody or functional fragment thereof of any one of items (1)-(3), wherein in the amino acid sequence SEQ ID NO:9 the residue X is T.
(19) The antibody or functional fragment thereof of any one of items (1)-(3), wherein in the amino acid sequence SEQ ID NO:9 the residue X is W.
(20) The antibody or functional fragment thereof of any one of items (1)-(3), wherein in the amino acid sequence SEQ ID NO:9 the residue X is Y.
(21) The antibody or functional fragment thereof of any one of items (1)-(3), wherein in the amino acid sequence SEQ ID NO:9 the residue X is V.
(22) The antibody or functional fragment thereof of item (1), wherein the antibody or functional fragment comprises (i) a V_{L} domain comprising a CDR1 region having an amino acid sequence in accordance with the amino acid sequence as shown in SEQ ID NO:1, a CDR2 region having an amino acid sequence in accordance with the amino acid sequence as shown in SEQ ID NO:2, and a CDR3 region having an amino acid sequence in accordance with the amino acid sequence as shown in SEQ ID NO:3, and (ii) a V_{H} domain comprising a CDR1 region having an amino acid sequence in accordance with the amino acid sequence as shown in SEQ ID NO:4, a CDR2 region having an amino acid sequence in accordance with the amino acid sequence as shown in SEQ ID NO:5, and a CDR3 region having an amino acid sequence in accordance with the amino acid sequence as shown in SEQ ID NO:6.
(23) The antibody or functional fragment thereof of item (1), wherein in the amino acid sequence SEQ ID NO:13 the residue X at position 5 (X5) is D or E; and/or the residue X at position 6 (X6) is G or A.
(24) The antibody or functional fragment thereof of any one of the preceding items, which is monovalent.
(25) The functional fragment of any one of the preceding items, which is a fragment antigen-binding (Fab), a F(ab'), a Fv, a disulfide-linked variable fragment (dsFv), a monovalent IgG, or a single-chain variable fragment (scFv).
(26) The functional fragment of any one of the preceding items, which is a fragment antigen-binding (Fab).
(27) The antibody or functional fragment of any one of the preceding items, wherein the antibody or functional fragment thereof specifically binds to human GPVI.
(28) The antibody or functional fragment of any one of the preceding items, wherein said antibody or functional fragment thereof does not significantly bind to proteins structurally closely related to human GPVI.
(29) An antibody or functional fragment, in particular the antibody or functional fragment of any one of the preceding items, which is capable of binding human GPVI at a binding epitope corresponding to amino acids V178 to E192 and/or S223 to P237 of SEQ ID NO:36.
(30) The antibody of item (29), wherein the binding epitope is a discontinuous epitope comprising, or consisting of, amino acids V178 to E192 and S223 to P237 of SEQ ID NO:36.
(31) The antibody or functional fragment of any one of the preceding items, which is capable of binding human GPVI at the residues of GPVI proposed to be involved in dimerization.
(32) The antibody or functional fragment of any one of the preceding items, wherein binding of antibody or functional fragment to a human GPVI interrupts, or interferes with, GPVI dimerization.
(33) An antibody or functional fragment thereof binding to essentially the same binding epitope as the antibody or functional fragment of any one of the preceding items.
(34) The antibody or functional fragment of any one of the preceding items, wherein the antibody or functional fragment binds to human GPVI with a dissociation equilibrium constant (K_{D}) of less than 1 µM, preferably less than 900 pM, more preferably less than 800 pM, even more preferably less than 700 pM, even more preferably less than 600 pM, even more preferably less than 500 pM, even more preferably less than 400 pM, even more preferably less than 350 pM, even more preferably less than 300 pM, even more preferably less than 250 pM, even more preferably less than 200 pM, even more preferably about 195 pM or less, even more preferably about 175 pM or less.
(35) The antibody or functional fragment of any one of the preceding items, which binds to human GPVI with a dissociation equilibrium constant (K_{D}) of 195 pM or less.
(36) The antibody or functional fragment of item (34) or (35), wherein the dissociation equilibrium constant (K_{D}) is determined using bio-layer interferometry (BLI), surface plasmon resonance (SPR) technology (for example in a BIACORE instrument), or ELISA; preferably BLI (for example with an Octet instrument and using Fortebio software).
(37) The antibody or functional fragment of any one of items (34) to (36), wherein the dissociation equilibrium constant (K_{D}) is determined using bio-layer interferometry (BLI) (for example using an Octet instrument), preferably wherein the determination of the K_{D} is carried out as described in the Examples in section 1.16.
(38) The antibody or functional fragment of any one of the preceding items, wherein the dissociation equilibrium constant (K_{D}) is determined using bio-layer interferometry (BLI) preferably using an Octet instrument, at 25°C, and preferably at a constant orbital flow rate of e.g. 1000 rpm; using an antigen (human GPVI) loading concentration for immobilizing of antigen on biosensors of 1.2 µg/ml, a time for association of 900 s and a time for dissociation of 1200 s, an antibody screening range with seven concentrations ranging from 10 to 0.014 nM using a 3-fold serial dilution, and a 1:1 fitting model.
(39) The antibody or functional fragment of any one of the preceding items, wherein the antibody or functional fragment comprises a V_{L} domain comprising, or consisting of, an amino acid sequence having a sequence identity of at least 80 %, preferably at least 90 %, more preferably at least 92%, even more preferably at least 94 %, even more preferably at least 95%, even more preferably at least 96 %, even more preferably at least 97%, even more preferably at least 98 %, even more preferably at least 99 %, to a sequence selected from the group consisting of SEQ ID NO:16, 17, 18, 19, 26, 27, 28, 29, 30, 31, 32, 33, 34, and 35, preferably 16, 17, 18, 19, 32, 33, 34 and 35, more preferably 16, 17, 18, 32, 33, and 34.
(40) The antibody or functional fragment of any one of the preceding items, wherein the antibody or functional fragment comprises a V_{L} domain comprising, or consisting of, an amino acid sequence having a sequence identity of at least 80 %, preferably at least 90 %, more preferably at least 92%, even more preferably at least 94 %, even more preferably at least 95%, even more preferably at least 96 %, even more preferably at least 97%, even more preferably at least 98 %, even more preferably at least 99 %, to a sequence selected from the group consisting of SEQ ID NO: 26, 27, 28, 32, 33, 34, and 35, preferably 32, 33, 34 and 35, more preferably 32, 33, and 34.
(41) The antibody or functional fragment of any one of the preceding items, wherein the antibody or functional fragment comprises a V_{L} domain comprising, or consisting of, an amino acid sequence having a sequence identity of at least 80 %, preferably at least 90 %, more preferably at least 92%, even more preferably at least 94 %, even more preferably at least 95%, even more preferably at least 96 %, even more preferably at least 97%, even more preferably at least 98 %, even more preferably at least 99 %, to a sequence selected from the group consisting of SEQ ID NO:16, 17, 18, 19, 29, 30 and 31, preferably 16, 17, 18 and 19, more preferably 16, 17 and 18.
(42) The antibody or functional fragment of any one of the preceding items, wherein the antibody or functional fragment comprises a V_{H} domain comprising, or consisting of, an amino acid sequence having a sequence identity of at least 80 %, preferably at least 90 %, more preferably at least 92 %, even more preferably at least 94 %, even more preferably at least 95 %, even more preferably at least 96 %, even more preferably at least 97 %, even more preferably at least 98 %, even more preferably at least 99 %, to a sequence selected from the group consisting of SEQ ID NO:21, 22, 23, 24 and 25, preferably 21, 22 and 23.
(43) The antibody or functional fragment of any one of the preceding items, wherein the antibody or functional fragment comprises a V_{L} domain comprising, or consisting of, an amino acid sequence as shown in SEQ ID NO:16, 17, 18, 19, 26, 27, 28, 29, 30, 31, 32, 33, 34, or 35, preferably 16, 17, 18, 19, 32, 33, 34, or 35, more preferably 16, 17, 18, 32, 33, or 34.
(44) The antibody or functional fragment of any one of the preceding items, wherein the antibody or functional fragment comprises a V_{L} domain comprising, or consisting of, an amino acid sequence as shown in SEQ ID NO: 26, 27, 28, 32, 33, 34, or 35, preferably 32, 33, 34, or 35, more preferably 32, 33, or 34.
(45) The antibody or functional fragment of any one of items (1)-(43), wherein the antibody or functional fragment comprises a V_{L} domain comprising, or consisting of, an amino acid sequence as shown in SEQ ID NO:16, 17, 18, 19, 29, 30 or 31, preferably 16, 17, 18 or 19, more preferably 16, 17 or 18.
(46) The antibody or functional fragment of any one of the preceding items, wherein the antibody or functional fragment comprises a V_{H} domain comprising, or consisting of, an amino acid sequence as shown in SEQ ID NO:21, 22, 23, 24 or 25, preferably 21, 22, or 23.
(47) The antibody or functional fragment of any one of the preceding items, wherein the V_{L} domain and V_{H} domain, comprise, or consist of a pair of amino acids sequences as shown SEQ ID NO: 32 and 21, 32 and 22, 32 and 23, 32 and 24, 32 and 25, 33 and 21, 33 and 22, 33 and 23, 33 and 24, 33 and 25, 34 and 21, 34 and 22, 34 and 23, 34 and 24, 34 and 25, 35 and 21, 35 and 22, 35 and 23, 35 and 24, 35 and 25, 26 and 21, 26 and 22, 26 and 23, 26 and 24, 26 and 25, SEQ ID NO: 16 and 21, 16 and 22, 16 and 23, 16 and 24, 16 and 25, 17 and 21, 17 and 22, 17 and 23, 17 and 24, 17 and 25, 18 and 21, 18 and 22, 18 and 23, 18 and 24, 18 and 25, 19 and 21, 19 and 22, 19 and 23, 19 and 24, 19 and 25, 29 and 21, 29 and 22, 29 and 23, 29 and 24, or 29 and 25; preferably 32 and 22, 32 and 23, 32 and 25, 33 and 21, 33 and 22, 33 and 23, 33 and 24, 33 and 25, 34 and 21, 34 and 22, 34 and 23, 16 and 22, 16 and 23, 16 and 25, 17 and 21, 17 and 22, 17 and 23, 17 and 24, 17 and 25, 18 and 21, 18 and 22, or 18 and 23; more preferably 32 and 22, 33 and 21, 33 and 22, 33 and 23, 34 and 21, 34 and 22, 34 and 23, 16 and 22, 17 and 21, 17 and 22, 17 and 23, 18 and 21, 18 and 22, or 18 and 23; even more preferably 33 and 21, 33 and 22, 33 and 23, 34 and 21, 34 and 22, 34 and 23, 17 and 21, 17 and 22, 17 and 23, 18 and 21, 18 and 22, or 18 and 23; even more preferably 33 and 21, 33 and 22, 33 and 23, 34 and 21, 34 and 22, 17 and 21, 17 and 22, 17 and 23, 18 and 21, or 18 and 22; even more preferably 33 and 21, 33 and 22, 34 and 22, 17 and 21, 17 and 22, or 18 and 22.
(48) The antibody or functional fragment of any one of the preceding items, wherein the V_{L} domain and V_{H} domain, comprise, or consist of a pair of amino acids sequences as shown SEQ ID NO:32 and 21, 32 and 22, 32 and 23, 32 and 24, 32 and 25, 33 and 21, 33 and 22, 33 and 23, 33 and 24, 33 and 25, 34 and 21, 34 and 22, 34 and 23, 34 and 24, 34 and 25, 35 and 21, 35 and 22, 35 and 23, 35 and 24, 35 and 25, 26 and 21, 26 and 22, 26 and 23, 26 and 24, 26 and 25, 27 and 21, 27 and 22, 27 and 23, 27 and 24, 27 and 25, 28 and 21, 28 and 22, 28 and 23, 28 and 24, or 28 and 25; preferably 32 and 22, 32 and 23, 32 and 25, 33 and 21, 33 and 22, 33 and 23, 33 and 24, 33 and 25, 34 and 21, 34 and 22, 34 and 23, 26 and 22, 26 and 23, 26 and 25, 27 and 21, 27 and 22, or 27 and 23; more preferably 32 and 22, 33 and 21, 33 and 22, 33 and 23, 34 and 21, 34 and 22, 34 and 23, 26 and 22, 27 and 21, 27 and 22, or 27 and 23; even more preferably 33 and 21, 33 and 22, 33 and 23, 34 and 21, 34 and 22, 34 and 23, 27 and 21, 27 and 22, or 27 and 23; even more preferably 33 and 21, 33 and 22, 33 and 23, 34 and 21, 34 and 22, 27 and 21, or 27 and 22; even more preferably 33 and 21, 33 and 22, 34 and 22, or 27 and 22.
(49) The antibody or functional fragment of any one of the preceding items, wherein the V_{L} domain and V_{H} domain, comprise, or consist of a pair of amino acids sequences as shown SEQ ID NO: 32 and 21, 32 and 22, 32 and 23, 32 and 24, 32 and 25, 33 and 21, 33 and 22, 33 and 23, 33 and 24, 33 and 25, 34 and 21, 34 and 22, 34 and 23, 34 and 24, 34 and 25, 35 and 21, 35 and 22, 35 and 23, 35 and 24, 35 and 25, 26 and 21, 26 and 22, 26 and 23, 26 and 24, or 26 and 25; preferably 32 and 22, 32 and 23, 32 and 25, 33 and 21, 33 and 22, 33 and 23, 33 and 24, 33 and 25, 34 and 21, 34 and 22, or 34 and 23; more preferably 32 and 22, 33 and 21, 33 and 22, 33 and 23, 34 and 21, 34 and 22, or 34 and 23; even more preferably 33 and 21, 33 and 22, 33 and 23, 34 and 21, 34 and 22, or 34 and 23; even more preferably 33 and 21, 33 and 22, 33 and 23, 34 and 21, or 34 and 22; even more preferably 33 and 21, 33 and 22, or 34 and 22.
(50) The antibody or functional fragment of any one of items (1)-(47), wherein the V_{L} domain and V_{H} domain, comprise, or consist of a pair of amino acids sequences as shown SEQ ID NO:16 and 21, 16 and 22, 16 and 23, 16 and 24, 16 and 25, 17 and 21, 17 and 22, 17 and 23, 17 and 24, 17 and 25, 18 and 21, 18 and 22, 18 and 23, 18 and 24, 18 and 25, 19 and 21, 19 and 22, 19 and 23, 19 and 24, 19 and 25, 29 and 21, 29 and 22, 29 and 23, 29 and 24, 29 and 25, 30 and 21, 30 and 22, 30 and 23, 30 and 24, 30 and 25, 31 and 21, 31 and 22, 31 and 23, 31 and 24, or 31 and 25; preferably 16 and 22, 16 and 23, 16 and 25, 17 and 21, 17 and 22, 17 and 23, 17 and 24, 17 and 25, 18 and 21, 18 and 22, 18 and 23, 29 and 22, 29 and 23, 29 and 25, 30 and 21, 30 and 22, or 30 and 23; more preferably 16 and 22, 17 and 21, 17 and 22, 17 and 23, 18 and 21, 18 and 22, 18 and 23, 29 and 22, 30 and 21, 30 and 22, or 30 and 23; even more preferably 17 and 21, 17 and 22, 17 and 23, 18 and 21, 18 and 22, 18 and 23, 30 and 21, 30 and 22, or 30 and 23; even more preferably 17 and 21, 17 and 22, 17 and 23, 18 and 21, 18 and 22, 30 and 21, or 30 and 22; even more preferably 17 and 21, 17 and 22, 18 and 22, or 30 and 22.
(51) The antibody or functional fragment of any one of items (1)-(47), wherein the V_{L} domain and V_{H} domain, comprise, or consist of a pair of amino acids sequences as shown SEQ ID NO: 16 and 21, 16 and 22, 16 and 23, 16 and 24, 16 and 25, 17 and 21, 17 and 22, 17 and 23, 17 and 24, 17 and 25, 18 and 21, 18 and 22, 18 and 23, 18 and 24, 18 and 25, 19 and 21, 19 and 22, 19 and 23, 19 and 24, 19 and 25, 29 and 21, 29 and 22, 29 and 23, 29 and 24, or 29 and 25; preferably 16 and 22, 16 and 23, 16 and 25, 17 and 21, 17 and 22, 17 and 23, 17 and 24, 17 and 25, 18 and 21, 18 and 22, or 18 and 23; more preferably 16 and 22, 17 and 21, 17 and 22, 17 and 23, 18 and 21, 18 and 22, or 18 and 23; even more preferably 17 and 21, 17 and 22, 17 and 23, 18 and 21, 18 and 22, or 18 and 23; even more preferably 17 and 21, 17 and 22, 17 and 23, 18 and 21, or 18 and 22; even more preferably 17 and 21, 17 and 22, or 18 and 22.
(52) The antibody or functional fragment of any one of the preceding items, wherein the antibody or functional fragment comprises a V_{L} domain comprising, or consisting of, an amino acid sequence having a sequence identity of at least 80%, preferably at least 90 %, more preferably at least 92%, even more preferably at least 94%, even more preferably at least 95%, even more preferably at least 96%, even more preferably at least 97%, even more preferably at least 98%, even more preferably at least 99%, to the amino acid sequence as shown in SEQ ID NO:17; and a V_{H} domain comprising, or consisting of, an amino acid sequence having a sequence identity of at least 80%, preferably at least 90 %, more preferably at least 92%, even more preferably at least 94%, even more preferably at least 95%, even more preferably at least 96%, even more preferably at least 97%, even more preferably at least 98%, even more preferably at least 99%, to the amino acid sequence as shown in SEQ ID NO: 21.
(53) The antibody or functional fragment of any one of the preceding items, wherein the antibody or functional fragment comprises a V_{L} domain comprising, or consisting of, an amino acid sequence having a sequence identity of at least 80%, preferably at least 90 %, more preferably at least 92%, even more preferably at least 94%, even more preferably at least 95%, even more preferably at least 96%, even more preferably at least 97%, even more preferably at least 98%, even more preferably at least 99%, to the amino acid sequence as shown in SEQ ID NO:33; and a V_{H} domain comprising, or consisting of, an amino acid sequence having a sequence identity of at least 80%, preferably at least 90 %, more preferably at least 92%, even more preferably at least 94%, even more preferably at least 95%, even more preferably at least 96%, even more preferably at least 97%, even more preferably at least 98%, even more preferably at least 99%, to the amino acid sequence as shown in SEQ ID NO: 21.
(54) The antibody or functional fragment of any one of items (1)-(47) and (50)-(52), wherein the antibody or functional fragment comprises a V_{L} domain comprising, or consisting of, an amino acid sequence as shown in SEQ ID NO:17; and a V_{H} domain comprising, or consisting of, an amino acid sequence as shown in SEQ ID NO: 21.
(55) The antibody or functional fragment of any one of items (1)-(49), wherein the antibody or functional fragment comprises a V_{L} domain comprising, or consisting of, an amino acid sequence as shown in SEQ ID NO:33; and a V_{H} domain comprising, or consisting of, an amino acid sequence as shown in SEQ ID NO: 21.
(56) The antibody or functional fragment of any one of items (1) to (42), wherein the antibody or functional fragment comprises a V_{L} domain comprising, or consisting of, an amino acid sequence as shown in SEQ ID NO:15; and/or a V_{H} domain comprising, or consisting of, an amino acid sequence as shown in SEQ ID NO: 20; optionally wherein the V_{L} domain and/or the V_{H} domain comprise 10, 9, 8, 7, 6, 5, 4, 3, 2 or 1 amino acid substitutions relative to the sequences shown in SEQ ID NO:15 and SEQ ID NO:20, respectively.
(57) The antibody or functional fragment of any one of the preceding items, which is an immunoglobulin G (IgG) or functional fragment thereof, preferably an IgG1 or a functional fragment thereof.
(58) The antibody or functional fragment of any one of the preceding items, wherein the antibody or functional fragment comprises, or consists of, a light chain comprising the V_{L} domain and a heavy chain comprising the V_{H} domain.
(59) The antibody or functional fragment of any one of the preceding items, wherein the antibody or functional fragment comprises, or consists of, a light chain comprising, in addition to the V_{L} domain, a constant domain (C_{L}), preferably C-terminal to the V_{L} domain, and a heavy chain comprising, in addition to the V_{H} domain, a constant domain (C_{H}), preferably C-terminal to the V_{H} domain.
(60) The antibody or functional fragment of item (58) or (59), wherein the antibody or functional fragment comprises a light chain comprising a constant domain derived from human IgK light chain constant domain (allotype Km3), and preferably wherein the sum of the number of amino acids, which are different from the amino acid sequence of the human IgK light chain constant domain (allotype Km3) e.g. as shown in SEQ ID NO:60, is less than 10, 9, 8, 7, 6, 5, 4, 3, or 2; and/or wherein the antibody or functional fragment comprises a heavy chain comprising a constant domain derived from human IgG1 heavy chain C_{H}1 constant domain (allotype G1m17,1), and preferably wherein the sum of the number of amino acids, which are different from the amino acid sequence of the human IgG1 heavy chain C_{H}1 constant domain (allotype G1m17,1) e.g. as shown in SEQ ID NO:61, is less than 10, 9, 8, 7, 6, 5, 4, 3, or 2.
(61) The antibody or functional fragment of any one of items (59) to (60), wherein C_{L} domain comprises, or consists of, an amino acid sequence having a sequence identity of at least 80 %, preferably at least 90 %, more preferably at least 92 %, even more preferably at least 94%, even more preferably at least 95 %, even more preferably at least 96 %, even more preferably at least 97 %, even more preferably at least 98 %, even more preferably at least 99 %, to the amino acid shown in SEQ ID NO:60.
(62) The antibody or functional fragment of any one of items (59) to (61), wherein C_{H} domain comprises, or consists of, an amino acid sequence having a sequence identity of at least 80 %, preferably at least 90 %, more preferably at least 92 %, even more preferably at least 94 %, even more preferably at least 95 %, even more preferably at least 96 %, even more preferably at least 97%, even more preferably at least 98 %, even more preferably at least 99 %, to the amino acid shown in SEQ ID NO:61.
(63) The antibody or functional fragment of any one of items (58) to (62), wherein the light chain comprises an N-terminal light chain signal peptide comprising, or consisting of, an amino acid sequence as shown in SEQ ID NO:62, and/or a light chain constant domain (C_{L}) comprising, or consisting of, an amino acid sequence as shown in SEQ ID NO:60, and preferably C-terminal to the V_{L} domain.
(64) The antibody or functional fragment of any one of items (58) to (63), wherein the heavy chain comprises an N-terminal heavy chain signal peptide comprising, or consisting of, an amino acid sequence as shown in SEQ ID NO:63, and/or a heavy chain constant domain (C_{H}) comprising, or consisting of, an amino acid sequence as shown in SEQ ID NO:61, and preferably C-terminal to the V_{H} domain.
(65) The antibody or functional fragment of any one of the preceding items, which is monovalent, preferably a Fab, comprising, or consisting of, a light chain, which comprises, or consist of, an amino acid sequence having a sequence identity of at least 80%, preferably at least 90%, more preferably at least 92%, even more preferably at least 94%, even more preferably at least 95%, even more preferably at least 96%, even more preferably at least 97%, even more preferably at least 98%, even more preferably at least 99%, even more preferably at least 99.5%, to a sequence selected from the group consisting of SEQ ID NO: 38, 39, 40, 41, 49, 50, 51, 52, 53, 54, 55, 56, 57 and 58, preferably of SEQ ID NO: 38, 39, 40, 41, 55, 56, 57 and 58; and a heavy chain, which comprises, or consist of, an amino acid sequence having a sequence identity of at least 80%, preferably at least 90 %, more preferably at least 92%, even more preferably at least 94%, even more preferably at least 95%, even more preferably at least 96%, even more preferably at least 97%, even more preferably at least 98%, even more preferably at least 99%, even more preferably at least 99.5%, to a sequence selected from the group consisting of SEQ ID NO: 44, 45, 46, 47 and 48.
(66) The antibody or functional fragment of any one of the preceding items, which is monovalent, preferably a Fab, comprising, or consisting of, a light chain, which comprises, or consist of, an amino acid sequence having a sequence identity of at least 80%, preferably at least 90%, more preferably at least 92%, even more preferably at least 94%, even more preferably at least 95%, even more preferably at least 96%, even more preferably at least 97%, even more preferably at least 98%, even more preferably at least 99%, even more preferably at least 99.5%, to a sequence selected from the group consisting of SEQ ID NO: 38, 39, 40 and 41; and a heavy chain, which comprises, or consist of, an amino acid sequence having a sequence identity of at least 80%, preferably at least 90 %, more preferably at least 92%, even more preferably at least 94%, even more preferably at least 95%, even more preferably at least 96%, even more preferably at least 97%, even more preferably at least 98%, even more preferably at least 99%, even more preferably at least 99.5%, to a sequence selected from the group consisting of SEQ ID NO: 44, 45, 46, 47 and 48.
(67) The antibody or functional fragment of any one of the preceding items, comprising, or consisting of, a light chain, which comprises, or consists of, an amino acid sequence as shown in one of SEQ ID NO:38, 39, 40, 41, 49, 50, 51, 52, 53, 54, 55, 56, 57 and 58, and a heavy chain, which comprises, or consists of, an amino acid sequence as shown in one of SEQ ID NO:44, 45, 46, 47 and 48.
(68) The antibody or functional fragment of any one of the preceding items, comprising, or consisting of, a light chain, which comprises, or consists of, an amino acid sequence as shown in one of SEQ ID NO: 55, 56, 57 and 58, preferably SEQ ID NO: 55, 56 and 57; and a heavy chain, which comprises, or consists of, an amino acid sequence as shown in one of SEQ ID NO:44, 45, 46, 47 and 48, preferably SEQ ID NO: 44, 45, and 46.
(69) The antibody or functional fragment of any one of items (1)-(67), comprising, or consisting of, a light chain, which comprises, or consists of, an amino acid sequence as shown in one of SEQ ID NO: 38, 39, 40, and 41, preferably SEQ ID NO: 38, 39, and 40; and a heavy chain, which comprises, or consists of, an amino acid sequence as shown in one of SEQ ID NO:44, 45, 46, 47 and 48, preferably SEQ ID NO: 44, 45, and 46.
(70) The antibody or functional fragment of any one of the preceding items, which is monovalent, preferably a fragment antigen-binding (Fab), comprising, or consisting of, a light chain, which comprises, or consists of, an amino acid sequence as shown in one of SEQ ID NO:38, 39, 40, 41, 49, 50, 51, 52, 53, 54, 55, 56, 57 and 58, and a heavy chain, which comprises, or consists of, an amino acid sequence as shown in one of SEQ ID NO:44, 45, 46, 47 and 48.
(71) The antibody or functional fragment of any one of the preceding items, which is monovalent, preferably a fragment antigen-binding (Fab), comprising, or consisting of, a light chain, which comprises, or consists of, an amino acid sequence as shown in one of SEQ ID NO:52, 53, 54, 55, 56, 57 and 58, and a heavy chain, which comprises, or consists of, an amino acid sequence as shown in one of SEQ ID NO:44, 45, 46, 47 and 48.
(72) The antibody or functional fragment of any one of the preceding items, which is monovalent, preferably a fragment antigen-binding (Fab), comprising, or consisting of, a light chain, which comprises, or consists of, an amino acid sequence as shown in one of SEQ ID NO: 55, 56, 57 and 58, and a heavy chain, which comprises, or consists of, an amino acid sequence as shown in one of SEQ ID NO:44, 45, 46, 47 and 48.
(73) The antibody or functional fragment of any one of the preceding items, which is monovalent, preferably a fragment antigen-binding (Fab), comprising, or consisting of, a light chain, which comprises, or consists of, an amino acid sequence as shown in one of SEQ ID NO: 55, 56 and 57, and a heavy chain, which comprises, or consists of, an amino acid sequence as shown in one of SEQ ID NO: 44, 45, and 46.
(74) The antibody or functional fragment of any one of items (1)-(70), which is monovalent, preferably a fragment antigen-binding (Fab), comprising, or consisting of, a light chain, which comprises, or consists of, an amino acid sequence as shown in one of SEQ ID NO:38, 39, 40, 41, 49, 50 and 51, and a heavy chain, which comprises, or consists of, an amino acid sequence as shown in one of SEQ ID NO:44, 45, 46, 47 and 48.
(75) The antibody or functional fragment of any one of items (1)-(70), which is monovalent, preferably a fragment antigen-binding (Fab), comprising, or consisting of, a light chain, which comprises, or consists of, an amino acid sequence as shown in one of SEQ ID NO:38, 39, 40 and 41, and a heavy chain, which comprises, or consists of, an amino acid sequence as shown in one of SEQ ID NO:44, 45, 46, 47 and 48.
(76) The antibody or functional fragment of any one of items (1)-(70), which is monovalent, preferably a fragment antigen-binding (Fab), comprising, or consisting of, a light chain, which comprises, or consists of, an amino acid sequence as shown in one of SEQ ID NO: 38, 39, and 40, and a heavy chain, which comprises, or consists of, an amino acid sequence as shown in one of SEQ ID NO: 44, 45, and 46.
(77) The antibody or functional fragment of any one of items (1)-(70), which (preferably is monovalent, more preferably a Fab, and) comprises, or consists of, a pair of light chain and heavy chain, having the amino acid sequence as shown in SEQ ID NO:55 and 44, 55 and 45, 55 and 46, 55 and 47, 55 and 48, 56 and 44, 56 and 45, 56 and 46, 56 and 47, 56 and 48, 57 and 44, 57 and 45, 57 and 46, 57 and 47, 57 and 48, 58 and 44, 58 and 45, 58 and 46, 58 and 47, 58 and 48, 52 and 44, 52 and 45, 52 and 46, 52 and 47, 52 and 48, 53 and 44, 53 and 45, 53 and 46, 53 and 47, 53 and 48, 54 and 44, 54 and 45, 54 and 46, 54 and 47, or 54 and 48; preferably 55 and 45, 55 and 46, 55 and 48, 56 and 44, 56 and 45, 56 and 46, 56 and 47, 56 and 48, 57 and 44, 57 and 45, 57 and 46, 52 and 44, 52 and 45, 52 and 46, 53 and 44, 53 and 45, or 53 and 46; more preferably 55 and 45, 56 and 44, 56 and 45, 56 and 46, 57 and 44, 57 and 45, 57 and 46, 52 and 45, 53 and 44, 53 and 45, or 53 and 46; even more preferably 56 and 44, 56 and 45, 56 and 46, 57 and 44, 57 and 45, 57 and 46, 53 and 44, 53 and 45, or 53 and 46; even more preferably 56 and 44, 56 and 45, 56 and 46, 57 and 44, 57 and 45, 53 and 44, or 53 and 45; even more preferably 56 and 44, 56 and 45, 57 and 45, or 53 and 45; even more preferably 56 and 44.
(78) The antibody or functional fragment of any one of items (1)-(70), which (preferably is monovalent, more preferably a Fab, and) comprises, or consists of, a pair of light chain and heavy chain, comprising, or consisting of, the amino acid sequence as shown in SEQ ID NO:55 and 44, 55 and 45, 55 and 46, 55 and 47, 55 and 48, 56 and 44, 56 and 45, 56 and 46, 56 and 47, 56 and 48, 57 and 44, 57 and 45, 57 and 46, 57 and 47, 57 and 48, 58 and 44, 58 and 45, 58 and 46, 58 and 47, or 58 and 48; preferably 55 and 45, 55 and 46, 55 and 48, 56 and 44, 56 and 45, 56 and 46, 56 and 47, 56 and 48, 57 and 44, 57 and 45, or 57 and 46; more preferably 55 and 45, 55 and 46, 55 and 48, 56 and 44, 56 and 45, 56 and 46, 56 and 47, 56 and 48, 57 and 44, 57 and 45, or 57 and 46; even more preferably 55 and 45, 56 and 44, 56 and 45, 56 and 46, 57 and 44, 57 and 45, or 57 and 46; even more preferably 56 and 44, 56 and 45, 56 and 46, 57 and 44, 57 and 45, or 57 and 46; even more preferably 56 and 44, 56 and 45, 56 and 46, 57 and 44, or 57 and 45; even more preferably 56 and 44, 56 and 45, 56 and 46, or 57 and 45; even more preferably 56 and 44, 56 and 45, or 57 and 45, even more preferably 56 and 44.
(79) The antibody or functional fragment of any one of items (1)-(70), which (preferably is monovalent, more preferably a Fab, and) comprises, or consists of, a pair of light chain and heavy chain, having the amino acid sequence as shown in SEQ ID NO:38 and 44, 38 and 45, 38 and 46, 38 and 47, 38 and 48, 39 and 44, 39 and 45, 39 and 46, 39 and 47, 39 and 48, 40 and 44, 40 and 45, 40 and 46, 40 and 47, 40 and 48, 41 and 44, 41 and 45, 41 and 46, 41 and 47, 41 and 48, 49 and 44, 49 and 45, 49 and 46, 49 and 47, 49 and 48, 50 and 44, 50 and 45, 50 and 46, 50 and 47, 50 and 48, 51 and 44, 51 and 45, 51 and 46, 51 and 47, or 51 and 48; preferably 38 and 45, 38 and 46, 38 and 48, 39 and 44, 39 and 45, 39 and 46, 39 and 47, 39 and 48, 40 and 44, 40 and 45, 40 and 46, 49 and 44, 49 and 45, 49 and 46, 50 and 44, 50 and 45, or 50 and 46; more preferably 38 and 45, 39 and 44, 39 and 45, 39 and 46, 40 and 44, 40 and 45, 40 and 46, 49 and 45, 50 and 44, 50 and 45, or 50 and 46; even more preferably 39 and 44, 39 and 45, 39 and 46, 40 and 44, 40 and 45, 40 and 46, 50 and 44, 50 and 45, or 50 and 46; even more preferably 39 and 44, 39 and 45, 39 and 46, 40 and 44, 40 and 45, 50 and 44, or 50 and 45; even more preferably 39 and 44, 39 and 45, 40 and 45, or 50 and 45; even more preferably 39 and 44.
(80) The antibody or functional fragment of any one of items (1)-(70), which (preferably is monovalent, more preferably a Fab, and) comprises, or consists of, a pair of light chain and heavy chain, comprising, or consisting of, the amino acid sequence as shown in SEQ ID NO:38 and 44, 38 and 45, 38 and 46, 38 and 47, 38 and 48, 39 and 44, 39 and 45, 39 and 46, 39 and 47, 39 and 48, 40 and 44, 40 and 45, 40 and 46, 40 and 47, 40 and 48, 41 and 44, 41 and 45, 41 and 46, 41 and 47, or 41 and 48; preferably 38 and 45, 38 and 46, 38 and 48, 39 and 44, 39 and 45, 39 and 46, 39 and 47, 39 and 48, 40 and 44, 40 and 45, or 40 and 46; more preferably 38 and 45, 38 and 46, 38 and 48, 39 and 44, 39 and 45, 39 and 46, 39 and 47, 39 and 48, 40 and 44, 40 and 45, or 40 and 46; even more preferably 38 and 45, 39 and 44, 39 and 45, 39 and 46, 40 and 44, 40 and 45, or 40 and 46; even more preferably 39 and 44, 39 and 45, 39 and 46, 40 and 44, 40 and 45, or 40 and 46; even more preferably 39 and 44, 39 and 45, 39 and 46, 40 and 44, or 40 and 45; even more preferably 39 and 44, 39 and 45, 39 and 46, or 40 and 45; even more preferably 39 and 44, 39 and 45, or 40 and 45, even more preferably 39 and 44.
(81) The antibody or functional fragment of any one of items (1)-(70), which is monovalent, preferably a Fab, consisting of a pair of light chain and heavy chain, having the amino acid sequences as shown SEQ ID NO: 38 and 44, 38 and 45, 38 and 46, 38 and 47, 38 and 48, 39 and 44, 39 and 45, 39 and 46, 39 and 47, 39 and 48, 40 and 44, 40 and 45, 40 and 46, 40 and 47, 40 and 48, 41 and 44, 41 and 45, 41 and 46, 41 and 47, 41 and 48, 49 and 44, 49 and 45, 49 and 46, 49 and 47, 49 and 48, 50 and 44, 50 and 45, 50 and 46, 50 and 47, 50 and 48, 51 and 44, 51 and 45, 51 and 46, 51 and 47, or 51 and 48.
(82) The antibody or functional fragment of any one of items (1)-(70), which is monovalent, preferably a Fab, consisting of a pair of light chain and heavy chain, having the amino acid sequences as shown SEQ ID NO:38 and 45, 38 and 46, 38 and 48, 39 and 44, 39 and 45, 39 and 46, 39 and 47, 39 and 48, 40 and 44, 40 and 45, 40 and 46, 49 and 45, 49 and 46, 49 and 48, 50 and 44, 50 and 45, or 50 and 46.
(83) The antibody or functional fragment of any one of items (1)-(70), which is monovalent, preferably a Fab, consisting of a pair of light chain and heavy chain, having the amino acid sequences as shown SEQ ID NO:38 and 45, 38 and 46, 38 and 48, 39 and 44, 39 and 45, 39 and 46, 39 and 47, 39 and 48, 40 and 44, 40 and 45, or 40 and 46.
(84) The antibody or functional fragment of any one of items (1)-(70), which is monovalent, preferably a Fab, consisting of a pair of light chain and heavy chain, having the amino acid sequences as shown SEQ ID NO: 38 and 45, 39 and 44, 39 and 45, 39 and 46, 40 and 44, 40 and 45, or 40 and 46.
(85) The antibody or functional fragment of any one of items (1)-(70), which is monovalent, preferably a Fab, consisting of a pair of light chain and heavy chain, having the amino acid sequences as shown SEQ ID NO: 39 and 44, 39 and 45, 39 and 46, 40 and 44, 40 and 45, or 40 and 46.
(86) The antibody or functional fragment of any one of items (1)-(70), which is monovalent, preferably a Fab, consisting of a pair of light chain and heavy chain, having the amino acid sequences as shown SEQ ID NO:39 and 44, 39 and 45, 39 and 46, 40 and 44, or 40 and 45.
(87) The antibody or functional fragment of any one of items (1)-(70), which is monovalent, preferably a Fab, consisting of a pair of light chain and heavy chain, having the amino acid sequences as shown SEQ ID NO:39 and 44, 39 and 45, or 40 and 45.
(88) The antibody or functional fragment of any one of items (1)-(70), which is monovalent, preferably a Fab, consisting of a pair of light chain and heavy chain, having the amino acid sequences as shown SEQ ID NO:39 and 44.
(89) The antibody or functional fragment of any one of items (1) to (66), which is monovalent, preferably a Fab, comprising, or consisting of, a pair of light chain and heavy chain, comprising, or consisting of the amino acid sequences as shown SEQ ID NO:37 and 43.
(90) The antibody or functional fragment of any one of the preceding items, which is capable of sustained inhibition of human GPVI in blood plasma, preferably for at least 12 hours, more preferably for at least 24 hours, even more preferably at least 36 hours, even more preferably at least 48 hours, even more preferably at least 60 hours, even more preferably at least 72 hours, even more preferably at least 84 hours, even more preferably at least 96 hours.
(91) The antibody or functional fragment of any one of the preceding items, which is, preferably in monovalent form, e.g. as a Fab, capable of prolonged binding to circulating platelets, preferably with > 50% GPVI receptor epitope occupancy when normalized with a negative control (e.g. control Fab not specific to GPVI), for for at least 12 hours, preferably at least 24 hours, more preferably at least 36 hours, even more preferably at least 48 hours, even more preferably at least 60 hours, after intravenous administration of e.g. 4 mg/kg anti-GPVI antibody or functional fragment, as determined for example by flow cytometric analysis in diluted blood ex *vivo,* for example using competitive binding with a different anti-GPVI antibody or functional fragment that is fluorescently-labeled and using the mean fluorescence intensity (MFI) as a measure of GPVI receptor epitope occupancy, e.g. as described in the examples in section 1.10.
(92) The antibody or functional fragment of any one of the preceding items, which is preferably in monovalent form, e.g. as a Fab, capable of prolonged binding to circulating platelets, preferably with at least 20% GPVI receptor epitope occupancy when normalized with a negative control (e.g. control Fab not specific to GPVI), for at least 36 hours, preferably at least 48 hours, more preferably at least 60 hours, even more preferably at least 72 hours, even more preferably at least 84 hours, even more preferably at least 96 hours, after intravenous administration of e.g. 4 mg/kg anti-GPVI antibody or functional fragment, as determined for example by flow cytometric analysis in diluted blood ex *vivo,* for example using competitive binding with a different anti-GPVI antibody or functional fragment that is a fluorescently-labeled and using the mean fluorescence intensity (MFI) as a measure of GPVI receptor epitope occupancy, e.g. as described in the examples in section 1.10.
(93) The antibody or functional fragment of any one of the preceding items, which, at a concentration of ≤ 10 µg/ml, preferably ≤ 5 µg/ml, more preferably ≤ 2 µg/ml, even more preferably ≤ 1 µg/ml, is capable of complete inhibition of CRP- and/or collagen-induced aggregation of washed human platelets as determined using standard light-transmission aggregometry, for example as described in the examples in section 1.7.
(94) The antibody or functional fragment of any one of the preceding items, which, for at least 12 hours, preferably for at least 24 hours, more preferably at least 48 hours, even more preferably at least 60 hours, after intravenous administration to a mouse model humanized for GPVI (*hGP6*^{*tg*/*tg*} mouse) at a dose of 4 mg/kg, is capable of complete inhibition of CRP- and/or collagen-induced aggregation of washed murine *hGP6*^{*tg*/*tg*} platelets, as determined using standard light-transmission aggregometry, for example as described in the examples in section 1.7.
(95) The antibody or functional fragment of any one of the preceding items, which, at a concentration of 5 µg/ml is capable, in heparinized human blood under flow (preferably using a shear rate of 1000 s-1), of complete inhibition of human platelet adhesion (platelet surface coverage) and thrombus formation (relative thrombus volume) on a collagen coated surface, as determined using a flow adhesion assay, for example as described in the examples in section 1.5; optionally wherein complete inhibition refers to a reduction of platelet surface coverage by at least by at least 60 %, preferably at least 65 %, more preferably at least 70 % and/or a reduction of relative thrombus volume by at least 80 %, preferably at least 85 %, more preferably at least 87 %, even more preferably at least 90 %, compared to a negative control.
(96) The antibody or functional fragment of any one of the preceding items, which at a concentration of ≤ 10 µg/ml, preferably ≤ 5 µg/ml, more preferably ≤ 2 µg/ml, even more preferably ≤ 1 µg/ml, is capable, in heparinized human blood under flow (preferably using a shear rate of 1000 s-1), of reducing, in a flow adhesion assay (for example as described in the examples in section 1.5), the relative thrombus volume on a collagen coated surface (preferably coated at 200 µg/ml), by at least 50 %, preferably by at least 60 %, 65 %, 70 %, 80 %, 85 %, 90 %, or 95 %, compared to a negative control.
(97) The antibody or functional fragment of any one of the preceding items, which, for at least 12 hours, preferably for at least 24 hours, more preferably at least 48 hours, even more preferably at least 60 hours, after intravenous administration to a mouse model humanized for GPVI (*hGP6*^{*tg*/*tg*} mouse) at a dose of 4 mg/kg is capable, in heparinized human blood under flow (preferably using a shear rate of 1000 s⁻¹), of reducing, in a flow adhesion assay (for example as described in the examples in section 1.5), the relative thrombus volume on a collagen coated surface (preferably coated at 200 µg/ml), by at least 50 %, preferably by at least 60 %, compared to a negative control.
(98) The antibody or functional fragment of any one of the preceding items, which, at a concentration of 10 µg/ml, is capable, in citrated and recalcified human blood under flow (preferably using a shear rate of 1000 s-1), of complete inhibition of thrombus formation (relative thrombus volume), phosphatidylserine (PS)-exposure and fibrin deposition, on a surface coated with collagen and tissue factor (preferably at 200 µg/ml and 500 pM for collagen and tissue factor, respectively), as determined in a flow adhesion assay adapted for coagulation (for example as described in the examples in section 1.6); optionally wherein complete inhibition refers to a reduction of relative thrombus volume / phosphatidylserine (PS)-exposure / fibrin deposition by at least 90 %, preferably at least 95 %, compared to a negative control.
(99) The antibody or functional fragment of any one of the preceding items, which, when used at a concentration of 10 µg/ml, in a spreading assay (for example as described in the examples in section 1.8) on washed human platelet that are allowed to bind to a fibrinogen-coated surface, is capable of reducing the portion of phase 4 (fully spread) platelets by at least 40 %, preferably at least 50 %, and increasing the portion of phase 2 (filopodia-forming platelets) by similar extent, compared to a negative control.
(100) The antibody or functional fragment of any one of the preceding items, which, at a concentration of 10 µg/ml, is capable of complete inhibition of human platelet adhesion (platelet surface coverage) and thrombus formation (relative thrombus volume) on a collagen coated surface (preferably coated at 200 µg/ml) in heparinized human blood under flow (preferably using a shear rate of 1000 s⁻¹), determined using a flow adhesion assay, for example as described in the examples in section 1.5; optionally wherein complete inhibition refers to reduction of platelet surface coverage by at least by at least 80 %, preferably at least 85 %, and/or of relative thrombus volume by at least 90 %, preferably at least 95 %, compared to a negative control.
(101) The antibody or functional fragment of any one of the preceding items, which, preferably in monovalent form, preferably as a Fab, causes a significantly impaired CRP-induced activation of circulating platelets for at least 12 hours, at least 24 hours, at least 36 hours, at least 48 hours, at least 60 hours, at least 72 hours, at least 84 hours, or at least 96 hours, after intravenous administration e.g. to a mouse model humanized for GPVI (*hGP6*^{*tg*/*tg*} mouse) at a dose of 4 mg/kg, as determined for example by flow cytometric analysis in diluted blood ex *vivo,* preferably using a fluorescently labeled antibody capable of specifically binding activated integrin αllbβ3 and/or a fluorescently labeled antibody capable of specifically binding P-selectin, e.g. as described in the examples in section 1.10; wherein "significantly impaired" preferably means a reduction of fluorescent signal of >50% when using circulating platelets sampled at least 12 hours, at least 24 hours, at least 36 hours, at least 48 hours, at least 60 hours, at least 72 hours, at least 84 hours, or at least 96 hours post-administration, compared to a negative control.
(102) The antibody or functional fragment of any one of the preceding items, which preferably is monovalent, preferably a Fab, and is capable of inhibiting dimerization of GPVI, optionally while not or not completely abolishing ligand binding to GPVI.
(103) The antibody or functional fragment of any one of the preceding items, which is monovalent, preferably a Fab, and is capable of inhibiting GPVI-induced activation of platelets, optionally while leaving the initial adhesive functions of the GPVI receptor intact.
(104) The antibody or functional fragment of any one of the preceding items, which is capable of sustained protection from occlusive thrombosis, e.g. arterial thrombosis, preferably as determined using a mouse model of occlusive arterial thrombus formation that is humanized for GPVI (*hGP6*^{*tg*/*tg*} mouse), as described e.g. in the examples in section 1.11.
(105) The antibody or functional fragment of any one of the preceding items, which is capable of inhibiting the formation of stable thrombi at sites of arterial injury, preferably through reduced platelet activation at a site of exposed extracellular matrix inside a blood vessel and/or by potent inhibition of local platelet-dependent coagulation, optionally as determined using a mouse model of occlusive arterial thrombus formation that is humanized for GPVI (*hGP6*^{*tg*/*tg*} mouse), as described e.g. in the examples in section 1.11.
(106) The antibody or functional fragment of any one of the preceding items, wherein the antibody or functional fragment, compared to ACT017 (glenzocimab), has a significantly greater potency to inhibit GPVI function i) as determined by an aggregometry assay using standard light-transmission aggregometry, for example as described in the examples in section 1.7; ii) as determined using heparinized human blood under flow (preferably using a shear rate of 1000 s-1), in a flow adhesion assay (for example as described in the examples in section 1.5); and/or iii) as determined 1 hour after intravenous administration, based on CRP-induced activation of circulating platelets by flow cytometric analysis in diluted blood ex *vivo* using a fluorescently labeled antibody capable of specifically binding activated integrin αllbβ3.
(107) The antibody or functional fragment of any one of the preceding items, which is, in monovalent form, preferably as a Fab, capable of increased binding to circulating platelets (preferably with a ≥ 1.5-fold, more preferably ≥ 1.7-fold, ≥ 1.8-fold, or ≥ 1.9-fold, increase in GPVI receptor epitope binding) compared to ACT017 (glenzocimab), when normalized with a negative control (e.g. control Fab not specific to GPVI), as measured, 1 hour after intravenous administration of e.g. 4 mg/kg anti-GPVI antibody or functional fragment or ACT017 (glenzocimab), as determined for example by flow cytometric analysis in diluted blood ex *vivo,* e.g. with a fluorescently-labeled Fab specific antibody and using the mean fluorescence intensity (MFI) as a measure of GPVI receptor epitope binding, e.g. as described in the examples in section 1.10.
(108) The antibody or functional fragment of any one of the preceding items, which is, in monovalent form, preferably as a Fab, capable of increased binding to circulating platelets, (preferably with a ≥ 2-fold, more preferably ≥ 2.5-fold, ≥ 3-fold, ≥ 3.5-fold, or ≥ 3.75-fold, increase in GPVI receptor epitope binding) compared to compared to ACT017 (glenzocimab), when normalized with a negative control (e.g. control Fab not specific to GPVI), as measured, 3 hour after intravenous administration of e.g. 4 mg/kg anti-GPVI antibody or functional fragment or ACT017, as determined for example by flow cytometric analysis in diluted blood ex *vivo,* e.g. with a fluorescently-labeled Fab specific antibody and using the mean fluorescence intensity (MFI) as a measure of GPVI receptor epitope binding, e.g. as described in the examples in section 1.10.
(109) The antibody or functional fragment of any one of the preceding items, which, preferably in monovalent form, more preferably as a Fab, results in a significantly impaired CRP-induced activation of circulating platelets 1 hour after intravenous administration e.g. to a mouse model humanized for GPVI (*hGP6*^{*tg*/*tg*} mouse) at a dose of 4 mg/kg, as determined for example by flow cytometric analysis in diluted blood ex *vivo,* preferably using a fluorescently labeled antibody capable of (specifically) binding activated integrin αllbβ3, e.g. as described in the examples in section 1.10 (while ACT017 (glenzocimab) does not causes a significantly impaired CRP-induced activation); optionally wherein "significantly impaired" means a reduction of fluorescent signal of >85%, preferably >90%, >95%, >97%, >98%, >99%, or >99.5%, when using circulating platelets sampled 24 hours, post-administration, compared to a negative control.
(110) The antibody or functional fragment of any one of the preceding items, wherein, compared to ACT017 (glenzocimab), the antibody or functional fragment, preferably at a concentration of 10 µg/ml, preferably 5 µg/ml, more preferably 1 µg/ml, has a > 2-fold, preferably > 4-fold, > 6-fold, > 8-fold, or > 10-fold higher potency to inhibit GPVI function in human platelets *in vitro* as measured using standard light-transmission aggregometry, for example as described in the examples in section 1.7, optionally wherein the fold potency is determined as the ratio of percentage maximum aggregation of the antibody or functional fragment treated sample to the percentage maximum aggregation of ACT017 treated sample.
(111) The antibody or functional fragment of any one of the preceding items, wherein, at a concentration of 10 µg/ml, preferably 5 µg/ml, more preferably 1 µg/ml, the antibody or functional fragment, compared to ACT017 (glenzocimab), has a > 2-fold, preferably greater than > 4-fold, > 6-fold, > 8-fold, or > 10-fold higher potency to inhibit collagen- and/or CRP-induced aggregation of washed human platelets *in vitro* as measured using standard light-transmission aggregometry, for example as described in the examples in section 1.7, optionally wherein the fold potency is determined as the ratio of percentage maximum aggregation of the antibody or functional fragment treated sample to the percentage maximum aggregation of ACT017 treated sample.
(112) The antibody or functional fragment of any one of the preceding items, which at a concentration of 10 µg/ml, preferably 5 µg/ml, more preferably 1 µg/ml, is capable, in heparinized human blood under flow (preferably using a shear rate of 1000 s-1), of significantly reducing (e.g. by greater than 1.5-fold, preferably greater than 2-fold, 2.5-fold, or 3-fold), in a flow adhesion assay (for example as described in the examples in section 1.5), the platelet surface coverage on a collagen coated surface (preferably coated at 200 µg/ml), compared to a negative control (while a ACT017 (glenzocimab) treated sample does not result in a (significant) reduction).
(113) The antibody or functional fragment of any one of the preceding items, which at a concentration of 10 µg/ml, preferably 5 µg/ml, more preferably 1 µg/ml, is capable, in heparinized human blood under flow (preferably using a shear rate of 1000 s-1), of reducing, in a flow adhesion assay (for example as described in the examples in section 1.5), the platelet surface coverage on a collagen coated surface (preferably coated at 200 µg/ml), by greater than 1.5-fold, preferably greater than 2-fold, 2.5-fold, or 3-fold, compared to a ACT017 (glenzocimab) treated sample.
(114) The antibody or functional fragment of any one of the preceding items, which, at a concentration of 10 µg/ml, preferably 5 µg/ml, more preferably 1 µg/ml, is capable, in heparinized human blood under flow (preferably using a shear rate of 1000 s-1), of reducing, in a flow adhesion assay (for example as described in the examples in section 1.5), the relative thrombus volume on a collagen coated surface (preferably coated at 200 µg/ml), by greater than 2-fold, preferably greater than 4-fold, 6-fold, 8-fold, or 10-fold, compared to a ACT017 (glenzocimab) treated sample.
(115) An antibody or functional fragment thereof binding to essentially the same epitope on human GPVI as an antibody exhibiting one or more of the features referred to in items (1) to (114) herein above.
(116) A nucleic acid encoding the antibody or functional fragment of any one of the preceding items.
(117) A vector or plasmid comprising the nucleic acid of item (116).
(118) A cell comprising the nucleic acid of item (116) or the vector or plasmid of item (117).
(119) A method of preparing the antibody or functional fragment of any one of items (1) to (115), comprising culturing the cell of item (118) in a medium under conditions that allow expression of the nucleic acid encoding the antibody or functional fragment, and recovering the antibody or functional fragment from the cells or from the medium.
(120) The method of item (119), wherein the cell is a Chinese Hamster Ovary (CHO) cell and the nucleic acid, codon optimized for expression in CHO cells, is transiently expressed from a vector or plasmid, and wherein the recovery involves affinity chromatography.
(121) A pharmaceutical composition comprising the antibody or functional fragment of any one of items (1) to (115), and optionally a pharmaceutically acceptable carrier and/or excipient.
(122) The antibody or functional fragment as defined in any one of items (1) to (115) for use in a method of treating or preventing a GPVI-related condition in a subject.
(123) The antibody or functional fragment for use according to item (122), wherein the GPVI-related condition is a thrombo-inflammatory disease.
(124) The antibody or functional fragment for use according to item (122), wherein the GPVI-related condition is a cardiovascular disease.
(125) The antibody or functional fragment for use according to item (123) or (124), wherein the cardiovascular disease is selected from thrombosis and thrombotic disorder (such as arterial thrombosis, venous thrombosis, atherothrombosis, stent thrombosis, venous thromboembolism diseases, thrombotic occlusion of coronary arteries, thrombotic microangiopathies, cancer-associated thrombosis (Trousseau syndrome), immunothrombosis and thrombosis associated to infection [e.g. cerebral malaria]), restenosis, acute coronary syndrome, ischemic cerebrovascular accidents, cerebral vascular diseases, and vascular purpura, coronary artery diseases and cerebral artery diseases, ischemic events, acute coronary artery syndrome, myocardial infarction (heart attack), acute cerebrovascular ischemia (stroke), percutaneous coronary intervention, ischemic restenosis, acute ischemia, chronic ischemia, diseases of the aorta and its branches (such as aortic aneurysm, thrombosis), peripheral artery disease, acute phlebitis, and pulmonary embolism.
(126) The antibody or functional fragment for use according to item (123) or (124), wherein the cardiovascular disease is thrombosis or a thrombotic disorder, preferably selected from arterial thrombosis, venous thrombosis, atherothrombosis, stent thrombosis, venous thromboembolism diseases, thrombotic occlusion of coronary arteries, thrombotic microangiopathies, cancer-associated thrombosis (Trousseau syndrome), immunothrombosis and thrombosis associated to infection [e.g. cerebral malaria].
(127) The antibody or functional fragment for use according to item (124), wherein the cardiovascular disease is selected from arterial or venous thrombosis, restenosis, acute coronary syndrome, or cerebrovascular accidents due to atherosclerosis, preferably arterial or venous thrombosis.
(128) The antibody or functional fragment for use according to item (122) or (123), wherein the GPVI-related condition is inflammation or thrombo-inflammation, preferably selected from sustained or prolonged inflammation associated with infection [e.g. cerebral malaria], arthritis, autoimmune diseases [such as celiac disease, post-infectious IBS, diabetes mellitus type 1, Henoch-Schönlein purpura (HSP) sarcoidosis, systemic lupus erythematosus (SLE), Sjögren syndrome, eosinophilic granulomatosis with polyangiitis, Hashimoto's thyroiditis, Graves' disease, idiopathic thrombocytopenic purpura, Addison's disease, rheumatoid arthritis (RA), ankylosing spondylitis, polymyositis (PM), dermatomyositis (DM), Alopecia Areata and multiple sclerosis (MS)], fibrosis, acute respiratory distress syndrome (ARDS), ischemia-reperfusion injury (IRI) of various organs (liver, colon, etc.), peripheral vascular disease, antiphospholipid syndrome (APS), deep vein thrombosis, thrombophlebitis & vasculitis, transfusion-related acute lung injury (TRALI), transplant rejection, pre-eclampsia, severe burns, atherosclerosis, hypertension, antiphospholipid syndrome, sickle cell disease, bacterial and viral infection ischemic restenosis, sepsis, major trauma, and disorders in which platelets modulate cell functions including, without limitation, cancer cells proliferation and/or dissemination.
(129) The antibody or functional fragment for use according to item (122), wherein the GPVI-related condition is cancer, preferably skin cancer (in particular malignant cutaneous melanoma), colon cancer, breast cancer, ovarian cancer, lung cancer, or metastatic cancer.
(130) The antibody or functional fragment for use according to item (122), wherein the GPVI-related condition is a disorder associated with abnormal or aberrant megakaryocyte and/or platelet proliferation, differentiation, morphology, migration, aggregation, degranulation and/or function.
(131) The antibody or functional fragment for use according to any of items (122)-(130), wherein the subject is a human subject.
(132) The antibody or functional fragment for use according to any one of items (122) to (131), wherein said method comprises intravenous administering the antibody or functional fragment to a human subject.
(133) A method of treating or preventing a GPVI-related condition, comprising administering to a patient in need thereof an effective amount of the anti-GPVI antibody or functional fragment thereof as defined in any of items (1) to (115) or the pharmaceutical composition of item (121), preferably wherein the GPVI-related condition one specified in items (123) to (130).

### BRIEF DESCRIPTION OF THE DRAWINGS

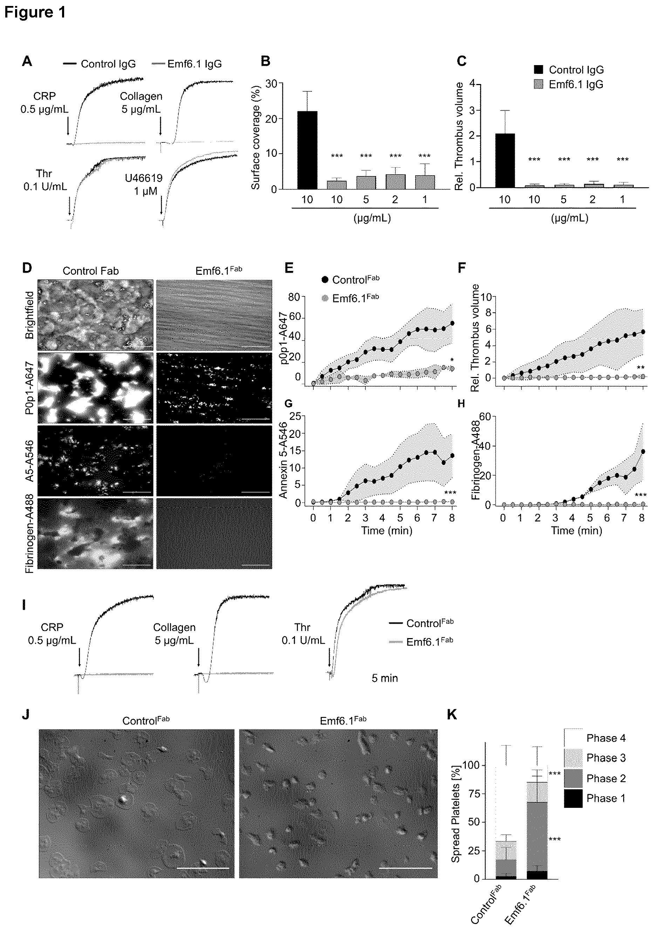

**Figure 1****: Emf6.1^{Fab} inhibits GPVI-induced aggregation, thrombus formation and spreading of human platelets.** (A) Aggregation responses of washed human platelets treated with 10µg/mL Emf6.1 IgG or control IgG in turbidimetric aggregometry. Grey arrows indicate the addition of the agonist. (B-C) Assessment of platelet adhesion (B) and aggregate formation (C) on Horm collagen (200 µg/mL) under flow (1000 s⁻¹) in heparinized human blood treated with 10 µg/mL Emf6.1 IgG or control IgG. (D-H) Assessment of platelet adhesion (E), thrombus formation (F), phosphatidylserine-exposure (G) and fibrin deposition (H) on Horm collagen (200 µg/mL) plus tissue factor (500 pM) under flow (1000 s⁻¹) in recalcified human blood treated with 10 µg/mL Emf6.1^{Fab} or control Fab. Values are mean ± SD (n = 4). Unpaired, Mann-U-Whitney test. **P<0.01, ***P<0.001. (D) Representative images are shown, scale 50 µm. (I) Aggregation responses of washed human platelets treated with 10 µg/mL Emf6.1^{Fab} or control Fab in light-transmission aggregometry (n = 4). (J-K) Washed human platelets were allowed to spread on fibrinogen (100 µg/mL) for 45 min at 37°C. DIC pictures were taken (100x objective) (J) and phase abundance was determined. Phase 1: adhesion, phase 2: filopodia formation; phase 3: lamellipodia formation; phase 4: fully spread platelet (K). Values are mean ± SD (n = 8); Unpaired, two-tailed Student's t-test ***P<0.001.

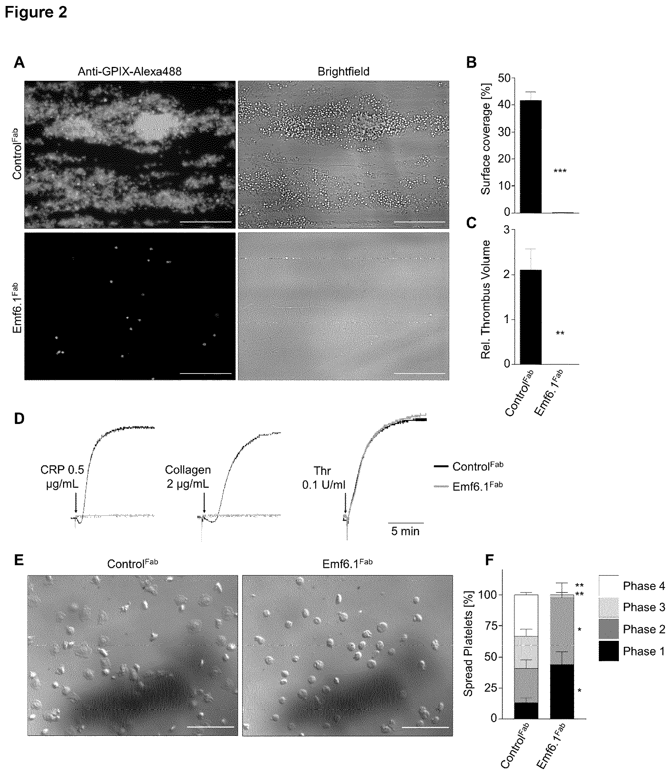

**Figure 2****: Emf6.1^{Fab} inhibits GPVI-induced aggregation, thrombus formation and spreading of *hGP6*^{*tg*/*tg*} platelets.** (A-C) Assessment of platelet adhesion (B) and aggregate formation (C) on Horm collagen (200 µg/mL) under flow (1000 s⁻¹) in heparinized *hGP6*^{*tg*/*tg*} blood treated with 5 µg/mL of either Emf6.1^{Fab} or control Fab. Values are mean ± SD (n = 5). Unpaired, Mann-U-Whitney test. **P<0.01, ***P<0.001. (A) Representative images are shown, scale 50µm. (D) Aggregation responses of washed *hGP6*^{*tg*/*tg*} platelets treated with either 10 µg/mL Emf6.1^{Fab} or control Fab in light-transmission aggregometry (n = 5); grey arrows indicate the addition of the agonist. (E-F) Washed *hGP6*^{*tg*/*tg*} platelets were allowed to spread on fibrinogen (100 µg/mL) for 45 min at 37°C. DIC pictures were taken (100x objective, scale 30 µm) (E) and phase abundance was determined. Phase 1: adhesion, phase 2: filopodia formation; phase 3: lamellipodia formation; phase 4: fully spread platelet (F). Values are mean ± SD (n = 3); Unpaired, Mann-U-Whitney test. *P<0.05, **P<0.01.

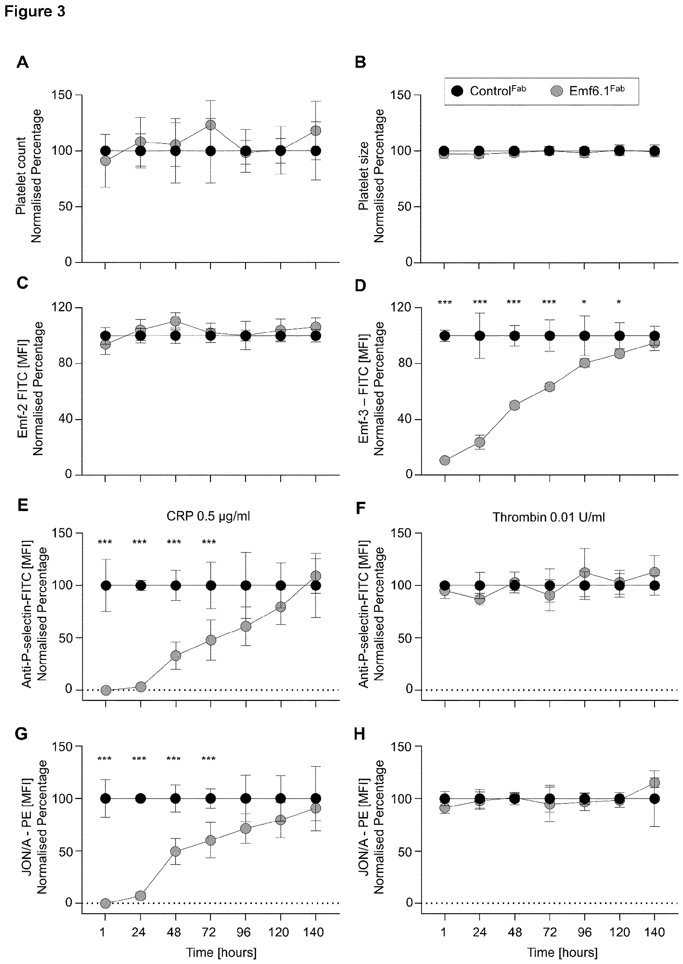

**Figure 3****: Emf6.1^{Fab} efficiently blocks GPVI function in *hGP6*^{*tg*/*tg*} mice.** *hGP6*^{*tg*/*tg*} animals (n=5) were treated with 4 mg/kg b.w. Emf6.1^{Fab} or control Fab. (A-B) Platelet count (A) and size (B) were determined at the indicated time points after treatment using an automated cell counter. (C-D) GPVI exposure was tested using Emf2 IgG-FITC (C), whereas epitope saturation by Emf6.1 was tested using Emf3 IgG-FITC, (D). (E-H) Degranulation (α-P-selectin-FITC) (E-F) and activation of platelet αllbβ3 integrin (JON/A-PE) (G, H)) in response to CRP (E, G) or thrombin (F, H) was determined at the indicated time points after treatment by flow cytometry. Data are expressed as mean ± SD; significance is expressed as * p< 0.05, *** p < 0.001, vs. indicated group (two-way Anova) (n = 5).

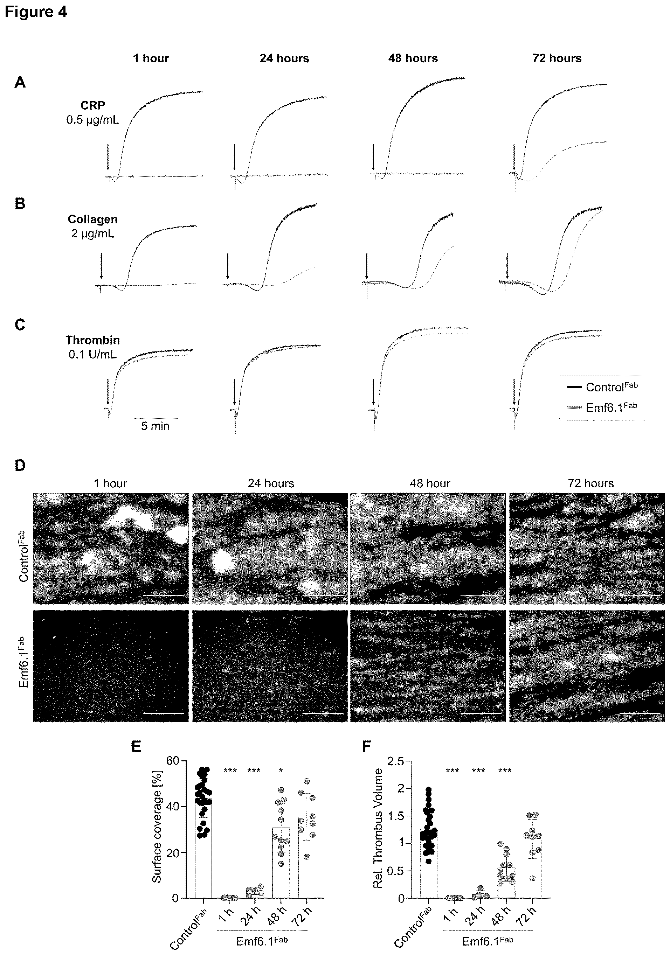

**Figure 4****: Sustained GPVI inhibition by Emf6.1^{Fab} *in vivo.*** (A-C) Aggregation responses of washed platelets from *hGP6*^{*tg*/*tg*} mice intravenously treated with 4 mg/kg b.w. Emf6.1^{Fab} or control Fab to CRP (A), collagen (B) or thrombin (C) in light-transmission aggregometry (n = 6); arrows indicate addition of the agonist. (D-F) Assessment of platelet adhesion (E) and aggregate formation (F) on Horm collagen (200 µg/mL) under flow (1000 s⁻¹) in heparinized blood from *hGP6*^{*tg*/*tg*} mice that had received 4 mg/kg b.w. Emf6.1^{Fab} or control Fab at different time points after injection. Values are mean ± SD. Two-way Anova *P<0.05, ***P<0.001.

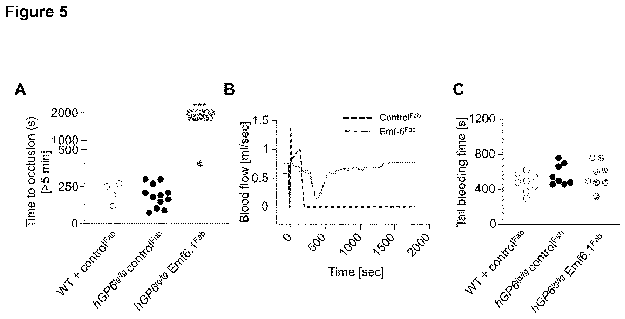

**Figure 5****: Emf6.1^{Fab} treatment (4 mg/kg b.w.) is highly protective in a model arterial thrombosis without affecting bleeding times.** (A-B). Arterial thrombosis was assessed for a maximum period of 30 min after control Fab or Emf6.1^{Fab}-treatment in the mechanical injury of the aorta thrombosis model. (A) Time to vessel occlusion is depicted with each circle representing one animal. ***P < 0.001 using Fisher's exact test. (B) Representative blood flow traces are shown from control Fab and Emf6.1^{Fab} treated mice. (C) Hemostasis was assessed using the tail bleeding time with each circle representing one mouse.

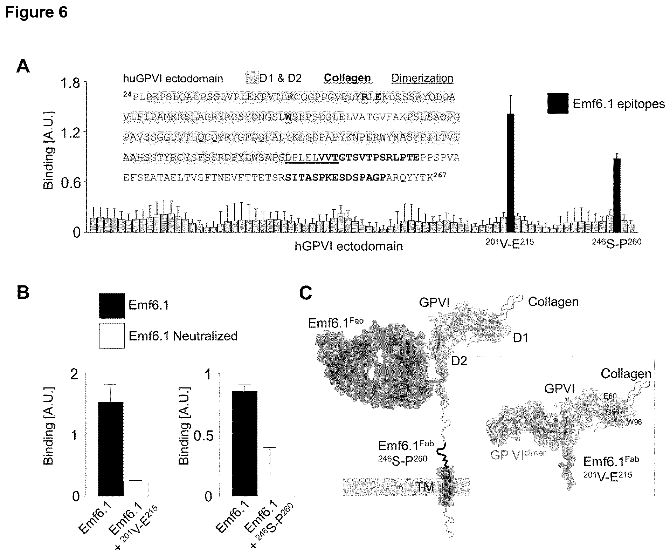

**Figure 6****: Emf6.1 binds a novel GPVI epitope. Mapping of the Emf6.1 binding epitope in the human GPVI ectodomain.** (A) 15-mer overlapping peptides covering the ectodomain (residues 24 to 267) of human GPVI were displayed in peptide-microarray format. Emf6.1 IgG binding was visualized by HRP-labelled anti-mouse IgG secondary antibody. Mapping identified two distinct epitopes 201V-E215 and 246S-P260. (B) Emf6.1 binding is neutralized in presence of the soluble GPVI peptide fragments 201V-E215 and 246S-P260. (C) Surface representation of human GPVI (PDB-ID 2gi7). The Emf6.1 binding epitope overlaps with the proposed dimerization site (D2 region) of GPVI but not the ligand binding site (shown for collagen, PDB-ID 5ou9).

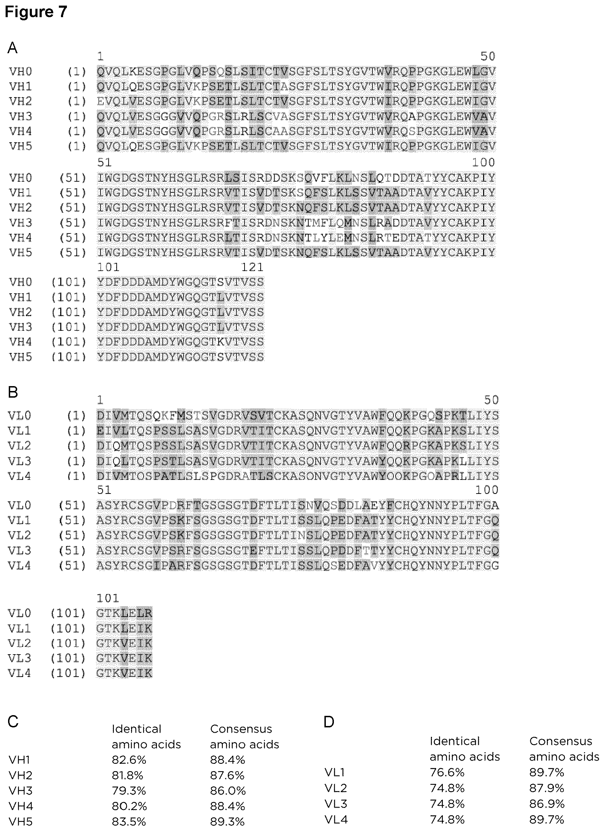

**Figure 7****: Alignment of the humanized variants.** (A-B) Emf6.1 VL sequence (VL0) has been aligned with the four humanized VL variants (A), and Emf6.1 VH sequence (VH0) has been aligned with the five humanized VH variants (B). VL0 is the murine sequence and VL1-4 are the humanized variants; VH0 is the murine sequence and VH1-5 are the humanized variants. The CDRs are underlined. Key residues important for the VH/VL interface and canonical loop structure have been maintained as much as possible in the humanized variants using the CDRx platform. (C) Humanized variants homology to murine V_{L} (i.e. to VL0). (D) Humanized variants homology to murine V_{H} (i.e. to VH0).

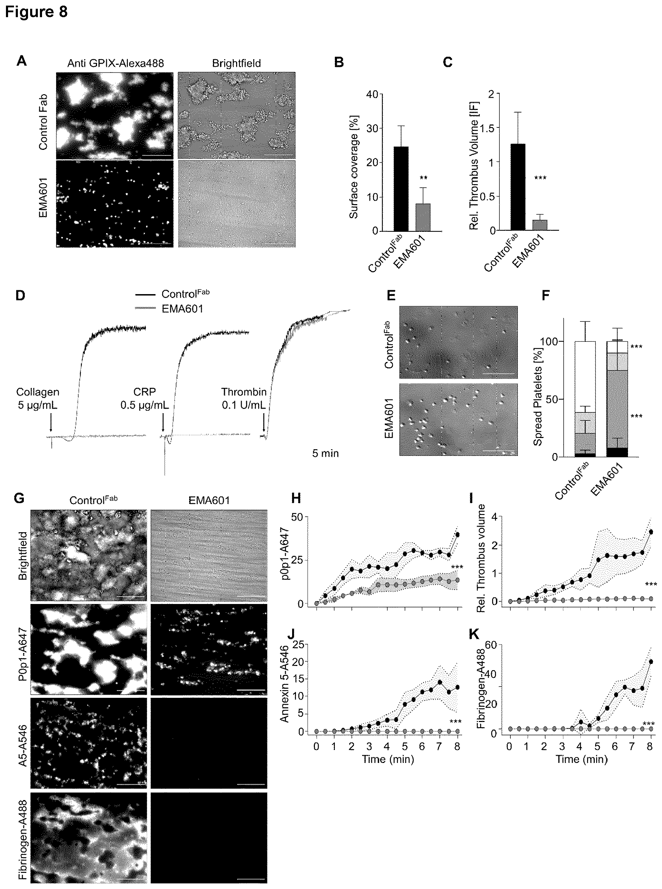

**Figure 8****: EMA601 inhibits GPVI function.** (A-C) Assessment of platelet adhesion (B) and aggregate formation (C) on Horm collagen (200 µg/mL) under flow (1000 s⁻¹) in heparinized *hGP6*^{*tg*/*tg*} blood treated with 5 µg/mL EMA601 or control Fab. Values are mean ± SD (n = 3). Unpaired, Mann-U-Whitney test *P<0.05, ** P<0.01. (A) Representative images are shown, scale 50 µm. (D) Aggregation responses of washed *hGP6*^{*tg*/*tg*} platelets treated with either 5 µg/mL EMA601 or control Fab in light-transmission aggregometry (n = 3), arrows indicate the addition of the agonist. (E-F) Washed *hGP6*^{*tg*/*tg*} platelets were allowed to spread on fibrinogen (100 µg/mL) for 45 min at 37°C. DIC pictures were taken (100x objective, scale 30 µM) (E) and phase abundance was determined. Phase 1: adhesion, phase 2: filopodia formation; phase 3: lamellipodia formation; phase 4: fully spread platelet (F). Values are mean ± SD (n = 3); Unpaired, Mann-U-Whitney test *P<0.05, **P<0.01. (G-K) Assessment of platelet adhesion (H), thrombus formation (I), phosphatidylserine exposure (J) and fibrin deposition (K) on Horm collagen (200 µg/mL) plus tissue factor (500 pM) under flow (1000 s⁻¹) in recalcified human blood treated with 10 µg/mL EMA601 (grey) or control Fab (black). Values are mean ± SD (n = 4). Unpaired, Mann-U-Whitney test. ***P<0.001. (G) Representative images are shown, scale: 50 µm.

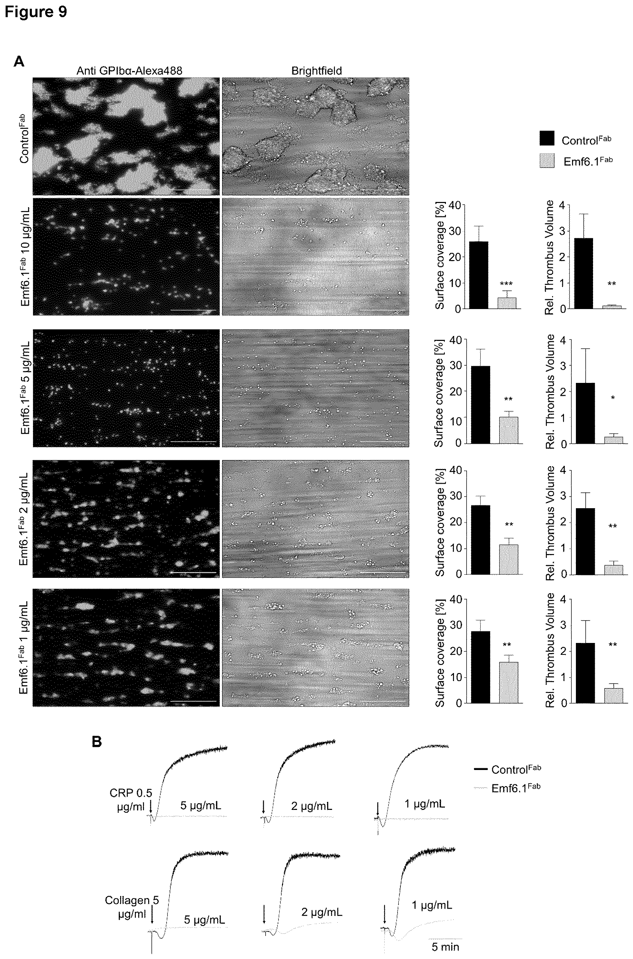

**Figure 9****: Emf6.1^{Fab} inhibits GPVI-induced activation and aggregation of human platelets.** (A) Assessment of platelet adhesion (bar graphs next to the images) and aggregate formation (bar graphs on the right-handed side) on Horm collagen (200 µg/mL) under flow (1000 s⁻¹) in heparinized human blood treated either with 10 µg/mL (2^{nd} row), 5 µg/mL (3^{rd} row), 2 µg/mL (4^{th} row) or 1 µg/mL (bottom row) Emf6.1^{Fab} or a control Fab (top row). Values are mean ± SD (n = 4). Unpaired, Mann-U-Whitney test. *P<0.05, **P<0.001. Representative images are shown on the left-handed side, scale 50 µm. (B) Aggregation responses of washed human platelets treated with 5, 2 or 1 µg/mL Emf6.1^{Fab} or control Fab in light-transmission aggregometry (n = 4).

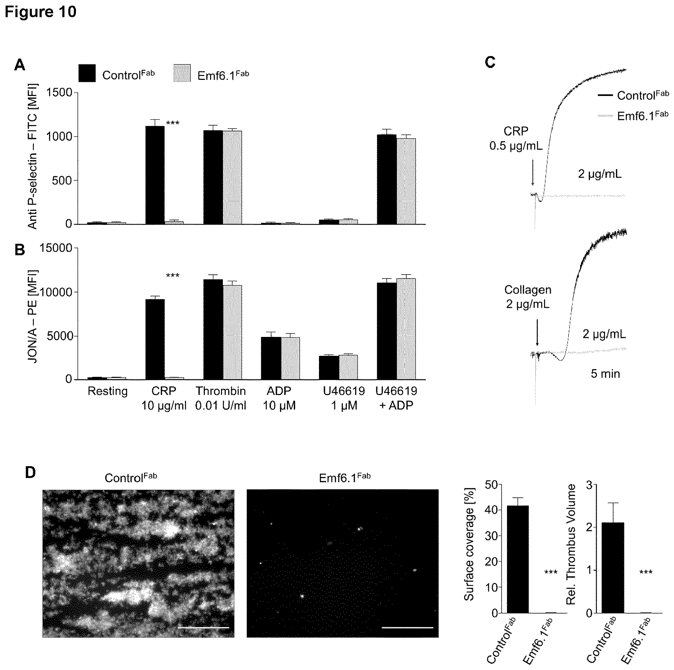

**Figure 10****: Emf6.1^{Fab} inhibits GPVI-induced activation and aggregate formation of *hGP6*^{*tg*/*tg*} platelets.** (A-B) Degranulation (α-P-selectin-FITC) (A) and activation of platelet αllbβ3 integrin (JON/A-PE) (B) in *hGP6*^{*tg*/*tg*} platelets treated with 10 µg/mL Emf6.1^{Fab} or control^{Fab} was determined by flow cytometry upon activation with the indicated agonists (n = 5). Unpaired, Mann-U-Whitney test. ***P<0.001. (C) Aggregation responses of washed *hGP6*^{*tg*/*tg*} platelets preincubated with either 2 µg/mL Emf6.1^{Fab} or control^{Fab} in light-transmission aggregometry (n = 4). Arrows indicate the addition of the agonist. (D) Platelet adhesion and aggregate formation on Horm collagen (200 µg/mL) under flow (1000 s⁻¹) in heparinized *hGP6*^{*tg*/*tg*} blood treated either with 2 µg/mL Emf6.1^{Fab} or control^{Fab}. Left: Representative images are shown, scale 50 µM. Right: Data for surface area coverage and thrombus volume at the end of the perfusion are presented as mean ± SD (n = 4). Unpaired, Mann-U-Whitney test ***P<0.001.

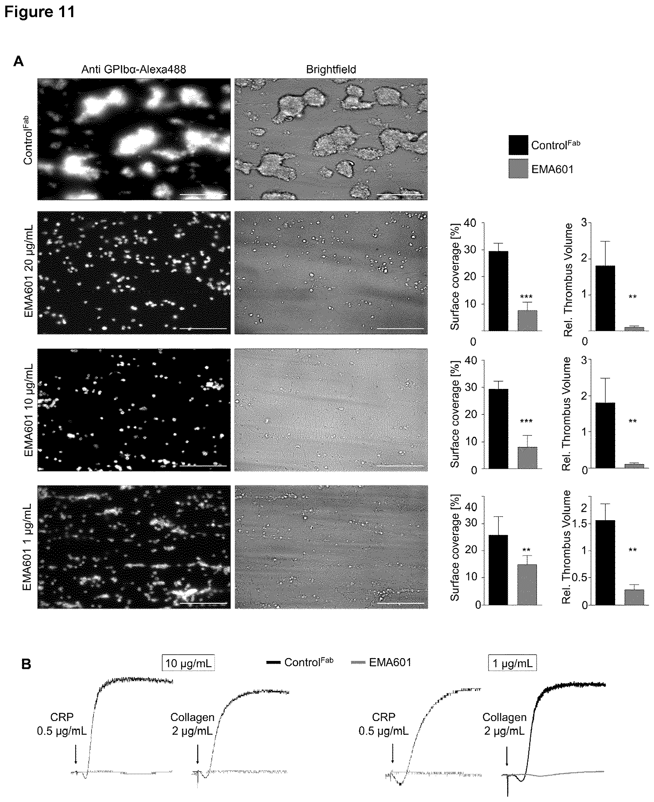

**Figure 11****: EMA601 inhibits GPVI function in human platelets** (A) Assessment of platelet adhesion and aggregate formation on Horm collagen (200 µg/mL) under flow (1000 s⁻¹) in heparinized human blood preincubated with control^{Fab} (10 µg/mL) or EMA601 (20, 10 or 1 µg/mL). Left: Representative fluorescence and brightfield images at the end of the experiment are shown, scale 50 µM. Right: Data for surface area coverage and thrombus volume at the end of the perfusion are presented as mean ± SD (n = 3). Unpaired, Mann-U-Whitney test. *P<0.05, **P<0.01. (B) Aggregation responses of washed human platelets preincubated for 10 min with 10 µg/mL (left) or 1 µg/mL (right) EMA601 or control^{Fab} in light-transmission aggregometry (n = 4).

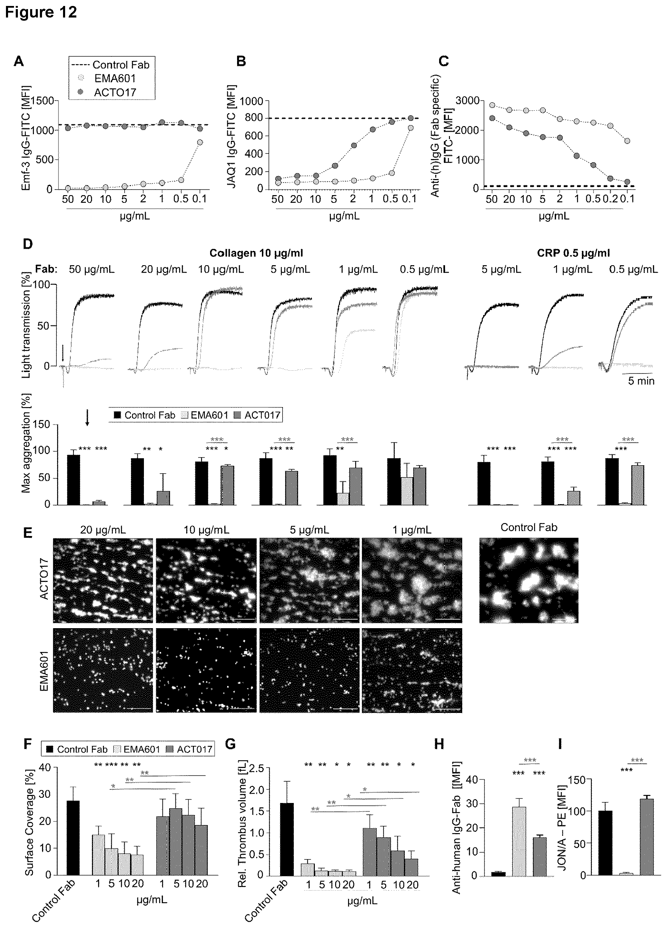

**Figure 12****: Head-to-head comparison of EMA601 and ACT017 (Glenzocimab) *in vitro* and** *in vivo.* (**A-B**) Diluted heparinized blood of *hGP6*^{*tg*/*tg*} mice was pre-incubated with the indicated concentrations of either EMA601 or ACT017. Epitope saturation was tested by flow cytometry using (**A**) Emf3^{FITC} for EMA601 and (**B**) JAQ1^{FITC} for ACT017, respectively (n=4). (**C**) Diluted heparinized blood of *hGP6*^{*tg*/*tg*} mice was pre-incubated with the indicated concentrations of either EMA601 or ACT017 and bound the Fabs were detected using a fluorescently labelled anti-human IgG-Fab antibody (n=4). (**D-E**) Aggregation traces and quantification of washed human platelets pre-treated with the indicated concentrations of EMA601 or ACT017 and stimulated using collagen or CRP (**D**). (**E-G**) Assessment of platelet adhesion (**F**) and aggregate formation (**G**) on Horm collagen (200 µg/mL) under flow (1000 s⁻¹) in heparinized human blood treated with the indicated concentrations of EMA601, ACT017 or control Fab. Scale bar 50 µm. (**H-I**) *hGP6*^{*tg*/*tg*} mice (n=3) were treated with 4 mg/kg b.w. EMA601, ACT017 or control Fab intravenously (n=3). One hour after treatment, bound Fab was detected by flow cytometry ex vivo using a fluorescently labelled anti-human IgG-Fab antibody (**H**), while the activation of platelet αllbβ3 integrin in response to 0.5 µg/mL CRP was measured using JON/APE (**I**). Data are expressed as means ± SD; significance is expressed as * p <0.05, ** p <0.01, *** p <0.001, vs. indicated group (Ordinary One-Way-Anova).

### DETAILED DESCRIPTION

The present invention pertains to an antibody or a functional fragment thereof capable of binding to human GPVI. In the context of the present application, the term "antibody" is used as a synonym for "immunoglobulin" (Ig), which is defined as a protein belonging to the class IgG, IgM, IgE, IgA, or IgD (or any subclass thereof), and includes all conventionally known antibodies and functional fragments thereof. In the context of the present invention, a "functional fragment" of an antibody/immunoglobulin, also referred to as "functional antibody fragment", is defined as antigen-binding fragment, or other derivative of a parental antibody that essentially maintains one or more of the properties of such parental antibody referred to in items (1) to (114) herein above.

An "antigen-binding fragment" of an antibody/immunoglobulin is defined as a fragment (e.g. a variable region of an IgG) that retains the antigen-binding region. An "antigen-binding region" of an antibody typically is found in one or more hypervariable region(s) of an antibody, i.e. the CDR-1, -2, and/or -3 regions. "Antigen-binding fragments" of the invention include a Fab fragment, a F(ab')₂ fragment and a F(ab') fragment. "Functional fragments" of the invention include, scFv, Fv, dsFv, monovalent IgG, diabodies, triabodies, tetrabodies and Fc fusion proteins. The antibodies or functional fragments of the present invention may be part of bi- or multifunctional constructs.

A "Fab", also referred to as "F(ab)", "fragment antigen-binding", or "Fab fragment", as used herein refers to a monovalent antigen-binding fragment, which comprises both a heavy and a light chain, each comprising a variable domain and a constant domain (that is a variable heavy domain ("V_{H}") + constant heavy 1 domain ("C_{H}1") and variable light domain ("V_{L}") + constant light domain ("C_{L}"), but lacks the fragment crystallizable region (Fc region). In general, the light and heavy chain in a Fab are linked by a disulfide bond. The Fab may comprise additional N- and/or C-terminal sequences, e.g. an N-terminal signal peptide and/or C-terminally part of a hinge region, however, not including such residues of a hinge region involved in forming disulfide bonds.

A "F(ab')₂", also referred to as "F(ab')2 fragment", is divalent, comprising two antigen-binding regions (i.e. two fragment antigen-binding region), and retains a portion of the hinge region over which they are linked by one or more disulfide bonds. (Fab')₂ usually lacks most but not all of the Fc region. Reduction of a F(ab')₂ fragment produces two monovalent Fab' fragments, also referred to as "F(ab') fragment". Since Fab' is derived from F(ab')₂, it may contain a small portion of the Fc region. The F(ab')₂, F(ab') or Fab may be engineered to minimize or completely remove the intermolecular disulfide interactions that occur between the C_{H}1 and C_{L} domains.

A "Fv" fragment comprises two chains one comprising a V_{H} region and one the V_{L} region making up the antigen-binding site, but it lacks the constant regions of Fab (C_{H}1 and C_{L}), and no disulfide bond linking the two chains. A "monovalent IgG" preferably refers to a functional fragment comprising only one full-length light chain and one full-length heavy chain, and thus one fragment antigen-binding region. A "dsFv" is a disulfide-linked variable fragment, comprising a V_{H}, and V_{L} chain which are linked by a constructed interchain disulfide bond. A "scFv" is a single chain Fv fragment in which the variable light ("V_{L}") and variable heavy ("V_{H}") domains are linked by a peptide bridge.

A "diabody" is a dimer consisting of two fragments, each having variable regions joined together via a linker or the like (hereinafter referred to as diabody-forming fragments), and typically contain two V_{L}s and two V_{H}s. Diabody-forming fragments include those consisting of V_{L} and V_{H}, V_{L} and V_{L}, V_{H} and V_{H}, etc., preferably V_{H} and V_{L}. In diabody-forming fragments, the linker joining variable regions is not specifically limited, but preferably enough short to avoid noncovalent bonds between variable regions in the same fragment. The length of such a linker can be determined as appropriate by those skilled in the art, but typically 2-14 amino acids, preferably 3-9 amino acids, especially 4-6 amino acids. In this case, the V_{L} and V_{H} encoded on the same fragment are joined via a linker short enough to avoid noncovalent bonds between the V_{L} and V_{H} on the same chain and to avoid the formation of single-chain variable region fragments so that dimers with another fragment can be formed. The dimers can be formed via either covalent or noncovalent bonds or both between diabody-forming fragments.

Moreover, diabody-forming fragments can be joined via a linker or the like to form single-chain diabodies (sc(Fv)₂). By joining diabody-forming fragments using a long linker of about 15-20 amino acids, noncovalent bonds can be formed between diabody-forming fragments existing on the same chain to form dimers. Based on the same principle as for preparing diabodies, polymerized antibodies such as trimers or tetramers can also be prepared by joining three or more diabody-forming fragments.

An antibody or functional fragment may be monovalent or multivalent, e.g. bivalent like the full-length IgG molecule. According to a preferred embodiment of the present invention, the antibody or functional fragment thereof is monovalent. According to another preferred embodiment of the present invention, the antibody or functional fragment thereof is a monovalent functional fragment of an antibody. Monovalent fragments of an antibody include fragment antigen-binding (Fab), F(ab'), Fv, disulfide-linked variable fragment (dsFv), monovalent IgG, and single-chain variable fragment (scFv). Preferred functional fragments of the present invention are Fab, Fv, dsFv, scFv and diabodies. A particularly preferred functional antibody fragment of the invention is the antigen-binding fragment Fab. According to another preferred embodiment of the invention the antibody or functional fragment, preferably functional antibody fragment, is multivalent, e.g. bivalent, however, comprising only one GPVI antigen binding region.

The term "human Glycoprotein VI" or "human GPVI" is known by the skilled person and refers to a platelet membrane glycoprotein that is involved in platelet-collagen interactions. GPVI is a transmembrane collagen receptor expressed on the surface of platelets. In one embodiment, human GPVI refers to a protein comprising, or consisting of, an amino acid having the UniProt accession number Q9HCN6 (preferably Q9HCN6-1 with the NCBI accession number NP_057447), or any amino acid sequence presenting at least about 90% identity with the amino acid sequence of Q9HCN6-1, Q9HCN6-2 or Q9HCN6-3 preferably at least about 91, 92, 93, 94, 95, 96, 97, 98, 99% identity or more with the amino acid sequence of Q9HCN6-1, Q9HCN6-2 or Q9HCN6-3.

Preferably, the antibody or functional fragment of the invention specifically binds to GPVI. As used herein, an antibody or functional fragment thereof "specifically recognizes", or "specifically binds to" human GPVI, when the antibody or functional fragment is able to discriminate between human GPVI and one or more reference molecule(s). Preferably, the IC₅₀ value for binding to each of the reference molecules is at least 1,000 times greater than the IC₅₀ value for binding to GPVI. In its most general form (and when no defined reference is mentioned), "specific binding" refers to the ability of the antibody or functional fragment to discriminate between human GPVI and an unrelated biomolecule, as determined, for example, in accordance with a specificity assay method known in the art. Such methods comprise, but are not limited to, bio-layer interferometry (BLI), surface plasmon resonance (SPR), ELISA tests, Western blots and immunofluorescence-based assays such as immuno-histochemistry (IHC). For example, a standard BLI, SPR or ELISA assay can be carried out. Typically, determination of binding specificity is performed by using not a single reference biomolecule, but a set of about three to five unrelated biomolecules, such as milk powder, BSA, transferrin or the like.

The binding epitope on GPVI to which the antibody or functional fragment of the invention binds is not particularly limited. According to one embodiment of the invention, the antibody or functional fragment binds to human GPVI close to or at the proposed GPVI dimerization residues. According to a preferred embodiment of the invention, the antibody or functional fragment binds to human GPVI at a binding epitope which at least partially overlaps with the proposed GPVI dimerization residues. The proposed GPVI dimerization residues can be found in the ectodomain of the GPVI, and preferably refer to residues D173 to T180 of SEQ ID NO:36 (SEQ ID NO:36 corresponding to part of the ectodomain of GPVI isoform identified by UniProt as isoform 1 and by NCBI as isoform 2), which correspond to residues D196 to T203 of GPVI isoforms 1 to 3 in UniProt (i.e. Q9HCN6-1, Q9HCN6-2 and Q9HCN6-3). According to another embodiment of the invention, the binding of the antibody or functional fragment to a human GPVI interrupts, or interferes with, GPVI dimerization. According to a preferred embodiment of the invention, the antibody or functional fragment binds to human GPVI at a binding epitope comprising, or consisting of, amino acids V178 to E192 and/or S223 to P237 of SEQ ID NO:36. According to another preferred embodiment of the invention, the antibody or functional fragment binds to a discontinuous binding epitope comprising, or consisting of, amino acids V178 to E192 and S223 to P237 in SEQ ID NO:36.

The antibody of the invention or the functional fragment of the invention comprises a V_{L} domain and a V_{H} domain. The V_{L} domain comprises a CDR1 region (CDRL1), a CDR2 region (CDRL2), a CDR3 region (CDRL3) and Framework regions. The V_{H} domain comprises a CDR1 region (CDRH1), a CDR2 region (CDRH2), a CDR3 region (CDRH3) and Framework regions.

The term "CDR" or "complementarity determining region" refers to one of the six hypervariable regions within the variable domains of an antibody that mainly contribute to antigen binding. One of the most commonly used definitions for the six CDRs was provided by Kabat E. A. et al., (1991) "Sequences of proteins of immunological interest." NIH Publication 91-3242*.* Another commonly used system for defining/identifying CDRs was provided by Lefranc at al. (2003) "IMGT unique numbering for immunoglobulin and T cell receptor variable domains and Ig superfamily V-like domains". Dev. Comp. Immunol., 27, 55-77. The CDR sequences of the present antibody or functional fragment were identified using antibody numbering systems from IMGT and Kabat, that is, as a combination of IMGT/Kabat CDR sequences.

The V_{L} domain of the antibody or functional fragment of the present invention comprises a CDR1 region having an amino acid sequence in accordance with the amino acid sequence as shown in SEQ ID NO:1, a CDR2 region having an amino acid sequence in accordance with the amino acid sequence as shown in SEQ ID NO:2 or SEQ ID NO:9, and a CDR3 region having an amino acid sequence in accordance with the amino acid sequence as shown in SEQ ID NO:3. In a preferred embodiment, the V_{L} domain of the antibody or functional fragment of the present invention comprises a CDR1 region having an amino acid sequence in accordance with the amino acid sequence as shown in SEQ ID NO:1, a CDR2 region having an amino acid sequence in accordance with the amino acid sequence as shown in SEQ ID NO:9, and a CDR3 region having an amino acid sequence in accordance with the amino acid sequence as shown in SEQ ID NO:3. In another preferred embodiment, the V_{L} domain of the antibody or functional fragment of the present invention comprises a CDR1 region having an amino acid sequence in accordance with the amino acid sequence as shown in SEQ ID NO:1, a CDR2 region having an amino acid sequence in accordance with the amino acid sequence as shown in SEQ ID NO:2, and a CDR3 region having an amino acid sequence in accordance with the amino acid sequence as shown in SEQ ID NO:3. In another preferred embodiment, the V_{L} domain of the antibody or functional fragment of the present invention comprises a CDR1 region having an amino acid sequence in accordance with the amino acid sequence as shown in SEQ ID NO:1, a CDR2 region having an amino acid sequence in accordance with the amino acid sequence as shown in SEQ ID NO:8, and a CDR3 region having an amino acid sequence in accordance with the amino acid sequence as shown in SEQ ID NO:3.

In the amino acid sequence SEQ ID NO:9 the residue X preferably is alanine (A), arginine (R), asparagine (N), aspartic acid (D), glutamine (Q), glutamic acid (E), glycine (G), histidine (H), isoleucine (I), leucine (L), lysine (K), methionine (M), phenylalanine (F), serine (S), threonine (T), tryptophane (W), tyrosine (Y), or valine (V). In a particular embodiment of the present invention, in the amino acid sequence SEQ ID NO:9 the residue X is A. In another particular embodiment of the present invention, in the amino acid sequence SEQ ID NO:9 the residue X is R. In yet another particular embodiment of the present invention, in the amino acid sequence SEQ ID NO:9 the residue X is N. In yet another particular embodiment of the present invention, in the amino acid sequence SEQ ID NO:9 the residue X is D. In yet another particular embodiment of the present invention, in the amino acid sequence SEQ ID NO:9 the residue X is Q. In yet another particular embodiment of the present invention, in the amino acid sequence SEQ ID NO:9 the residue X is E. In yet another particular embodiment of the present invention, in the amino acid sequence SEQ ID NO:9 the residue X is G. In yet another particular embodiment of the present invention, in the amino acid sequence SEQ ID NO:9 the residue X is H. In yet another particular embodiment of the present invention, in the amino acid sequence SEQ ID NO:9 the residue X is I. In yet another particular embodiment of the present invention, in the amino acid sequence SEQ ID NO:9 the residue X is L. In yet another particular embodiment of the present invention, in the amino acid sequence SEQ ID NO:9 the residue X is K. In yet another particular embodiment of the present invention, in the amino acid sequence SEQ ID NO:9 the residue X is M. In yet another particular embodiment of the present invention, in the amino acid sequence SEQ ID NO:9 the residue X is F. In yet another particular embodiment of the present invention, in the amino acid sequence SEQ ID NO:9 the residue X is S. In yet another particular embodiment of the present invention, in the amino acid sequence SEQ ID NO:9 the residue X is T. In yet another particular embodiment of the present invention, in the amino acid sequence SEQ ID NO:9 the residue X is W. In yet another particular embodiment of the present invention, in the amino acid sequence SEQ ID NO:9 the residue X is Y. In yet another particular embodiment of the present invention, in the amino acid sequence SEQ ID NO:9 the residue X is V.

The V_{H} domain of the antibody or functional fragment of the present invention comprises a CDR1 region having an amino acid sequence in accordance with the amino acid sequence as shown in SEQ ID NO:4, a CDR2 region having an amino acid sequence in accordance with the amino acid sequence as shown in SEQ ID NO:5 or SEQ ID NO:13, and a CDR3 region having an amino acid sequence in accordance with the amino acid sequence as shown in SEQ ID NO:6. SEQ ID NO:13 basically corresponds to the amino acid sequence of SEQ ID NO:5, but with the proviso that preferably the residues in positions 5 and 6 are selected from A, R, N, D, Q, E, G, H, I, L, K, M, F, S, T, W, Y, and V. In a particular embodiment, in SEQ ID NO:13, the residue in position 5 is D or E, and the residue in position 6 is G or A. In a preferred embodiment of the present invention, the V_{H} domain of the antibody or functional fragment of the present invention comprises a CDR1 region having an amino acid sequence in accordance with the amino acid sequence as shown in SEQ ID NO:4, a CDR2 region having an amino acid sequence in accordance with the amino acid sequence as shown in SEQ ID NO:5, and a CDR3 region having an amino acid sequence in accordance with the amino acid sequence as shown in SEQ ID NO:6.

In an alternative embodiment (Kabat only), the antibody of the invention or the functional fragment of the invention comprises (i) a V_{L} domain comprising a CDR1 region having the amino acid sequence as shown in SEQ ID NO:1, a CDR2 region having the amino acid sequence as shown in SEQ ID NO:2, and a CDR3 region having the amino acid sequence as shown in SEQ ID NO:3, and (ii) a V_{H} domain comprising a CDR1 region having the amino acid sequence as shown in SEQ ID NO:10, a CDR2 region having the amino acid sequence as shown in SEQ ID NO:5, and a CDR3 region having the amino acid sequence as shown in SEQ ID NO:14.

In another alternative embodiment, the antibody of the invention or the functional fragment of the invention comprises (i) a V_{L} domain comprising a CDR1 region having the amino acid sequence as shown in SEQ ID NO:7, a CDR2 region having the amino acid sequence as shown in SEQ ID NO:8, and a CDR3 region having the amino acid sequence as shown in SEQ ID NO:3, and (ii) a V_{H} domain comprising a CDR1 region having the amino acid sequence as shown in SEQ ID NO:11, a CDR2 region having the amino acid sequence as shown in SEQ ID NO:12, and a CDR3 region having the amino acid sequence as shown in SEQ ID NO:6.

In another embodiment, the antibody or functional fragment of the invention comprises a V_{L} domain comprising, or consisting of, an amino acid sequence having a sequence identity of at least 80 %, preferably at least 90 %, more preferably at least 92%, even more preferably at least 94 %, even more preferably at least 95%, even more preferably at least 96 %, even more preferably at least 97%, even more preferably at least 98 %, even more preferably at least 99 %, to a sequence selected from the group consisting of SEQ ID NO:16, 17, 18, 19, 26, 27, 28, 29, 30, 31, 32, 33, 34, and 35, preferably 16, 17, 18, 19, 32, 33, 34 and 35, more preferably 16, 17, 18, 32, 33, and 34. In yet another embodiment, the antibody or functional fragment of the invention comprises a V_{L} domain comprising, or consisting of, an amino acid sequence having a sequence identity of at least 80 %, preferably at least 90 %, more preferably at least 92%, even more preferably at least 94 %, even more preferably at least 95%, even more preferably at least 96 %, even more preferably at least 97%, even more preferably at least 98 %, even more preferably at least 99 %, to a sequence selected from the group consisting of SEQ ID NO: 26, 27, 28, 32, 33, 34, and 35, preferably 32, 33, 34 and 35, more preferably 32, 33, and 34.

In yet another embodiment, the antibody or functional fragment of the invention comprises a V_{L} domain comprising, or consisting of, an amino acid sequence having a sequence identity of at least 80 %, 90 %, 92%, 94%, 95%, 96%, 97%, 98%, or 99%, to a sequence selected from the group consisting of SEQ ID NO:16, 17, 18, 19, 29, 30 and 31. In another embodiment, the antibody or functional fragment of the invention comprises a V_{L} domain comprising, or consisting of, an amino acid sequence having a sequence identity of at least 80 %, 90 %, 92%, 94%, 95%, 96%, 97%, 98%, or 99%, to a sequence selected from the group consisting of SEQ ID NO:16, 17, 18 and 19, preferably 16, 17 and 18. In a further embodiment, the antibody or functional fragment of the invention comprises a V_{H} domain comprising, or consisting of, an amino acid sequence having a sequence identity of at least 80 %, 90 %, 92%, 94%, 95%, 96%, 97%, 98%, or 99%, to a sequence selected from the group consisting of SEQ ID NO:21, 22, 23, 24 and 25, preferably 21, 22 and 23.

According to a preferred embodiment, the antibody or functional fragment of the present invention comprises a V_{L} domain comprising, or consisting of, an amino acid sequence having a sequence identity of at least 80 %, 90 %, 92%, 94%, 95%, 96%, 97%, 98%, or 99%, to the amino acid sequence as shown in SEQ ID NO:17; and a V_{H} domain comprising, or consisting of, an amino acid sequence having a sequence identity of at least 80 %, 90 %, 92%, 94%, 95%, 96%, 97%, 98%, or 99%, to the amino acid sequence as shown in SEQ ID NO:21.

According to one embodiment, the antibody or functional fragment of the present invention comprises a V_{L} domain comprising, or consisting of, an amino acid sequence as shown in SEQ ID NO:16, 17, 18, 19, 26, 27, 28, 29, 30, 31, 32, 33, 34, or 35, preferably 16, 17, 18, 19, 32, 33, 34, or 35, more preferably 16, 17, 18, 32, 33, or 34. According to another embodiment, the antibody or functional fragment of the present invention comprises a V_{L} domain comprising, or consisting of, an amino acid sequence as shown in SEQ ID NO: 26, 27, 28, 32, 33, 34, or 35, preferably 32, 33, 34, or 35, more preferably 32, 33, or 34.

In yet another embodiment, the antibody or functional fragment of the invention comprises a V_{L} domain comprising, or consisting of, an amino acid sequence as shown in SEQ ID NO:16, 17, 18, 19, 29, 30 or 31. In another embodiment, the antibody or functional fragment of the invention comprises a V_{L} domain comprising, or consisting of, an amino acid sequence as shown in SEQ ID NO:16, 17, 18 or 19. In another embodiment, the antibody or functional fragment of the invention comprises a V_{L} domain comprising, or consisting of, an amino acid sequence as shown in SEQ ID NO:29, 17, 30 or 31. In a preferred embodiment, the antibody or functional fragment of the invention comprises a V_{L} domain comprising, or consisting of, an amino acid sequence as shown in SEQ ID NO:16, 17, 18, 32, 33, or 34. In another preferred embodiment, the antibody or functional fragment of the invention comprises a V_{L} domain comprising, or consisting of, an amino acid sequence as shown in SEQ ID NO:32, 33, or 34. In yet another preferred embodiment, the antibody or functional fragment of the invention comprises a V_{L} domain comprising, or consisting of, an amino acid sequence as shown in SEQ ID NO:16, 17, or 18. In a particularly preferred embodiment, the antibody or functional fragment of the invention comprises a V_{L} domain comprising, or consisting of, an amino acid sequence as shown in SEQ ID NO:17. In another particularly preferred embodiment, the antibody or functional fragment of the invention comprises a V_{L} domain comprising, or consisting of, an amino acid sequence as shown in SEQ ID NO:33.

In a further embodiment, the antibody or functional fragment of the invention comprises a V_{H} domain comprising, or consisting of, an amino acid sequence as shown in SEQ ID NO:21, 22, 23, 24 or 25. In a preferred embodiment, the antibody or functional fragment of the invention comprises a V_{H} domain comprising, or consisting of, an amino acid sequence as shown in SEQ ID NO: 21, 22, or 23. In a particularly preferred embodiment, the antibody or functional fragment of the invention comprises a V_{H} domain comprising, or consisting of, an amino acid sequence as shown in SEQ ID NO:21.

According to a specific preferred embodiment of the present invention, the V_{L} domain and V_{H} domain, comprise, or consist of a pair of amino acids sequences as shown SEQ ID NO: 32 and 21, 32 and 22, 32 and 23, 32 and 24, 32 and 25, 33 and 21, 33 and 22, 33 and 23, 33 and 24, 33 and 25, 34 and 21, 34 and 22, 34 and 23, 34 and 24, 34 and 25, 35 and 21, 35 and 22, 35 and 23, 35 and 24, 35 and 25, 26 and 21, 26 and 22, 26 and 23, 26 and 24, 26 and 25, 27 and 21, 27 and 22, 27 and 23, 27 and 24, 27 and 25, 28 and 21, 28 and 22, 28 and 23, 28 and 24, 28 and 25, 16 and 21, 16 and 22, 16 and 23, 16 and 24, 16 and 25, 17 and 21, 17 and 22, 17 and 23, 17 and 24, 17 and 25, 18 and 21, 18 and 22, 18 and 23, 18 and 24, 18 and 25, 19 and 21, 19 and 22, 19 and 23, 19 and 24, or 19 and 25 (that is, V_{L} comprises, or consists of, sequence as shown in SEQ ID NO:32, and V_{H} comprises, or consists of, the sequence as shown in SEQ ID NO:21, or V_{L} comprises, or consists of, sequence as shown in SEQ ID NO:32, and V_{H} comprises, or consists of, the sequence as shown in SEQ ID NO:22, etc.); preferably 33 and 21, 33 and 22, 34 and 22, 17 and 21, 17 and 22, or 18 and 22.

According to another specific preferred embodiment of the present invention, the V_{L} domain and V_{H} domain, comprise, or consist of a pair of amino acids sequences as shown SEQ ID NO:32 and 21, 32 and 22, 32 and 23, 32 and 24, 32 and 25, 33 and 21, 33 and 22, 33 and 23, 33 and 24, 33 and 25, 34 and 21, 34 and 22, 34 and 23, 34 and 24, 34 and 25, 35 and 21, 35 and 22, 35 and 23, 35 and 24, 35 and 25, 26 and 21, 26 and 22, 26 and 23, 26 and 24, 26 and 25, 27 and 21, 27 and 22, 27 and 23, 27 and 24, 27 and 25, 28 and 21, 28 and 22, 28 and 23, 28 and 24, or 28 and 25; preferably 32 and 22, 33 and 21, 33 and 22, 33 and 23, 34 and 21, 34 and 22, 34 and 23, 26 and 22, 27 and 21, 27 and 22, or 27 and 23. According to yet another specific preferred embodiment of the present invention, the V_{L} domain and V_{H} domain, comprise, or consist of a pair of amino acids sequences as shown SEQ ID NO: 32 and 21, 32 and 22, 32 and 23, 32 and 24, 32 and 25, 33 and 21, 33 and 22, 33 and 23, 33 and 24, 33 and 25, 34 and 21, 34 and 22, 34 and 23, 34 and 24, 34 and 25, 35 and 21, 35 and 22, 35 and 23, 35 and 24, 35 and 25, 26 and 21, 26 and 22, 26 and 23, 26 and 24, or 26 and 25; preferably 32 and 22, 33 and 21, 33 and 22, 33 and 23, 34 and 21, 34 and 22, or 34 and 23.

According to yet another specific preferred embodiment of the present invention, the V_{L} domain and V_{H} domain, comprise, or consist of a pair of amino acids sequences as shown SEQ ID NO:16 and 21, 16 and 22, 16 and 23, 16 and 24, 16 and 25, 17 and 21, 17 and 22, 17 and 23, 17 and 24, 17 and 25, 18 and 21, 18 and 22, 18 and 23, 18 and 24, 18 and 25, 19 and 21, 19 and 22, 19 and 23, 19 and 24, 19 and 25, 29 and 21, 29 and 22, 29 and 23, 29 and 24, 29 and 25, 30 and 21, 30 and 22, 30 and 23, 30 and 24, 30 and 25, 31 and 21, 31 and 22, 31 and 23, 31 and 24, or 31 and 25 (that is, V_{L} comprises, or consists of, sequence as shown in SEQ ID NO:16, and V_{H} comprises, or consists of, the sequence as shown in SEQ ID NO:21, or V_{L} comprises, or consists of, sequence as shown in SEQ ID NO:16, and V_{H} comprises, or consists of, the sequence as shown in SEQ ID NO:22, etc.). According to another specific preferred embodiment of the present invention, the V_{L} domain and V_{H} domain, comprise, or consist of a pair of amino acids sequences as shown SEQ ID NO:16 and 22, 16 and 23, 16 and 25, 17 and 21, 17 and 22, 17 and 23, 17 and 24, 17 and 25, 18 and 21, 18 and 22, 18 and 23, 29 and 22, 29 and 23, 29 and 25, 30 and 21, 30 and 22, or 30 and 23. According to another specific preferred embodiment of the present invention, the V_{L} domain and V_{H} domain, comprise, or consist of a pair of amino acids sequences as shown SEQ ID NO:16 and 22, 17 and 21, 17 and 22, 17 and 23, 18 and 21, 18 and 22, 18 and 23, 29 and 22, 30 and 21, 30 and 22, or 30 and 23. According to yet another specific preferred embodiment of the present invention, the V_{L} domain and V_{H} domain, comprise, or consist of a pair of amino acids sequences as shown SEQ ID NO:17 and 21, 17 and 22, 17 and 23, 18 and 21, 18 and 22, 18 and 23, 30 and 21, 30 and 22, or 30 and 23. According to yet another specific preferred embodiment of the present invention, the V_{L} domain and V_{H} domain, comprise, or consist of a pair of amino acids sequences as shown SEQ ID NO:17 and 21, 17 and 22, 17 and 23, 18 and 21, 18 and 22, 30 and 21, or 30 and 22. According to yet another specific preferred embodiment of the present invention, the V_{L} domain and V_{H} domain, comprise, or consist of a pair of amino acids sequences as shown SEQ ID NO:17 and 21, 17 and 22, 18 and 22, or 30 and 22.

According to a further specific preferred embodiment of the present invention, the V_{L} domain and V_{H} domain, comprise, or consist of a pair of amino acids sequences as shown SEQ ID NO:16 and 21, 16 and 22, 16 and 23, 16 and 24, 16 and 25, 17 and 21, 17 and 22, 17 and 23, 17 and 24, 17 and 25, 18 and 21, 18 and 22, 18 and 23, 18 and 24, 18 and 25, 19 and 21, 19 and 22, 19 and 23, 19 and 24, 19 and 25 (that is, V_{L} comprises, or consists of, sequence as shown in SEQ ID NO:16, and V_{H} comprises, or consists of, the sequence as shown in SEQ ID NO:21, or V_{L} comprises, or consists of, sequence as shown in SEQ ID NO:16, and V_{H} comprises, or consists of, the sequence as shown in SEQ ID NO:22, etc.). According to another specific preferred embodiment of the present invention, the V_{L} domain and V_{H} domain, comprise, or consist of a pair of amino acids sequences as shown SEQ ID NO:16 and 22, 16 and 23, 16 and 25, 17 and 21, 17 and 22, 17 and 23, 17 and 24, 17 and 25, 18 and 21, 18 and 22, or 18 and 23. According to yet another specific preferred embodiment of the present invention, the V_{L} domain and V_{H} domain, comprise, or consist of a pair of amino acids sequences as shown SEQ ID NO:16 and 22, 17 and 21, 17 and 22, 17 and 23, 18 and 21, 18 and 22, or 18 and 23. According to yet another specific preferred embodiment of the present invention, the V_{L} domain and V_{H} domain, comprise, or consist of a pair of amino acids sequences as shown SEQ ID NO:17 and 21, 17 and 22, 17 and 23, 18 and 21, 18 and 22, or 18 and 23. According to yet another specific preferred embodiment of the present invention, the V_{L} domain and V_{H} domain, comprise, or consist of a pair of amino acids sequences as shown SEQ ID NO:17 and 21, 17 and 22, 17 and 23, 18 and 21, or 18 and 22. According to yet another specific preferred embodiment of the present invention, the V_{L} domain and V_{H} domain, comprise, or consist of a pair of amino acids sequences as shown SEQ ID NO:17 and 21, 17 and 22, or 18 and 22.

According to a particularly preferred embodiment of the present invention, the V_{L} domain and V_{H} domain, comprise, or consist of a pair of amino acids sequences as shown SEQ ID NO:33 and 21. According to another particularly preferred embodiment of the present invention, the V_{L} domain and V_{H} domain, comprise, or consist of a pair of amino acids sequences as shown SEQ ID NO:17 and 21.

In an alternative embodiment, the V_{L} domain of the antibody or functional fragment of the invention comprises, or consists of, instead of SEQ ID NO:16, SEQ ID NO:18 and SEQ ID NO:19 as described in any embodiment described above, an amino acid sequence as shown in SEQ ID NO:26, SEQ ID NO:27 and SEQ ID NO:28, respectively.

In a further embodiment, the antibody or functional fragment of the invention comprises a V_{L} domain comprising, or consisting of, an amino acid sequence as shown in SEQ ID NO:15; and/or a V_{H} domain comprising, or consisting of, an amino acid sequence as shown in SEQ ID NO: 20.

In another particular embodiment the antibody of the invention is an immunoglobulin or a functional fragment thereof, preferably an immunoglobulin G (IgG) or a functional fragment thereof. The subclass of IgG suitable for the present invention is not limited and includes IgG₁, IgG₂, IgG₃, and IgG₄. Preferably, the IgG is of subclass 1, i.e. it is an IgG₁ molecule.

Usually, the antibody or functional fragment of the present invention comprises a light chain comprising the V_{L} domain and a heavy chain comprising the V_{H} domain. According to a specific embodiment of the present invention, the antibody or functional fragment consists of a light chain comprising the V_{L} domain and a heavy chain comprising the V_{H} domain. According to a preferred embodiment of the present invention, the antibody or functional fragment is monovalent, e.g. a Fab, with a light chain comprising the V_{L} domain, which comprises, or consists of an amino acid sequence as specified in any of the embodiment for V_{L} domain above, and with a heavy chain comprising the V_{H} domain, which comprises, or consists of an amino acid sequence as specified in any of the embodiment for V_{H} domain above.

According to a preferred embodiment of the present invention, the light chain, in addition to the V_{L} domain, comprises a light chain constant domain (C_{L}), preferably positioned C-terminally to the V_{L} domain. According to a further preferred embodiment of the present invention, the heavy chain, in addition to the V_{H} domain, comprises a heavy chain constant domain (C_{H}), preferably positioned C-terminally to the V_{H} domain.

According to a specific embodiment, the antibody or functional fragment of the present invention comprises a light chain derived from a human Immunoglobulin kappa (IgK) light chain (preferably allotype Km3). According to another specific embodiment, the antibody or functional fragment of the present invention comprises a light chain comprising a constant domain derived from a human IgK light chain constant domain (preferably allotype Km3). According to yet another specific embodiment, the antibody or functional fragment of the present invention comprises a light chain constant domain comprising, or consisting of, a human IgK light chain constant domain (preferably allotype Km3). According to another specific embodiment, the antibody or functional fragment of the present invention comprises a heavy chain comprising a constant domain derived from human IgG1 heavy chain CH1 constant domain (preferably allotype G1m17,1). According to another specific embodiment, the antibody or functional fragment of the present invention comprises a heavy chain comprising a constant domain comprising, or consisting of, a human IgG1 heavy chain CH1 constant domain (preferably allotype G1m17,1).

According to yet another specific embodiment, the antibody or functional fragment of the present invention comprises a light chain comprising a constant domain derived from human IgK light chain constant domain (allotype Km3), preferably wherein the sum of the number of amino acids, which are different from the amino acid sequence of the human IgK light chain constant domain (allotype Km3) e.g. as shown in SEQ ID NO:60, is less than 10, 9, 8, 7, 6, 5, 4, 3, or 2; and/or a heavy chain comprising a constant domain derived from human IgG1 heavy chain CH1 constant domain (allotype G1m17,1), preferably wherein the sum of the number of amino acids, which are different from the amino acid sequence of the human IgG1 heavy chain CH1 constant domain (allotype G1m17,1) e.g. as shown in SEQ ID NO:61, is less than 10, 9, 8, 7, 6, 5, 4, 3, or 2.

According to a preferred embodiment of the present invention, the light chain constant domain (C_{L}) comprises an amino acid sequence having a sequence identity of at least 80 %, 85 %, 90 %, 92 %, 93 %, 94 %, 95 %, 96 %, 97 %, 98 %t, or 99 %, to an amino acid shown in SEQ ID NO:60. According to another preferred embodiment of the present invention, the light chain constant domain (C_{L}) consists of an amino acid sequence having a sequence identity of at least 80 %, 85 %, 90 %, 92 %, 93 %, 94 %, 95 %, 96 %, 97 %, 98 %t, or 99 %, to an amino acid shown in SEQ ID NO:60.

According to a preferred embodiment of the present invention, the heavy chain constant domain (C_{H}) comprises an amino acid sequence having a sequence identity of at least 80 %, 85 %, 90 %, 92 %, 93 %, 94 %, 95 %, 96 %, 97 %, 98 %t, or 99 %, to an amino acid shown in SEQ ID NO:61. According to another preferred embodiment of the present invention, the light chain constant domain (C_{H}) consists of an amino acid sequence having a sequence identity of at least 80 %, 85 %, 90 %, 92 %, 93 %, 94 %, 95 %, 96 %, 97 %, 98 %t, or 99 %, to an amino acid shown in SEQ ID NO:61.

According to a further preferred embodiment of the present invention, the light chain comprises an N-terminal light chain signal peptide comprising, or consisting of, an amino acid sequence as shown in SEQ ID NO:62, and/or a light chain constant domain (CL) comprising, or consisting of, an amino acid sequence as shown in SEQ ID NO:60, and preferably C-terminal to the V_{L} domain. According to another preferred embodiment of the present invention, the heavy chain comprises an N-terminal heavy chain signal peptide comprising, or consisting of, an amino acid sequence as shown in SEQ ID NO:63, and/or a heavy chain constant domain (CH) comprising, or consisting of, an amino acid sequence as shown in SEQ ID NO:61, and preferably C-terminal to the VH domain. The N-terminal signal peptide and/or the constant region may be arranged on the light chain and/or heavy chain such that they are directly linked to V_{L} and/or V_{H} domain, or may be linked via a linker peptide. Preferably, the N-terminal signal peptide and the constant region are arranged on the light chain and heavy chain such that they are directly linked to V_{L} and V_{H} domain.

According to a preferred embodiment of the present invention, the antibody or functional fragment, preferably is monovalent, more preferably a Fab, and comprises (a) a light chain comprising, or consisting of, the V_{L} domain, which comprises, or consists of an amino acid as specified in any of the embodiment for V_{L} domain above, a C_{L} domain, which comprises, or consists of, an amino acid as specified in any of the embodiment for C_{L} domain above, and optionally a N-terminal light chain signal peptide as specified above; and (b) with a heavy chain comprising, or consisting of, the V_{H} domain, which comprises, or consists of an amino acid as specified in any of the embodiment for V_{H} domain above, a C_{H} domain, which comprises, or consists of, an amino acid as specified in any of the embodiment for C_{H} domain above, and optionally a N-terminal heavy chain signal peptide as specified above.

The light chain and heavy chain may comprise further amino acid sequences N- or C-terminal to the V_{L} and V_{H} domains, e.g. other signal peptides, binding peptides, linker peptides, other functional protein domains, etc. Moreover, the light chain and heavy chain may comprise further molecules covalently attached or otherwise bound at the N- or C-terminus e.g. other signal molecules, binding moieties, (cross-)linker molecules, other functional moieties, etc.

In a preferred embodiment of the present invention, the antibody or functional fragment thereof comprises, or consists of, a light chain comprising, or consisting of, an amino acid sequence as shown in one of SEQ ID NO:38, 39, 40, 41, 49, 50, 51, 52, 53, 54, 55, 56, 57 and 58, preferably SEQ ID NO:38, 39, 40, 41, 55, 56, 57 and 58, more preferably SEQ ID NO:38, 39, 40, 55, 56, and 57; and a heavy chain comprising, or consisting of, an amino acid sequence as shown in one of SEQ ID NO:44, 45, 46, 47 and 48, preferably SEQ ID NO:44, 45 and 46. In another preferred embodiment of the present invention, antibody or functional fragment thereof comprises, or consists of, a light chain comprising, or consisting of, an amino acid sequence as shown in one of SEQ ID NO:52, 53, 54, 55, 56, 57 and 58, preferably SEQ ID NO:52, 53 and 54; and a heavy chain comprising, or consisting of, an amino acid sequence as shown in one of SEQ ID NO:44, 45, 46, 47 and 48, preferably SEQ ID NO:44, 45 and 46. In yet another preferred embodiment of the present invention, the antibody or functional fragment thereof comprises, or consists of a light chain comprising, or consisting of, an amino acid sequence as shown in one of SEQ ID NO:38, 39, 40, 41, 49, 50 and 51, preferably SEQ ID NO:38, 39 and 40; and a heavy chain comprising, or consisting of, an amino acid sequence as shown in one of SEQ ID NO:44, 45, 46, 47 and 48, preferably SEQ ID NO:44, 45 and 46.

According to a preferred embodiment of the present invention, the antibody or functional fragment thereof is a monovalent functional antibody fragment, preferably a fragment antigen-binding (Fab), a F(ab'), a Fv, a disulfide-linked variable fragment (dsFv), a monovalent IgG, or a single-chain variable fragment (scFv). A particularly preferred functional antibody fragment of the invention is the monovalent Fab. In a preferred embodiment of the present invention, the functional fragment is a monovalent functional antibody fragment comprising, or consisting of, a light chain comprising, or consisting of, an amino acid sequence having a sequence identity of at least 80 %, 85 %, 90 %, 92 %, 93 %, 94 %, 95 %, 96 %, 97 %, 98 %t, 99 %, or 99.5%, to a sequence selected from the group consisting of SEQ ID NO: 38, 39, 40 or 41; and a heavy chain comprising, or consisting of, an amino acid sequence having a sequence identity of at least 80 %, 85 %, 90 %, 92 %, 93 %, 94 %, 95 %, 96 %, 97 %, 98 %t, 99 %, or 99.5%, to a sequence selected from the group consisting of SEQ ID NO: 44, 45, 46, 47 or 48.

In another preferred embodiment of the present invention, the functional fragment is a monovalent functional antibody fragment with a light chain comprising, or consisting of, an amino acid sequence as shown in one of SEQ ID NO:38, 39, 40, 41, 49, 50, 51, 52, 53, 54, 55, 56, 57 and 58, preferably SEQ ID NO:38, 39, 40, 41, 55, 56, 57 and 58, more preferably SEQ ID NO:38, 39, 40, 55, 56, and 57; and a heavy chain comprising, or consisting of, an amino acid sequence as shown in one of SEQ ID NO:44, 45, 46, 47 and 48, preferably SEQ ID NO:44, 45 and 46. In another preferred embodiment of the present invention, the functional fragment is a monovalent functional antibody fragment with a light chain comprising, or consisting of, an amino acid sequence as shown in one of SEQ ID NO:52, 53, 54, 55, 56, 57 and 58, and a heavy chain comprising, or consisting of, an amino acid sequence as shown in one of SEQ ID NO:44, 45, 46, 47 and 48. In another preferred embodiment of the present invention, the functional fragment is a monovalent functional antibody fragment with a light chain comprising, or consisting of, an amino acid sequence as shown in one of SEQ ID NO:38, 39, 40, 41, 49, 50 and 51, and a heavy chain comprising, or consisting of, an amino acid sequence as shown in one of SEQ ID NO:44, 45, 46, 47 and 48.

In a further preferred embodiment of the present invention, the functional fragment is monovalent, e.g. a fragment antigen-binding (Fab), comprising, or consisting of, (a) a light chain comprising, or consisting of, an amino acid sequence having a sequence identity of at least 80 %, 85 %, 90 %, 92 %, 93 %, 94 %, 95 %, 96 %, 97 %, 98 %t, 99 %, or 99.5%, to a sequence selected from the group consisting of SEQ ID NO: 38, 39, 40 or 41; and (b) a heavy chain comprising, or consisting of, an amino acid sequence having a sequence identity of at least 80 %, 85 %, 90 %, 92 %, 93 %, 94 %, 95 %, 96 %, 97 %, 98 %t, 99 %, or 99.5%, to a sequence selected from the group consisting of SEQ ID NO: 44, 45, 46, 47 or 48. In another preferred embodiment of the present invention, the functional fragment is monovalent, preferably a Fab, which comprises, or consists of, (a) a light chain comprising, or consisting of, an amino acid sequence as shown in one of SEQ ID NO: 38, 39, 40, 41, 49, 50 and 51, and (b) a heavy chain comprising, or consisting of, an amino acid sequence as shown in one of SEQ ID NO:44, 45, 46, 47 and 48. According to yet another preferred embodiment of the present invention, the functional fragment is monovalent, preferably a Fab, which comprises, or consists of, (a) a light chain comprising, or consisting of, an amino acid sequence as shown in one of SEQ ID NO: 38, 39, 40 and 41, and (b) a heavy chain comprising, or consisting of, an amino acid sequence as shown in one of SEQ ID NO:44, 45, 46, 47 and 48. According to another preferred embodiment of the present invention, the antibody or functional fragment thereof is monovalent, preferably a Fab, comprising, or consisting of, a light chain, which comprises, or consists of, an amino acid sequence as shown in one of SEQ ID NO:38, 39, 40, 41, 49, 50, 51, 52, 53, 54, 55, 56, 57 and 58, and a heavy chain, which comprises, or consists of, an amino acid sequence as shown in one of SEQ ID NO:44, 45, 46, 47 and 48. According to another preferred embodiment, the antibody or functional fragment thereof is monovalent, preferably a Fab comprising, or consisting of, a light chain, which comprises, or consists of, an amino acid sequence as shown in one of SEQ ID NO:52, 53, 54, 55, 56, 57 and 58, and a heavy chain, which comprises, or consists of, an amino acid sequence as shown in one of SEQ ID NO:44, 45, 46, 47 and 48. According to yet another preferred embodiment of the present invention, the antibody or functional fragment thereof is monovalent, preferably a Fab comprising, or consisting of, a light chain, which comprises, or consists of, an amino acid sequence as shown in one of SEQ ID NO: 55, 56, 57 and 58, and a heavy chain, which comprises, or consists of, an amino acid sequence as shown in one of SEQ ID NO:44, 45, 46, 47 and 48. According to yet another preferred embodiment, the antibody or functional fragment thereof is monovalent, preferably a Fab, comprising, or consisting of, a light chain, which comprises, or consists of, an amino acid sequence as shown in one of SEQ ID NO: 55, 56 and 57, and a heavy chain, which comprises, or consists of, an amino acid sequence as shown in one of SEQ ID NO: 44, 45, and 46.

In a specific preferred embodiment of the present invention, the antibody or functional fragment thereof (preferably is monovalent, more preferably a Fab, and) comprises, or consists of, a pair of light chain and heavy chain, comprising, or consisting of, the amino acid sequence as shown in SEQ ID NO:38 and 44, 38 and 45, 38 and 46, 38 and 47, 38 and 48, 39 and 44, 39 and 45, 39 and 46, 39 and 47, 39 and 48, 40 and 44, 40 and 45, 40 and 46, 40 and 47, 40 and 48, 41 and 44, 41 and 45, 41 and 46, 41 and 47, 41 and 48, 55 and 44, 55 and 45, 55 and 46, 55 and 47, 55 and 48, 56 and 44, 56 and 45, 56 and 46, 56 and 47, 56 and 48, 57 and 44, 57 and 45, 57 and 46, 57 and 47, 57 and 48, 58 and 44, 58 and 45, 58 and 46, 58 and 47, or 58 and 48 (that is, the light chain has sequence as shown in SEQ ID NO:38, and the heavy chain has the sequence as shown in SEQ ID NO:44, or the light chain has sequence as shown in SEQ ID NO:38, and the heavy chain has the sequence as shown in SEQ ID NO:45, etc.).

In another specific preferred embodiment of the present invention, the antibody or functional fragment thereof (preferably is monovalent, more preferably a Fab, and) comprises, or consists of, a pair of light chain and heavy chain, having the amino acid sequence as shown in SEQ ID NO:55 and 44, 55 and 45, 55 and 46, 55 and 47, 55 and 48, 56 and 44, 56 and 45, 56 and 46, 56 and 47, 56 and 48, 57 and 44, 57 and 45, 57 and 46, 57 and 47, 57 and 48, 58 and 44, 58 and 45, 58 and 46, 58 and 47, 58 and 48, 52 and 44, 52 and 45, 52 and 46, 52 and 47, 52 and 48, 53 and 44, 53 and 45, 53 and 46, 53 and 47, 53 and 48, 54 and 44, 54 and 45, 54 and 46, 54 and 47, or 54 and 48; preferably 55 and 45, 55 and 46, 55 and 48, 56 and 44, 56 and 45, 56 and 46, 56 and 47, 56 and 48, 57 and 44, 57 and 45, 57 and 46, 52 and 44, 52 and 45, 52 and 46, 53 and 44, 53 and 45, or 53 and 46; more preferably 55 and 45, 56 and 44, 56 and 45, 56 and 46, 57 and 44, 57 and 45, 57 and 46, 52 and 45, 53 and 44, 53 and 45, or 53 and 46; even more preferably 56 and 44, 56 and 45, 56 and 46, 57 and 44, 57 and 45, 57 and 46, 53 and 44, 53 and 45, or 53 and 46; even more preferably 56 and 44, 56 and 45, 56 and 46, 57 and 44, 57 and 45, 53 and 44, or 53 and 45; even more preferably 56 and 44, 56 and 45, 57 and 45, or 53 and 45.

In another specific preferred embodiment of the present invention, the antibody or functional fragment thereof is monovalent, preferably a Fab, comprising, or consisting of, a pair of light chain and heavy chain, comprising, or consisting of, the amino acid sequence as shown in SEQ ID NO:55 and 44, 55 and 45, 55 and 46, 55 and 47, 55 and 48, 56 and 44, 56 and 45, 56 and 46, 56 and 47, 56 and 48, 57 and 44, 57 and 45, 57 and 46, 57 and 47, 57 and 48, 58 and 44, 58 and 45, 58 and 46, 58 and 47, or 58 and 48; preferably 55 and 45, 55 and 46, 55 and 48, 56 and 44, 56 and 45, 56 and 46, 56 and 47, 56 and 48, 57 and 44, 57 and 45, or 57 and 46; more preferably 55 and 45, 55 and 46, 55 and 48, 56 and 44, 56 and 45, 56 and 46, 56 and 47, 56 and 48, 57 and 44, 57 and 45, or 57 and 46; even more preferably 55 and 45, 56 and 44, 56 and 45, 56 and 46, 57 and 44, 57 and 45, or 57 and 46; even more preferably 56 and 44, 56 and 45, 56 and 46, 57 and 44, 57 and 45, or 57 and 46; even more preferably 56 and 44, 56 and 45, 56 and 46, 57 and 44, or 57 and 45; even more preferably 56 and 44, 56 and 45, 56 and 46, or 57 and 45; even more preferably 56 and 44, 56 and 45, or 57 and 45, even more preferably 56 and 44.

In yet another specific preferred embodiment of the present invention, the antibody or functional fragment thereof (preferably is monovalent, more preferably a Fab, and) comprises, or consists of, a pair of light chain and heavy chain, comprising, or consisting of, the amino acid sequences as shown in SEQ ID NO:38 and 44, 38 and 45, 38 and 46, 38 and 47, 38 and 48, 39 and 44, 39 and 45, 39 and 46, 39 and 47, 39 and 48, 40 and 44, 40 and 45, 40 and 46, 40 and 47, 40 and 48, 41 and 44, 41 and 45, 41 and 46, 41 and 47, 41 and 48, 49 and 44, 49 and 45, 49 and 46, 49 and 47, 49 and 48, 50 and 44, 50 and 45, 50 and 46, 50 and 47, 50 and 48, 51 and 44, 51 and 45, 51 and 46, 51 and 47, or 51 and 48 (that is, the light chain comprises, or consists of, sequence as shown in SEQ ID NO:38, and the heavy chain comprises, or consists of, the sequence as shown in SEQ ID NO:44, or the light chain has sequence as shown in SEQ ID NO:38, and the heavy chain has the sequence as shown in SEQ ID NO:45, etc.). In another specific preferred embodiment of the present invention, the antibody or functional fragment thereof is monovalent, preferably a Fab, comprising, or consisting of, a pair of light chain and heavy chain, comprising, or consisting of, the amino acid sequences as shown in SEQ ID NO:38 and 45, 38 and 46, 38 and 48, 39 and 44, 39 and 45, 39 and 46, 39 and 47, 39 and 48, 40 and 44, 40 and 45, 40 and 46, 49 and 44, 49 and 45, 49 and 46, 50 and 44, 50 and 45, or 50 and 46. In yet another specific preferred embodiment of the present invention, the antibody or functional fragment thereof is monovalent, preferably a Fab, comprising, or consisting of, a pair of light chain and heavy chain, comprising, or consisting of, the amino acid sequences as shown in SEQ ID NO:38 and 45, 39 and 44, 39 and 45, 39 and 46, 40 and 44, 40 and 45, 40 and 46, 49 and 45, 50 and 44, 50 and 45, or 50 and 46. In yet another specific preferred embodiment of the present invention, the antibody or functional fragment thereof is monovalent, preferably a Fab, comprising, or consisting of, a pair of light chain and heavy chain, comprising, or consisting of, the amino acid sequences as shown in SEQ ID NO:39 and 44, 39 and 45, 39 and 46, 40 and 44, 40 and 45, 40 and 46, 50 and 44, 50 and 45, or 50 and 46. In yet another specific preferred embodiment of the present invention, the antibody or functional fragment thereof is monovalent, preferably a Fab, comprising, or consisting of, a pair of light chain and heavy chain, comprising, or consisting of, the amino acid sequences as shown in SEQ ID NO:39 and 44, 39 and 45, 39 and 46, 40 and 44, 40 and 45, 50 and 44, or 50 and 45. In yet another specific preferred embodiment of the present invention, the antibody or functional fragment thereof is monovalent, preferably a Fab, comprising, or consisting of, a pair of light chain and heavy chain, comprising, or consisting of, the amino acid sequences as shown in SEQ ID NO: 39 and 44, 39 and 45, 40 and 45, or 50 and 45.

In a further specific preferred embodiment of the present invention, the antibody or functional fragment (preferably is monovalent, more preferably a Fab, and) comprises, or consists of, a pair of light chain and heavy chain, having the amino acid sequences as shown in SEQ ID NO:38 and 44, 38 and 45, 38 and 46, 38 and 47, 38 and 48, 39 and 44, 39 and 45, 39 and 46, 39 and 47, 39 and 48, 40 and 44, 40 and 45, 40 and 46, 40 and 47, 40 and 48, 41 and 44, 41 and 45, 41 and 46, 41 and 47, or 41 and 48 (that is, the light chain has sequence as shown in SEQ ID NO:38, and the heavy chain has the sequence as shown in SEQ ID NO:44, or the light chain has sequence as shown in SEQ ID NO:38, and the heavy chain has the sequence as shown in SEQ ID NO:45, etc.). In another specific preferred embodiment of the present invention, the functional fragment (preferably is monovalent, more preferably a Fab, and) comprises, or consists of, a pair of light chain and heavy chain, having the amino acid sequences as shown in SEQ ID NO:38 and 45, 38 and 46, 38 and 48, 39 and 44, 39 and 45, 39 and 46, 39 and 47, 39 and 48, 40 and 44, 40 and 45, or 40 and 46. In yet another specific preferred embodiment of the present invention, the functional fragment (preferably is monovalent, more preferably a Fab, and) comprises, or consists of, a pair of light chain and heavy chain, having the amino acid sequences as shown in SEQ ID NO:38 and 45, 38 and 46, 38 and 48, 39 and 44, 39 and 45, 39 and 46, 39 and 47, 39 and 48, 40 and 44, 40 and 45, or 40 and 46. In yet another specific preferred embodiment of the present invention, the functional fragment (preferably is monovalent, more preferably a Fab, and) comprising, or consisting of, a pair of light chain and heavy chain, having the amino acid sequences as shown in SEQ ID NO:38 and 45, 39 and 44, 39 and 45, 39 and 46, 40 and 44, 40 and 45, or 40 and 46. In yet another specific preferred embodiment of the present invention, the functional fragment (preferably is monovalent, more preferably a Fab, and) comprises, or consists of, a pair of light chain and heavy chain, having the amino acid sequences as shown in SEQ ID NO:39 and 44, 39 and 45, 39 and 46, 40 and 44, 40 and 45, or 40 and 46.

In yet another specific preferred embodiment of the present invention, the antibody or functional fragment (preferably is monovalent, more preferably a Fab, and) comprises, or consists of, a pair of light chain and heavy chain, having the amino acid sequences as shown in SEQ ID NO:39 and 44, 39 and 45, 39 and 46, 40 and 44, or 40 and 45. In yet another specific preferred embodiment of the present invention, the antibody or functional fragment is (preferably is monovalent, more preferably a Fab, and) comprises, or consists of, a pair of light chain and heavy chain, having the amino acid sequences as shown in SEQ ID NO:39 and 44, 39 and 45, 39 and 46, or 40 and 45. In yet another specific preferred embodiment of the present invention, the antibody or functional fragment is (preferably is monovalent, more preferably a Fab, and) comprises, or consists of, a pair of light chain and heavy chain, having the amino acid sequences as shown in SEQ ID NO:39 and 44, 39 and 45, or 40 and 45. In another embodiment of the present invention, the functional fragment (preferably is monovalent, more preferably a Fab, and)consists of a pair of light chain and heavy chain, comprising, or consisting of the amino acid sequences as shown SEQ ID NO:37 and 43, respectively.

In a specific preferred embodiment of the present invention, the antibody or functional fragment thereof is monovalent, preferably a Fab, comprising, or consisting of, a pair of light chain and heavy chain, having the amino acid sequences as shown in SEQ ID NO:39 and 45. In another specific preferred embodiment of the present invention, the antibody or functional fragment thereof is monovalent, preferably a Fab, comprising, or consisting of, a pair of light chain and heavy chain, having the amino acid sequences as shown in SEQ ID NO:39 and 46. In another specific preferred embodiment of the present invention, the antibody or functional fragment thereof is monovalent, preferably a Fab, comprising, or consisting of, a pair of light chain and heavy chain, having the amino acid sequences as shown in SEQ ID NO:40 and 44. In another specific preferred embodiment of the present invention, the antibody functional fragment thereof is monovalent, preferably a Fab, comprising, or consisting of, a pair of light chain and heavy chain, having the amino acid sequences as shown in SEQ ID NO:40 and 45. In particularly preferred embodiment of the present invention, the antibody or functional fragment thereof is monovalent, preferably a Fab, comprising, or consisting of, a pair of light chain and heavy chain, having the amino acid sequences as shown in SEQ ID NO:39 and 44. In another particularly preferred embodiment of the present invention, the antibody or functional fragment thereof is monovalent, preferably a Fab, comprising, or consisting of, a pair of light chain and heavy chain, having the amino acid sequences as shown in SEQ ID NO:56 and 44.

According to a further embodiment of the present invention, in the amino acid sequences shown in SEQ ID NO:26, 27, 28, 32, 33, 34, 35, 52, 53, 54, 55, 56, 57, and 58, the residue X is selected from A, R, N, D, Q, E, G, H, I, L, K, M, F, S, T, W, Y, and V. In a particular embodiment of the present invention, in the amino acid sequences as shown in SEQ ID NO:26, 27, 28, 32, 33, 34, 35, 52, 53, 54, 55, 56, 57, and 58, the residue X is A. In another particular embodiment of the present invention, in the amino acid sequences shown in SEQ ID NO:26, 27, 28, 32, 33, 34, 35, 52, 53, 54, 55, 56, 57, and 58, the residue X is R. In yet another particular embodiment of the present invention, in the amino acid sequences shown in SEQ ID NO:26, 27, 28, 32, 33, 34, 35, 52, 53, 54, 55, 56, 57, and 58, the residue X is N. In yet another particular embodiment of the present invention, in the amino acid sequences shown in SEQ ID NO:26, 27, 28, 32, 33, 34, 35, 52, 53, 54, 55, 56, 57, and 58, the residue X is D. In yet another particular embodiment of the present invention, in the amino acid sequences shown in SEQ ID NO:26, 27, 28, 32, 33, 34, 35, 52, 53, 54, 55, 56, 57, and 58, the residue X is Q. In yet another particular embodiment of the present invention, in the amino acid sequences shown in SEQ ID NO:26, 27, 28, 32, 33, 34, 35, 52, 53, 54, 55, 56, 57, and 58, the residue X is E. In yet another particular embodiment of the present invention, in the amino acid sequences shown in SEQ ID NO:26, 27, 28, 32, 33, 34, 35, 52, 53, 54, 55, 56, 57, and 58, the residue X is G. In yet another particular embodiment of the present invention, in the amino acid sequence sequences shown in SEQ ID NO:26, 27, 28, 32, 33, 34, 35, 52, 53, 54, 55, 56, 57, and 58, the residue X is H. In yet another particular embodiment of the present invention, in the amino sequences shown in SEQ ID NO:26, 27, 28, 32, 33, 34, 35, 52, 53, 54, 55, 56, 57, and 58, the residue X is I. In yet another particular embodiment of the present invention, in the amino acid sequences shown in SEQ ID NO:26, 27, 28, 32, 33, 34, 35, 52, 53, 54, 55, 56, 57, and 58, the residue X is L. In yet another particular embodiment of the present invention, in the amino acid sequences shown in SEQ ID NO:26, 27, 28, 32, 33, 34, 35, 52, 53, 54, 55, 56, 57, and 58, the residue X is K. In yet another particular embodiment of the present invention, in the amino acid sequences shown in SEQ ID NO:26, 27, 28, 32, 33, 34, 35, 52, 53, 54, 55, 56, 57, and 58, the residue X is M. In yet another particular embodiment of the present invention, in the amino acid sequences shown in SEQ ID NO:26, 27, 28, 32, 33, 34, 35, 52, 53, 54, 55, 56, 57, and 58, the residue X is F. In yet another particular embodiment of the present invention, in the amino acid sequences shown in SEQ ID NO:26, 27, 28, 32, 33, 34, 35, 52, 53, 54, 55, 56, 57, and 58, the residue X is S. In yet another particular embodiment of the present invention, in the amino acid sequences shown in SEQ ID NO:26, 27, 28, 32, 33, 34, 35, 52, 53, 54, 55, 56, 57, and 58, the residue X is T. In yet another particular embodiment of the present invention, in the amino acid sequences shown in SEQ ID NO:26, 27, 28, 32, 33, 34, 35, 52, 53, 54, 55, 56, 57, and 58, the residue X is W. In yet another particular embodiment of the present invention, in the amino acid sequences shown in SEQ ID NO:26, 27, 28, 32, 33, 34, 35, 52, 53, 54, 55, 56, 57, and 58, the residue X is Y. In yet another particular embodiment of the present invention, in the amino acid sequences shown in SEQ ID NO:26, 27, 28, 32, 33, 34, 35, 52, 53, 54, 55, 56, 57, and 58, the residue X is V.

According to a particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is A. In another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is R. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is N. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is D. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is Q. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is E. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is G. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is H. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is I. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is L. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is K. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is M. In yet another particular embodiment of the present invention, in in any of the preceding embodiments directed to an antibody or functional fragment thereof with a light chain comprising a V_{L} comprising, or consisting of, an amino acid sequence shown in SEQ ID NO:32, 33, 34, and 35, the residue X is F. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is S. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is T. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is W. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is Y. In yet another particular embodiment of the present invention, in any of the preceding embodiments directed to an antibody or functional fragment thereof with a light chain comprising a V_{L} comprising, or consisting of, an amino acid sequence shown in SEQ ID NO:32, 33, 34, and 35, the residue X is V.

According to a particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is A. In another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is R. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is N. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is D. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is Q. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is E. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is G. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is H. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is I. In yet another particular embodiment of the present invention, i in any of the preceding embodiments directed to an antibody or functional fragment thereof with a light chain comprising, or consisting of, an amino acid sequence shown in SEQ ID NO:55, 56, 57, or 58, the residue X is L. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is K. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is M. In yet another particular embodiment of the present invention, in in any of the preceding embodiments directed to an antibody or functional fragment thereof with a light chain comprising, or consisting of, an amino acid sequence shown in SEQ ID NO:55, 56, 57, or 58, the residue X is F. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is S. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is T. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is W. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is Y. In yet another particular embodiment of the present invention, in any of the preceding embodiments directed to an antibody or functional fragment thereof with a light chain comprising, or consisting of, an amino acid sequence shown in SEQ ID NO:55, 56, 57, or 58, the residue X is V.

In a further embodiment, the present invention is directed to an antibody or functional fragment thereof binding to essentially the same binding epitope as defined in any of the embodiments described above, but comprising different CDR sequences and/or V_{L} and V_{H} sequences and/or light chain and heavy chain sequences as defined in any of the embodiments above.

### Affinity

The antibody or functional fragment of the invention has a very high affinity to human GPVI. The term "K_{D}," refers to the dissociation equilibrium constant of a particular antibody-antigen interaction. Typically, the antibody or functional fragment of the invention binds to human GPVI with a dissociation equilibrium constant (K_{D}) of less than approximately 1 µM, preferably less than 900 pM, more preferably less than 800 pM, even more preferably less than 700 pM, even more preferably less than 600 pM, even more preferably less than 500 pM, even more preferably less than 400 pM, even more preferably less than 350 pM, even more preferably less than 300 pM, even more preferably less than 250 pM, even more preferably less than 200 pM, e.g. about 195 pM or less, or about 175 pM or less. According to a particularly preferred embodiment, antibody or functional fragment of the invention binds to human GPVI with a dissociation equilibrium constant (K_{D}) of 195 pM or less.

The K_{D} can be readily determined using conventional techniques, such as bio-layer interferometry (BLI), surface plasmon resonance (SPR) technology, or ELISA. According to a specific embodiment, the dissociation equilibrium constant (K_{D}) is determined using BLI, SPR technology (for example in a BIACORE instrument), or ELISA.

Preferably, the dissociation equilibrium constant (K_{D}) is determined using BLI, for example in an Octet instrument and using Fortebio software. In particular, the determination of the K_{D} is carried out preferably as described in the examples, section 1.16. According to another specific embodiment, the dissociation equilibrium constant (K_{D}) is determined at 25 °C by BLI, for example with an Octet instrument preferably at a constant orbital flow rate of e.g. 1000 rpm; using antigen (human GPVI) loading concentration of 1.2 µg/ml for immobilizing of antigen on biosensors, a time for association of 900 s and a time for dissociation of 1200 s, an antibody screening range with seven concentrations ranging from 10 to 0.014 nM using a 3-fold serial dilution, and applying a 1:1 interaction/fitting model.

### Functional properties of the antibody or functional antibody fragment

The antibody or functional fragment of the invention, due to a very high affinity for human GPVI and a high stability in blood plasma, has a high potency to inhibit human GPVI on platelets in blood plasma. In a particular embodiment, the antibody or functional fragment of the invention is capable of sustained inhibition of human GPVI in blood plasma, for at least 12 hours, preferably at least 24 hours, more preferably at least 36 hours, even more preferably at least 48 hours, more preferably at least 60 hours, even more preferably at least 72 hours, even more preferably at least 84 hours, even more preferably at least 96 hours. The antibody or functional fragment of the invention as specified in any of the embodiments herein, preferably has one or more further functional properties described below.

Typically, the antibody or functional fragment of the invention, preferably in monovalent form, e.g. as a Fab, is capable of prolonged binding to GPVI on circulating platelets, while not affecting *in vivo* platelet count, size, as well as GPVI surface levels on circulating platelets.

Binding of an antibody or functional fragment to human GPVI on circulating platelets and GPVI surface levels on circulating platelets can be determined as described e.g. in the examples in section 1.10. Platelet count and size can be determined as described e.g. in the examples in section 1.9.

In one embodiment of the present invention, the antibody or functional fragment of the invention, preferably in monovalent form, e.g. as a Fab, is capable of prolonged binding to circulating platelets, with greater than 50% GPVI receptor epitope occupancy when normalized with a negative control (e.g. control Fab not specific to GPVI), for at least 12, 24, 36, 48, or 60 hours, after intravenous administration, e.g. to a mouse model humanized for GPVI (*hGP6*^{*tg*/*tg*} mouse) at a dose of 4 mg/kg, as determined for example by flow cytometric analysis in diluted blood ex *vivo,* for example using competitive binding with a different anti-GPVI antibody or functional fragment that is fluorescently labeled, and using the mean fluorescence intensity (MFI) as a measure of GPVI receptor epitope occupancy, e.g. as described in the examples in section 1.10.

In another embodiment of the present invention, the antibody or functional fragment of the invention, preferably in monovalent form, e.g. as a Fab, is capable of prolonged binding to circulating platelets, with greater than 50% GPVI receptor epitope occupancy when normalized with a negative control (e.g. control Fab not specific to GPVI), for at least 8, 12, 16, 20, 28 or 28 hours, after intravenous administration, e.g. to a mouse model humanized for GPVI (*hGP6^{tgltg}* mouse) at a dose of 2 mg/kg, as determined for example by flow cytometric analysis in diluted blood ex *vivo,* for example using competitive binding with a different anti-GPVI antibody or functional fragment that is fluorescently labeled, and using the mean fluorescence intensity (MFI) as a measure of GPVI receptor epitope occupancy, e.g. as described in the examples in section 1.10.

In another embodiment of the present invention, the antibody or functional fragment of the invention, preferably in monovalent form, e.g. as a Fab, is capable of prolonged binding to circulating platelets, with at least 20% GPVI receptor epitope occupancy when normalized with a negative control (e.g. control Fab not specific to GPVI), for at least 36, 48, 60, 72, 84, or 96 hours, after intravenous administration e.g. to a mouse model humanized for GPVI (*hGP6*^{*tg*/*tg*} mouse) at a dose of 4 mg/kg, as determined for example by flow cytometric analysis in diluted blood ex *vivo,* for example using competitive binding with a different anti-GPVI antibody or functional fragment that is a fluorescently-labeled, and using the mean fluorescence intensity (MFI) as a measure of GPVI receptor epitope occupancy, e.g. as described in the examples in section 1.10.

In another embodiment of the present invention, the antibody or functional fragment of the invention, preferably in monovalent form, e.g. as a Fab, does not affect GPVI surface levels on circulating platelets, as assessed for example 12, 24, 36, 48, 60, 72, 84, 96, 120, or 144 hours, after intravenous administration, e.g. to a mouse model humanized for GPVI (*hGP6*^{*tg*/*tg*} mouse) at a dose of 4 mg/kg, and determined for example by flow cytometric analysis in diluted blood ex *vivo,* e.g. as described in the examples in section 1.10.

In another embodiment of the present invention, the antibody or functional fragment of the invention, preferably in monovalent form, e.g. a Fab, does not affect platelet count and size of circulating platelets *in vivo,* as assessed for example 12, 24, 36, 48, 60, 72, 84, 96, 120, or 144 hours, after intravenous administration, e.g. to a mouse model humanized for GPVI (*hGP6*^{*tg*/*tg*} mouse) at a dose of 4 mg/kg, and tested ex *vivo* in sampled blood for example as described in the examples in section 1.9.

Typically, the antibody or functional fragment of the invention is capable of complete inhibition of CRP (collagen-related peptide)- and collagen-induced aggregation of washed human platelets. CRP- or collagen-induced aggregation of washed human platelets can be determined using standard light-transmission aggregometry, for example as described in the examples in section 1.7. According to a particular embodiment, antibody or functional fragment of the present invention, at a concentration of ≤ 10 µg/ml, preferably ≤ 5 µg/ml, more preferably ≤ 2 µg/ml, even more preferably ≤ 1 µg/ml, is capable of complete inhibition of CRP- and collagen-induced aggregation of washed human platelets as determined using standard light-transmission aggregometry as described e.g. in the examples in section 1.7.

According to another particular embodiment, the antibody or functional fragment of the present invention, for at least 12, 24, 48, or 60 hours after intravenous administration, e.g. to a mouse model humanized for GPVI (*hGP6*^{*tg*/*tg*} mouse) at a dose of 4 mg/kg, is capable of complete inhibition of CRP- and/or collagen-induced aggregation of washed murine *hGP6*^{*tg*/*tg*} platelets, as determined using standard light-transmission aggregometry, for example as described in the examples in section 1.7.

According to another particular embodiment, compared to ACT017 (glenzocimab), the antibody or functional fragment of the present invention, preferably at a concentration of 10 µg/ml, preferably 5 µg/ml, more preferably 1 µg/ml, has a > 2-fold, preferably > 4-fold, > 6-fold, > 8-fold, or > 10-fold higher potency to inhibit GPVI function in human platelets *in vitro,* measured using standard light-transmission aggregometry, for example as described in the examples in section 1.7. According to another particular embodiment, compared to ACT017 (glenzocimab), the antibody or functional fragment of the present invention, at a concentration of 10 µg/ml, preferably 5 µg/ml, more preferably 1 µg/ml, compared to glenzocimab, has a > 2-fold, preferably greater than > 4-fold, > 6-fold, > 8-fold, or > 10-fold higher potency to inhibit collagen- and/or CRP-induced aggregation of washed human platelets *in vitro* as measured using standard light-transmission aggregometry, for example as described in the examples in section 1.7. Optionally the fold-change in potency is determined as the ratio of percentage maximum aggregation of the antibody or functional fragment treated sample to the percentage maximum aggregation of glenzocimab treated sample.

Typically, the antibody or functional fragment of the invention is capable in heparinized human blood under flow of complete inhibition of human platelet adhesion (determined as platelet surface coverage) and thrombus formation (determined as relative thrombus volume) on a collagen coated surface (preferably coated at 200 µg/ml) in heparinized human blood using a flow adhesion assay.

According to a particular embodiment, the antibody or functional fragment of the present invention, at a concentration of 5 µg/ml, is capable, in heparinized human blood under flow (preferably using a shear rate of 1000 s⁻¹), of complete inhibition of human platelet adhesion (platelet surface coverage) and/or thrombus formation (relative thrombus volume) on a collagen coated surface (preferably coated at 200 µg/ml), as determined using a flow adhesion assay, for example as described in the examples in section 1.5. According to a specific preferred embodiment of the present invention, "complete inhibition" refers to a reduction of platelet surface coverage by at least by at least 60 %, 65 %, or 70 %, and/or a reduction of relative thrombus volume by at least 80 %, 85 %, 86 %, 87 %, 88 %, 89 %, 90 %, etc., compared to a negative control (e.g. a control Fab, if a functional fragment in the form of an anti-GPVI Fab is used), for example as described in the examples in section 1.5.

According to another particular embodiment, the antibody or functional fragment of the present invention, at a concentration of ≤ 10 µg/ml, preferably ≤ 5 µg/ml, more preferably ≤ 2 µg/ml, even more preferably ≤ 1 µg/ml, is capable, in heparinized human blood under flow (preferably using a shear rate of 1000 s-1), of reducing, in a flow adhesion assay for example as described in the examples in section 1.5, the relative thrombus volume on a collagen coated surface (preferably coated at 200 µg/ml), by at least 50 %, 60 %, 65 %, 70 %, 75 %, 80 %, 85 %, 90 %, or 95 % compared to a negative control (e.g. a control Fab, if a functional fragment in the form of an anti-GPVI Fab is used). According to another preferred embodiment, the antibody or functional fragment of the present invention, for at least 12, 24, 48, or 60 hours, after intravenous administration to a mouse model humanized for GPVI (*hGP6*^{*tg*/*tg*} mouse) at a dose of 4 mg/kg is capable, in heparinized human blood under flow (preferably using a shear rate of 1000 s-1), of reducing, in a flow adhesion assay for example as described in the examples in section 1.5, the relative thrombus volume on a collagen coated surface (preferably coated at 200 µg/ml), by at least 50 %, 55%, 60 %, or 60%, compared to a negative control.

According to another particular embodiment, the antibody or functional fragment of the present invention, at a concentration of 10 µg/ml, preferably 5 µg/ml, more preferably 1 µg/ml, is capable, in heparinized human blood under flow (preferably using a shear rate of 1000 s-1), of significantly reducing (e.g. by greater than 1.5-fold, preferably greater than 2-fold, 2.5-fold, or 3-fold), in a flow adhesion assay (for example as described in the examples in section 1.5), the platelet surface coverage on a collagen coated surface (preferably coated at 200 µg/ml), compared to a negative control, while a ACT017 (glenzocimab) treated sample does not result in a significant reduction.

According to another particular embodiment, the antibody or functional fragment of the present invention, at a concentration of 10 µg/ml, preferably 5 µg/ml, more preferably 1 µg/ml, is capable, in heparinized human blood under flow (preferably using a shear rate of 1000 s-1), of reducing, in a flow adhesion assay (for example as described in the examples in section 1.5), the platelet surface coverage on a collagen coated surface (preferably coated at 200 µg/ml), by greater than 1.5-fold, preferably greater than 2-fold, 2.5-fold, or 3-fold, compared to a ACT017 (glenzocimab) treated sample. According to another particular embodiment, the antibody or functional fragment of the present invention, at a concentration of 10 µg/ml, preferably 5 µg/ml, more preferably 1 µg/ml, is capable, in heparinized human blood under flow (preferably using a shear rate of 1000 s-1), of reducing, in a flow adhesion assay (for example as described in the examples in section 1.5), the relative thrombus volume on a collagen coated surface (preferably coated at 200 µg/ml), by greater than 2-fold, preferably greater than 4-fold, 6-fold, 8-fold, or 10-fold, compared to a glenzocimab treated sample.

Typically, the antibody or functional fragment of the invention is capable, in citrated and recalcified human blood under flow (preferably using a shear rate of 1000 s⁻¹) of complete inhibition of platelet deposition, thrombus formation, phosphatidylserine (PS)-exposure and/or fibrin deposition, on a surface coated with collagen and tissue factor (preferably at 200 µg/ml and 500 pM for collagen and tissue factor, respectively), as determined using a flow adhesion assay adapted for coagulation (for example as described in the examples in section 1.6).

According to a particular embodiment, the antibody or functional fragment of the present invention, at a concentration of 10 µg/ml, is capable, in citrated and recalcified human blood under flow (preferably using a shear rate of 1000 s⁻¹), of complete inhibition of thrombus formation (determined as relative thrombus volume), phosphatidylserine (PS)-exposure and fibrin deposition, on a surface coated with collagen and tissue factor (preferably at 200 µg/ml and 500 pM for collagen and tissue factor, respectively), as determined in a flow adhesion assay adapted for coagulation for example as described in the examples in section 1.6. According to a specific particular embodiment of the present invention, "complete inhibition" refers to a reduction of relative thrombus volume / phosphatidylserine (PS)-exposure / fibrin deposition by at least 85%, 90 %, 93%, 95 %, 97%, or 99%, compared to a negative control, for example as described in the examples in section 1.6.

Typically, the antibody or functional fragment of the invention is capable, when used on washed human platelets that are allowed binding to a fibrinogen-coated surface, in a spreading assay for example as described in the examples in section 1.8, to significantly reduce the portion of phase 4 (fully spread) platelets while increasing the portion of phase 2 (filopodia-forming platelets) by similar extent.

According to a particular embodiment, the antibody or functional fragment of the present invention, when used at a concentration of 10 µg/ml in a spreading assay (for example as described in the examples in section 1.8) on washed human platelet that are allowed binding to a fibrinogen-coated surface, is capable of reducing the portion of phase 4 (fully spread) platelets by at least 30 %, 35 %, 40 %, 45 %, 50 %, or 55 %, and increasing the portion of phase 2 (filopodia-forming platelets) by similar extent, compared to a negative control.

Typically, the antibody or functional fragment of the invention, preferably in monovalent form (e.g. as a Fab), causes a significantly impaired CRP-induced activation of circulating platelets for at least 12 hours, at least 24 hours, at least 36 hours, at least 48 hours, at least 60 hours, at least 72 hours, at least 84 hours, or at least 96 hours, after intravenous administration e.g. to a mouse model humanized for GPVI (*hGP6*^{*tg*/*tg*} mouse) at a dose of 4 mg/kg, as determined for example by flow cytometric analysis in diluted blood *ex vivo,* preferably using a fluorescentlabeled antibody capable of specifically binding activated integrin αllbβ3 and/or a fluorescentlabeled antibody capable of specifically binding P-selectin, e.g. as described in the examples in section 1.10. In this context "significantly impaired" preferably means a reduction of fluorescent signal of >50% when using circulating platelets sampled at least 12, 24, 36, 48, 60, 72, 84, or 96 hours post-administration, compared to a negative control, as for example determined in the examples in section 1.10.

According to a particular embodiment, the antibody or functional fragment of the present invention, in monovalent form, preferably as a Fab, is capable of increased binding to circulating platelets (preferably with a ≥ 1.5-fold, more preferably ≥ 1.7-fold, ≥ 1.8-fold, or ≥ 1.9-fold increase in GPVI receptor epitope binding) compared to ACT017 (glenzocimab), when normalized with a negative control (e.g. control Fab not specific to GPVI), measured 1 hour after intravenous administration of e.g. 4 mg/kg anti-GPVI antibody or functional fragment or glenzocimab, as determined for example by flow cytometric analysis in diluted blood ex *vivo,* e.g. with a fluorescently-labeled Fab specific antibody and using the mean fluorescence intensity (MFI) as a measure of GPVI receptor epitope binding, e.g. as described in the examples in section 1.10.

According to another particular embodiment, the antibody or functional fragment of the present invention, in monovalent form, preferably as a Fab, is capable of increased binding to circulating platelets (preferably with a ≥ 2-fold, more preferably ≥ 2.5-fold, ≥ 3-fold, ≥ 3.5-fold, or ≥ 3.75-fold, increase in GPVI receptor epitope binding) compared to glenzocimab, when normalized with a negative control (e.g. control Fab not specific to GPVI), measured 3 hour after intravenous administration of e.g. 4 mg/kg anti-GPVI antibody or functional fragment or glenzocimab, as determined for example by flow cytometric analysis in diluted blood ex *vivo,* e.g. with a fluorescently-labeled Fab specific antibody and using the mean fluorescence intensity (MFI) as a measure of GPVI receptor epitope binding, e.g. as described in the examples in section 1.10.

According to another particular embodiment, the antibody or functional fragment of the present invention, preferably in monovalent form, more preferably as a Fab, causes a significantly impaired CRP-induced activation of circulating platelets 1 hour after intravenous administration e.g. to a mouse model humanized for GPVI (*hGP6*^{*tg*/*tg*} mouse) at a dose of 4 mg/kg, as determined for example by flow cytometric analysis in diluted blood ex *vivo,* preferably using a fluorescently labeled antibody capable of specifically binding activated integrin αllbβ3, e.g. as described in the examples in section 1.10, while ACT017 (glenzocimab) does not causes a significantly impaired CRP-induced activation. Optionally, "significantly impaired" refers to a reduction of fluorescent signal of >85%, preferably >90%, >95%, >97%, >98%, >99%, or >99.5%, compared to a negative control.

According to a particular embodiment, the antibody or functional fragment of the invention, preferably in monovalent form (e.g. as a Fab), is capable of inhibiting dimerization of GPVI, optionally while not or not completely abolishing ligand binding to GPVI. Typically, the antibody or functional fragment of the invention, preferably in monovalent form (e.g. a Fab), is capable of inhibiting GPVI-induced activation of platelets, optionally while leaving the initial adhesive functions of the GPVI receptor intact.

Typically, the antibody or functional fragment of the invention is capable of sustained protection from occlusive thrombosis, e.g. arterial thrombosis, as determined for example using a mouse model of occlusive arterial thrombus formation that is humanized for GPVI (*hGP6*^{*tg*/*tg*} mouse), as described e.g. in the examples in section 1.11.

According to a specific embodiment, the antibody or functional fragment of the present invention, is capable of inhibiting the formation of stable thrombi at sites of arterial injury, preferably through reduced platelet activation at a site of exposed extracellular matrix inside a blood vessel and/or by potent inhibition of local platelet-dependent coagulation, optionally as determined in a mouse model of occlusive arterial thrombus formation that is humanized for GPVI (*hGP6*^{*tg*/*tg*} mouse), as described e.g. in the examples in section 1.11.

Typically, the *in vivo* use of the antibody or functional fragment of the invention, preferably the Fab, is associated with low hemorrhagic risk, for example by not significantly increasing bleeding times, for example as determined using a tail bleeding assay as described in the examples in section 1.12.

In certain embodiments, the antibody or the functional fragment of the invention has a high target selectivity, i.e. it can discriminate between GPVI and a structurally closely related protein. Preferably, the IC₅₀ value of structurally closely related protein is at least 1,000 times, preferably at least 5,000 times, more preferably at least 10,000, greater than the IC₅₀ value of GPVI, as determined in a competition BLI, ELISA, or SPR assay.

Typically, the antibody or functional fragment of the invention, preferably the Fab, has a high stability. Stability can be assessed by different methodologies. The "melting temperature" Tₘ of the Fab can be determined by differential scanning fluorimetry (DSF). Stability can also be assessed by prolonged binding to GPVI on circulating platelets, for example as determined in the examples in section 1.10. In one embodiment, the antibody or functional fragment of the invention, is capable of prolonged binding to circulating platelets, with greater than 50% GPVI receptor epitope occupancy when normalized with a negative control (e.g. control antibody or functional fragment not specific to GPVI), for at least 12, 24, 36, 48, 60, 72, 84, or 96 hours, when administered *in vivo,* such as, for example, when administered at a dose ranging from 0.01 to 500 mg, preferably to a human subject, for example as determined in the examples in section 1.10.

In one embodiment, the antibody or functional fragment of the invention does not induce a decrease in platelet count, i.e. thrombocytopenia, when administered *in vivo,* such as, for example, when administered at a dose ranging from 0.01 to 500 mg preferably to a human subject. In another embodiment, the antibody or functional fragment of the invention does not induce a decrease in GPVI surface levels on platelets, when administered *in vivo,* such as, for example, when administered at a dose ranging from 0.01 to 500 mg preferably to a human subject.

### Antibodies and functional fragments

Certain preferred embodiments of the invention relate to functional fragments of the antibodies described herein. Functional fragments include, but are not limited to, Fab, F(ab'), F(ab')₂, Fv, monovalent IgG, dsFv, scFv, diabodies, triabodies and tetrabodies. Preferably the functional fragment is monovalent, including, but not limited to, Fab, F(ab'), Fv, monovalent IgG, dsFv, and scFv. In a particularly preferred embodiment, the functional fragment is a fragment antigen-binding (Fab). More preferably, the non-CDR sequences of the Fab are human sequences.

Preferably the antibody or functional fragment thereof is a monoclonal antibody or a monoclonal antibody fragment. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Monoclonal antibodies can be prepared using a wide variety of techniques known in the art including the use of hybridoma, recombinant, and phage display technologies, or a combination thereof. (Harlow and Lane, "Antibodies, A Laboratory Manual" CSH Press 1988, Cold Spring Harbor N.Y.).

In other embodiments, including embodiments relating to the *in vivo* use of the anti-GPVI antibodies or functional fragments thereof in humans, chimeric, primatized, humanized, or human monovalent antibodies and antibody fragment can be used. In a preferred embodiment, the antibody or functional fragment thereof is a human or humanized antibody or functional fragment thereof, more preferably a monoclonal human a humanized antibody or functional fragment thereof, even more preferably a monovalent monoclonal human or humanized antibody or functional fragment thereof.

The term "chimeric" antibody or antibody fragment as used herein refers to an antibody having variable sequences derived from a non-human immunoglobulin, such as a rat or mouse antibody, and human immunoglobulin constant regions, typically chosen from a human immunoglobulin template. Methods for producing chimeric antibodies are known in the art. See, e.g. Morrison, 1985, Science 229(4719): 1202-7; Oi et al, 1986, BioTechniques 4:214-221; Gillies et al., 1985, J. Immunol. Methods 125: 191-202; U.S. Pat. Nos. 5,807,715; 4,816,567; and 4,816397, which are incorporated herein by reference in their entireties.

In some embodiments, the anti-GPVI antibodies or functional fragments thereof are humanized antibodies or functional fragments thereof. Different recombinant methodologies are available to one of ordinary skill in the art to render a non-human (e.g. murine) antibody more humanlike by generating immunoglobulins, immunoglobulin chains or fragments thereof (such as Fv, Fab, F(ab'), F(ab')₂ or other target-binding subsequences of antibodies), which contain minimal sequences derived from such non-human immunoglobulin. In general, the resulting recombinant antibody will comprise substantially all of at least one, and typically two, variable domains, in which all or substantially all of the CDR regions correspond to those of a non-human immunoglobulin and all or substantially all of the FR regions are those of a human immunoglobulin sequence, particularly a human immunoglobulin consensus sequence. CDR-grafted antibodies are antibody molecules having one or more complementarity determining regions (CDRs) from an antibody originally generated in a non-human species that bind the desired antigen and framework (FR) regions from a human immunoglobulin molecule (EP239400; PCT publication WO 91/09967; U.S. Patent Nos. 5,225,539; 5,530,101 and 5,585,089). Often, in a process called "humanization", framework residues in the human framework regions will additionally be substituted with the corresponding residue from the CDR donor antibody to alter, preferably improve, antigen binding. These framework substitutions are identified by methods well known in the art, e.g. by modeling of the interactions of the CDR and framework residues to identify framework residues important for antigen binding and sequence comparison to identify unusual framework residues at particular positions. See, e.g. Riechmann et al., 1988, Nature 332:323-7 and Queen et al, U.S. Patent Nos: 5,530,101; 5,585,089; 5,693,761; 5,693,762; and 6,180,370 (each of which is incorporated by reference in its entirety). Antibodies can be rendered more human using a variety of additional techniques known in the art including, for example, veneering or resurfacing (EP592106; EP519596; Padlan, 1991, Mol. Immunol, 28:489-498; Studnicka et al, 1994, Prot. Eng. 7:805-814; Roguska et al, 1994, Proc. Natl. Acad. Sci. 91:969-973, and chain shuffling (U.S. Patent No. 5,565,332), all of which are hereby incorporated by reference in their entireties. A CDR-grafted or humanized antibody can also comprise at least a portion of an immunoglobulin constant region (Fc), typically that of a human immunoglobulin template chosen.

In some embodiments, humanized antibodies or functional fragments thereof are prepared as described in Queen et al, U.S. Patent Nos: 5,530,101; 5,585,089; 5,693,761; 5,693,762; and 6,180,370.

In some embodiments, the anti-GPVI antibodies or functional fragments thereof are human antibodies or functional fragments. Completely "human" anti-GPVI antibodies can be desirable for therapeutic treatment of human patients. As used herein, "human antibodies or functional fragments" include antibodies having the amino acid sequence of a human immunoglobulin and include antibodies isolated from human immunoglobulin libraries or from animals transgenic for one or more human immunoglobulin and that do not express endogenous immunoglobulins. Human antibodies can be made by a variety of methods known in the art including phage display methods described above using antibody libraries derived from human immunoglobulin sequences. See U.S. Patent Nos. 4,444,887 and 4,716,111; and PCT publications WO 98/46645; WO 98/50433; WO 98/24893; WO 98/16654; WO 96/34096; WO 96/33735; and WO 91/10741, each of which is incorporated herein by reference in its entirety. Human antibodies can also be produced using transgenic mice which are incapable of expressing functional endogenous immunoglobulins, but which can express human immunoglobulin genes. See, e.g. PCT publications WO 98/24893; WO 92/01047; WO 96/34096; WO 96/33735; U.S. Patent Nos. 5,413,923; 5,625,126; 5,633,425; 5,569,825; 5,661,016; 5,545,806; 5,814,318; 5,885,793; 5,916,771; and 5,939,598, which are incorporated by reference herein in their entireties. Completely human antibodies that recognize a selected epitope can be generated using a technique referred to as "guided selection." In this approach a selected non-human monoclonal antibody, e.g. a mouse antibody, is used to guide the selection of a completely human antibody recognizing the same epitope (Jespers et al, 1988, Biotechnology 12:899-903).

In some embodiments, the anti-GPVI antibodies or functional fragments thereof are derivatized. For example, but not by way of limitation, the derivatized antibodies or functional fragments that have been modified, e.g. by glycosylation, acetylation, pegylation, phosphorylation, amidation, derivatization by known protecting/blocking groups, proteolytic cleavage, linkage to a cellular ligand or other protein (see below for a discussion of antibody conjugates), etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

In yet other aspects, an anti-GPVI antibody has one or more amino acids inserted into one or more of its hypervariable regions, for example as described in US 2007/0280931.

### Antibody Conjugates

In some embodiments, the anti-GPVI antibodies or functional fragments are antibody / antibody fragment conjugates that are modified, e.g. by the covalent attachment of any type of molecule (e.g. an effector molecule) to the antibody, such that covalent attachment does not interfere with binding to GPVI. Techniques for conjugating effector moieties to antibodies are well known in the art (See, e.g. Hellstrom et al., Controlled Drag Delivery, 2nd Ed., at pp. 623-53 (Robinson et al., eds., 1987)); Thorpe et al., 1982, Immunol. Rev. 62: 119-58 and Dubowchik et al., 1999, Pharmacology and Therapeutics 83:67-123).

In one example, the antibody or functional fragment thereof is fused via a covalent bond (e.g. a peptide bond), at optionally the N-terminus or the C-terminus, to an amino acid sequence of another protein (or portion thereof; preferably at least a 10, 20 or 50 amino acid portion of the protein). In one embodiment the antibody or functional fragment thereof is linked to the other protein at the C-terminus of the constant domain of the antibody or functional fragment. Recombinant DNA procedures can be used to create such fusions, for example as described in WO 86/01533 and EP0392745. In another example the effector molecule can increase halflife *in vivo.* Examples of suitable effector molecules of this type include polymers, albumin, albumin binding proteins or albumin binding compounds such as those described in WO 2005/117984.

In some embodiments, anti-GPVI antibodies or functional fragment thereof can be attached to poly(ethyleneglycol) (PEG) moieties. For example, if the antibody is an antibody fragment, the PEG moieties can be attached through any available amino acid side-chain or terminal amino acid functional group located in the antibody fragment, for example any free amino, imino, thiol, hydroxyl or carboxyl group. Such amino acids can occur naturally in the antibody fragment or can be engineered into the fragment using recombinant DNA methods. See for example U.S. Patent No. 5,219,996. Multiple sites can be used to attach two or more PEG molecules. Preferably PEG moieties are covalently linked through a thiol group of at least one cysteine residue located in the antibody fragment. Where a thiol group is used as the point of attachment, appropriately activated effector moieties, for example thiol selective derivatives such as maleimides and cysteine derivatives, can be used.

In another example, an anti-GPVI antibody conjugate is a modified Fab or F(ab') fragment, which is PEGylated, i.e. has PEG (poly(ethyleneglycol)) covalently attached thereto, e.g. according to the method disclosed in EP0948544. See also Poly(ethyleneglycol) Chemistry, Biotechnical and Biomedical Applications, (J. Milton Harris (ed.), Plenum Press, New York, 1992); Poly(ethyleneglycol) Chemistry and Biological Applications, (J. Milton Harris and S. Zalipsky, eds., American Chemical Society, Washington D. C, 1997); and Bioconjugation Protein Coupling Techniques for the Biomedical Sciences, (M. Aslam and A. Dent, eds., Grove Publishers, New York, 1998); and Chapman, 2002, Advanced Drug Delivery Reviews 54:531-545.

### Pharmaceutical Compositions and Treatment

Treatment of a disease encompasses the treatment of patients already diagnosed as having any form of the disease at any clinical stage or manifestation; the delay of the onset or evolution or aggravation or deterioration of the symptoms or signs of the disease; and/or preventing and/or reducing the severity of the disease.

A "subject" or "patient" to whom an anti-GPVI antibody or functional fragment thereof is administered can be a mammal, such as a non-primate (e.g. cow, pig, horse, cat, dog, rat, etc.) or a primate (e.g. monkey or human). Preferably the "subject" or "patient" is human. In certain aspects, the human is an adult patient. In other aspects, the human is a pediatric patient.

Pharmaceutical compositions comprising an anti-GPVI antibody and optionally a pharmaceutically acceptable carrier and/or excipient and, optionally one or more additional therapeutic agents, are described herein. The compositions typically are supplied as part of a sterile, pharmaceutical composition that includes a pharmaceutically acceptable carrier. This composition can be in any suitable form (depending upon the desired method of administering it to a patient).

The anti-GPVI antibodies and functional fragments can be administered to a patient by a variety of routes such as orally, transdermally, subcutaneously, intranasally, intravenously, intramuscularly, intrathecally, topically or locally. Typically, an anti-GPVI antibody or functional fragment thereof will be administered to a patient intravenously. In typical embodiments, an anti-GPVI antibody or functional fragment is present in a pharmaceutical composition at a concentration sufficient to permit intravenous administration at 0.5 mg/kg body weight to 20 mg/kg body weight. In some embodiments, the concentration of antibody or fragment suitable for use in the compositions and methods described herein includes, but is not limited to, 0.5 mg/kg, 0.75 mg/kg, 1 mg/kg, 2 mg/kg, 2.5 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, or a concentration ranging between any of the foregoing values, e.g. 1 mg/kg to 10 mg/kg, 5 mg/kg to 15 mg/kg, or 10 mg/kg to 18 mg/kg.

The effective dose of an anti-GPVI antibody or functional fragment can range from about 0.001 to about 750 mg/kg per single (e.g. bolus) administration, multiple administrations or continuous administration, or to achieve a serum concentration of 0.01-5000 µg/ml serum concentration per single (e.g. bolus) administration, multiple administrations or continuous administration, or any effective range or value therein depending on the condition being treated, the route of administration and the age, weight and condition of the subject. In certain embodiments, each dose can range from about 0.5 mg to about 50 mg per kilogram of body weight or from about 3 mg to about 30 mg per kilogram body weight. The antibody can be formulated as an aqueous solution.

Pharmaceutical compositions can be conveniently presented in unit dose forms containing a predetermined amount of an anti-GPVI antibody or functional fragment per dose. Such a unit can contain 0.5 mg to 5 g, for example, but without limitation, 1 mg, 10 mg, 20 mg, 30 mg, 40 mg, 50 mg, 100 mg, 200 mg, 300 mg, 400 mg, 500 mg, 750 mg, 1000 mg, 2000 mg or any range between any two of the foregoing values, for example 10 mg to 1000 mg, 20 mg to 500 mg, or 30 mg to 300 mg. Pharmaceutically acceptable carriers can take a wide variety of forms depending, e.g. on the condition to be treated or route of administration.

Determination of the effective dosage, total number of doses, and length of treatment an anti-GPVI antibody or functional fragment thereof is within the capabilities of those skilled in the art, and can be determined using a standard dose escalation study.

Therapeutic formulations of the anti-GPVI antibodies and functional fragments suitable in the methods described herein can be prepared for storage as lyophilized formulations or aqueous solutions by mixing the antibody or functional antibody fragment having the desired degree of purity with optional pharmaceutically-acceptable carriers, excipients or stabilizers typically employed in the art (all of which are referred to herein as "carriers"), i.e., buffering agents, stabilizing agents, preservatives, isotonifiers, non-ionic detergents, antioxidants, and other miscellaneous additives. See, Remington's Pharmaceutical Sciences, 16th edition (Osol, ed. 1980). Such additives must be nontoxic to the recipients at the dosages and concentrations employed.

Buffering agents help to maintain the pH in the range which approximates physiological conditions. They can present at concentration ranging from about 2 mM to about 50 mM. Suitable buffering agents include both organic and inorganic acids and salts thereof such as citrate buffers (e.g. monosodium citrate-disodium citrate mixture, citric acid-trisodium citrate mixture, citric acid-monosodium citrate mixture, etc.), succinate buffers (e.g. succinic acid-monosodium succinate mixture, succinic acid-sodium hydroxide mixture, succinic acid-disodium succinate mixture, etc.), tartrate buffers (e.g. tartaric acid-sodium tartrate mixture, tartaric acid-potassium tartrate mixture, tartaric acid-sodium hydroxide mixture, etc.), fumarate buffers (e.g. fumaric acid-monosodium fumarate mixture, fumaric acid-disodium fumarate mixture, monosodium fumarate-disodium fumarate mixture, etc.), gluconate buffers (e.g. gluconic acid-sodium gluconate mixture, gluconic acid-sodium hydroxide mixture, gluconic acid-potassium gluconate mixture, etc.), oxalate buffer (e.g. oxalic acid-sodium oxalate mixture, oxalic acid-sodium hydroxide mixture, oxalic acid-potassium oxalate mixture, etc.), lactate buffers (e.g. lactic acid-sodium lactate mixture, lactic acid-sodium hydroxide mixture, lactic acid-potassium lactate mixture, etc.) and acetate buffers (e.g. acetic acid-sodium acetate mixture, acetic acid-sodium hydroxide mixture, etc.). Additionally, phosphate buffers, histidine buffers and trimethylamine salts such as Tris can be used.

Preservatives can be added to retard microbial growth, and can be added in amounts ranging from 0.2%- 1% (w/v). Suitable preservatives include phenol, benzyl alcohol, metacresol, methyl paraben, propyl paraben, octadecyldimethylbenzyl ammonium chloride, benzalconium halides (e.g. chloride, bromide, and iodide), hexamethonium chloride, and alkyl parabens such as methyl or propyl paraben, catechol, resorcinol, cyclohexanol, and 3-pentanol. Isotonicifiers sometimes known as "stabilizers" can be added to ensure isotonicity of liquid compositions and include polyhydric sugar alcohols, preferably trihydric or higher sugar alcohols, such as glycerin, erythritol, arabitol, xylitol, sorbitol and mannitol. Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (e.g. peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran. Stabilizers can be present in the range from 0.1 to 10,000 weights per part of weight active protein.

Non-ionic surfactants or detergents (also known as "wetting agents") can be added to help solubilize the therapeutic agent as well as to protect the therapeutic protein against agitationinduced aggregation, which also permits the formulation to be exposed to shear surface stressed without causing denaturation of the protein. Suitable non-ionic surfactants include polysorbates (20, 80, etc.), polyoxamers (184, 188 etc.), Pluronic polyols, polyoxyethylene sorbitan monoethers (TWEEN^{®}-20, TWEEN^{®}-80, etc.). Non-ionic surfactants can be present in a range of about 0.05 mg/ml to about 1.0 mg/ml, or in a range of about 0.07 mg/ml to about 0.2 mg/ml.

Additional miscellaneous excipients include bulking agents (e.g. starch), chelating agents (e.g. EDTA), antioxidants (e.g. ascorbic acid, methionine, vitamin E), protease inhibitors and cosolvents.

The formulation herein can also contain a second therapeutic agent in addition to an anti-GPVI antibody or functional fragment thereof.

The dosing schedule can vary from once a month to daily depending on a number of clinical factors, including the type of disease, severity of disease, and the patient's sensitivity to the anti-GPVI antibody or functional fragment. In specific embodiments, an anti-GPVI antibody or functional fragment thereof is administered daily, twice weekly, three times a week, every other day, or every 5 days, etc.

The dosage of an anti-GPVI antibody or functional fragment to be administered will vary according to the particular antibody or functional fragment, the subject, and the nature and severity of the disease, the physical condition of the subject, the therapeutic regimen (e.g. whether a second therapeutic agent is used), and the selected route of administration; the appropriate dosage can be readily determined by a person skilled in the art.

It will be recognized by one of skill in the art that the optimal quantity and spacing of individual dosages of an anti-GPVI antibody or functional fragment thereof will be determined by the nature and extent of the condition being treated, the form, route and site of administration, and the age and condition of the particular subject being treated, and that a physician will ultimately determine appropriate dosages to be used. This dosage can be repeated as often as appropriate. If side effects develop the amount and/or frequency of the dosage can be altered or reduced, in accordance with normal clinical practice.

### Disorders to be treated

The invention further relates to a method of treating or preventing a GPVI-related condition in a subject, comprising administering to the subject the antibody or functional fragment as defined herein. Moreover, the present invention also relates to the antibody or functional fragment as defined in any one of the embodiments above, or a pharmaceutical composition comprising such antibody or functional fragment, for use in a method of treating or preventing a GPVI-related condition in a subject.

The term "GPVI-related condition" or "GPVI-related disease" as used herein specifically and exclusively refers to such disorders, the onset, progression or persistence of one or more symptoms or disease states of which involves the participation of GPVI. Thus, the "GPVI-related condition" or "GPVI-related disease", as used herein, is any condition/disease of which one or more symptoms or disease states can be treated or prevented by the (preferably functional) inhibition/blockage of GPVI through administration of an antibody capable of binding to GPVI. Exemplary GPVI-related conditions include, but are not limited to, thrombo-inflammatory diseases, cardiovascular diseases, cancer/cancer-related diseases, inflammation, and disorders associated with abnormal or aberrant megakaryocyte and/or platelet proliferation, differentiation, morphology, migration, aggregation, degranulation and/or function. As used herein, these categories of GPVI-related conditions are not necessarily mutually exclusive and may overlap. Similarly, as used herein, the exemplary conditions, diseases, disorders, disease states and the like in the different categories of GPVI-related conditions as detailed below are not necessarily mutually exclusive and may for example overlap. According to one embodiment, the GPVI-related condition is a thrombo-inflammatory disease, a cardiovascular disease, inflammation, a cancer/cancer-related disease, and/or a disorder associated with abnormal or aberrant megakaryocyte and/or platelet proliferation, differentiation, morphology, migration, aggregation, degranulation and/or function.

Exemplary cardiovascular diseases include, but are not limited to, thrombosis and thrombotic disorders (such as arterial thrombosis, venous thrombosis, atherothrombosis, stent thrombosis, venous thromboembolism diseases [such as, for example, diseases involving leg swelling, pain and ulceration, pulmonary embolism, abdominal venous thrombosis], thrombotic occlusion of coronary arteries, thrombotic microangiopathies, cancer-associated thrombosis (Trousseau syndrome), immunothrombosis and thrombosis associated to infection [e.g. cerebral malaria]), restenosis, ischemic cerebrovascular accidents, cerebral vascular diseases, vascular purpura, coronary disease [such as coronary artery diseases (e. g., arterial occlusive disorders), myocardial infarction, coronary artery revascularization, coronary restenosis, acute coronary syndrome, acute coronary artery syndrome cardiac ischemia, including complications related to coronary procedures, such as percutaneous coronary artery angioplasty (balloon angioplasty) procedures, percutaneous coronary intervention], atherosclerosis, plaque formation, cerebral artery diseases, ischemic events, unstable angina pectoris, acute cerebrovascular ischemia (stroke), ischemic restenosis, acute ischemia, chronic ischemia, diseases of the aorta and its branches, peripheral artery disease, acute phlebitis, pulmonary embolism, and a disorder resulting from any blood vessel damage that can cause platelet aggregation.

According to a specific embodiment, the GPVI-related condition is a cardiovascular disease, preferably selected from the cardiovascular diseases specified above. With respect to coronary procedures, such treatment can be achieved via administration of a protein of the invention prior to, during, or after the procedure. In a preferred embodiment, such administration can be utilized to prevent acute cardiac ischemia following angioplasty.

In another embodiment, the GPVI-related condition is thrombosis or a thrombotic disorder, a disease exhibiting quantitative or qualitative platelet dysfunction or a disease displaying endothelial dysfunction. These diseases include, but are not limited to, coronary artery and cerebral artery diseases. Generally, a thrombotic disorder as used herein may refer to a disorder associated with pathologic formation of thrombi in veins (e.g. deep venous thrombosis [DVT]), arteries (e.g. myocardial infarction, ischemic stroke), or cardiac chambers. According to a preferred embodiment, the GPVI-related condition is thrombosis or a thrombotic disorder (including but not limited to arterial thrombosis, venous thrombosis, atherothrombosis, stent thrombosis, venous thromboembolism diseases, thrombotic occlusion of coronary arteries, thrombotic microangiopathies, cancer-associated thrombosis, immunothrombosis and thrombosis associated to infection [e.g. cerebral malaria]).

In another embodiment, the GPVI-related condition is a disorder resulting from any blood vessel damage that can cause platelet aggregation. The term "blood vessel damage" as used herein includes, but is not limited to, vessel wall injury, such as vessel injuries that result in a highly thrombogenic surface exposed within an otherwise intact blood vessel e. g., vessel wall injuries that result in release of ADP, thrombin and/or epinephrine, fluid shear stress that occurs at the site of vessel narrowing, ruptures and/or tears at the sites of atherosclerotic plaques, and injury resulting from balloon angioplasty or atherectomy.

According to another embodiment, the GPVI-related condition is inflammation, that is inflammation or thrombo-inflammation, including, but not limited to, sustained or prolonged inflammation associated with infection [e.g. cerebral malaria], arthritis, fibrosis, acute respiratory distress syndrome (ARDS), ischemia-reperfusion injury (IRI) of various organs (liver, colon, etc.), peripheral vascular disease, antiphospholipid syndrome (APS), deep vein thrombosis, thrombophlebitis & vasculitis, transfusion-related acute lung injury (TRALI), transplant rejection, pre-eclampsia, severe burns, atherosclerosis, hypertension, antiphospholipid syndrome, sickle cell disease, bacterial and viral infection, ischemic restenosis, sepsis, major trauma, autoimmune diseases and disorders in which platelets modulate cell functions including, without limitation, cancer cells proliferation and/or dissemination. As used herein, the term "thrombo-inflammatory disease" preferably refers to a disease involving the activating interplay of platelets/coagulation and components of the immune system. A disorder/disease associated with the term "GPVI-related condition is inflammation or thrombo-inflammation", may also be a "thrombo-inflammatory disorder" or "thrombo-inflammatory disease". Similarly, for example a cardiovascular disease may be a thrombo-inflammatory disease. Autoimmune diseases include, without limitation, celiac disease, post-infectious IBS, diabetes mellitus type 1, Henoch-Schönlein purpura (HSP) sarcoidosis, systemic lupus erythematosus (SLE), Sjögren syndrome, eosinophilic granulomatosis with polyangiitis, Hashimoto's thyroiditis, Graves' disease, idiopathic thrombocytopenic purpura, Addison's disease, rheumatoid arthritis (RA), ankylosing spondylitis, polymyositis (PM), dermatomyositis (DM), Alopecia Areata and multiple sclerosis (MS).

According to another embodiment, the GPVI-related condition is cancer (including, but not limited to, colon cancer, breast cancer, ovarian cancer, lung cancer, skin cancer such as malignant cutaneous melanoma, metastatic cancer, etc.).

According to yet another embodiment, the GPVI-related condition is a disorder associated with abnormal or aberrant megakaryocyte and/or platelet proliferation, differentiation, morphology, migration, aggregation, degranulation and/or function.

In another embodiment, the antibody or functional fragment of the present invention or the pharmaceutical composition as described hereinabove is used to modulate, preferably to prevent, platelet aggregation and degranulation. In another embodiment, the antibody or functional fragment of the present invention or the pharmaceutical composition as described hereinabove is used to modulate immunoregulatory functions of platelets. In another embodiment, the antibody or functional fragment of the present invention or the pharmaceutical composition as described hereinabove is used to treat disorders of liver, bone marrow and peripheral blood.

Preferably, the antibody or functional fragment of the invention is used to treat a cardiovascular disease selected from thrombosis and thrombotic disorders such as arterial thrombosis, venous thrombosis, atherothrombosis, stent thrombosis, venous thromboembolism diseases, thrombotic microangiopathies, cancer-associated thrombosis [Trousseau syndrome], immunothrombosis and thrombosis associated to infection [e.g. cerebral malaria], restenosis, acute coronary syndrome, ischemic cerebrovascular accidents, cerebral vascular diseases, and vascular purpura, coronary artery diseases and cerebral artery diseases, ischemic events, acute coronary artery syndrome, myocardial infarction (heart attack), acute cerebrovascular ischemia (stroke), percutaneous coronary intervention, ischemic restenosis, acute ischemia, chronic ischemia, diseases of the aorta and its branches (such as aortic aneurysm, thrombosis), peripheral artery disease, acute phlebitis, and pulmonary embolism.

In a specific preferred embodiment, the antibody or functional fragment of the invention is used to treat a cardiovascular disease selected from arterial or venous thrombosis, restenosis, acute coronary syndrome or cerebrovascular accidents due to atherosclerosis, preferably arterial or venous thrombosis. The patient being treated with an anti-GPVI antibody or functional fragment thereof in any of the embodiments herein may be also treated with another conventional medicament.

In a further aspect, the present invention relates to the antibody or functional fragment as defined in any one of the embodiments above, or a pharmaceutical composition comprising such antibody or functional fragment, for use in a method of treating or preventing thrombosis or a thrombotic disorder. The "thrombosis or thrombotic disorder" is preferably one as defined above. In another aspect, the present invention relates to the antibody or functional fragment as defined in any one of the embodiments above, or a pharmaceutical composition comprising such antibody or functional fragment, for use as an anti-thrombotic medicament.

A further aspect of this invention is a method of treating a GPVI-related condition, comprising administering to a patient in need thereof an effective amount of an anti-GPVI antibody or functional fragment thereof as defined hereinabove. The GPVI-related condition is preferably one of the conditions described above. Yet a further aspect of this invention is a method of treating or preventing thrombosis or a thrombotic disorder, comprising administering to a patient in need thereof an effective amount of an anti-GPVI antibody or functional fragment thereof as defined hereinabove. The "thrombosis or thrombotic disorder" is preferably one as defined above. A further aspect of this invention is a method of preventing a GPVI-related condition, comprising administering to a patient in need thereof an effective amount of an anti-GPVI antibody or functional fragment as defined hereinabove. The GPVI-related condition is preferably one of the conditions described above.

**Table 1. Summary of the amino acid sequences**

| **SEQ ID NO:** | **Description** | **Sequence** |
|---|---|---|
| 1 | CDR L1 Emf6.1 | KASQNVGTYVA |
| 2 | CDR L2 Emf6.1 | SASYRCS |
| 3 | CDR L3 Emf6.1 | HQYNNYPLT |
| 4 | CDR H1 Emf6.1 | GFSLTSYGVT |
| 5 | CDR H2 Emf6.1 | VIWGDGSTNYHSGLRS |
| 6 | CDR H3 Emf6.1 | AKPIYYDFDDDAMDY |
| 7 | CDR L1 Emf6.1 (IMGT only) | QNVGTY |
| 8 | CDR L2 Emf6.1 (Kabat short) | SASYR |
| 9 | CDR L2 Emf6.1 derived consensus sequence (X6 is A, R, N, D, Q, E, G, H, I, L, K, M, F, S, T, W, Y, or V) | SASYRXS |
| 10 | CDR H1 Emf6.1 (Kabat only) | SYGVT |
| 11 | CDR H1 Emf6.1 (IMGT only) | GFSLTSYG |
| 12 | CDR H2 Emf6.1 (IMGT only) | IWGDGST |
| 13 | CDR H2 Emf6.1 derived consensus sequence (X5 is A, R, N, D, Q, E, G, H, I, L, K, M, F, S, T, W, Y, or V, preferably D or E; X6 is A, R, N, D, Q, E, G, H, I, L, K, M, F, S, T, W, Y, or V, preferably G or A) | VIWGXXSTNYHSGLRS |
| 14 | CDR H3 Emf6.1 (Kabat only) | PIYYDFDDDAMDY |
| 15 | Emf6.1 (murine) VL sequence (VLO) | DIVMTQSQKFMSTSVGDRVSVTCKASQNVGTYVAWF QQKPGQSPKTLIYSASYRCSGVPDRFTGSGSGTDFTLTIS NVQSDDLAEYFCHQYNNYPLTFGAGTKLELR |
| 16 | VL1 (based on BAH04725) | EIVLTQSPSSLSASVGDRVTITCKASQNVGTYVAWFQQK PGKAPKSLIYSASYRCSGVPSKFSGSGSGTDFTLTISSLQP EDFATYYCHQYNNYPLTFGQGTKLEIK |
| 17 | VL2 (based on AIT38570) (V_{L} of EMF601 and EMF602) | DIQMTQSPSSLSASVGDRVTITCKASQNVGTYVAWFQ QRPGKAPKSLIYSASYRCSGVPSKFSGSGSGTDFTLTINSL QPEDFATYYCHQYNNYPLTFGQGTKVEIK |
| 18 | VL3 (based on AMK70118) | DIQLTQSPSTLSASVGDRVTITCKASQNVGTYVAWYQQ KPGKAPKLLIYSASYRCSGVPSRFSGSGSGTEFTLTISSLQ PDDFTTYYCHQYNNYPLTFGQGTKVEIK |
| 19 | VL4 (based on APZ85375) | DIVMTQSPATLSLSPGDRATLSCKASQNVGTYVAWYQ QKPGQAPRLLIYSASYRCSGIPARFSGSGSGTDFTLTISSL QSEDFAVYYCHQYNNYPLTFGGGTKVEIK |
| 20 | Emf6.1 (murine) VH sequence (VHO) | QVQLKESGPGLVQPSQSLSITCTVSGFSLTSYGVTWVRQ PPGKGLEWLGVIWGDGSTNYHSGLRSRLSISRDDSKSQ VFLKLNSLQTDDTATYYCAKPIYYDFDDDAMDYWGQG TSVTVSS |
| 21 | VH1 (based on AAV40414) (V_{H} of EMF601) | QVQLQESGPGLVKPSETLSLTCTASGFSLTSYGVTWIRQ PPGKGLEWIGVIWGDGSTNYHSGLRSRVTISVDTSKSQF SLKLSSVTAADTAVYYCAKPIYYDFDDDAMDYWGQGTL VTVSS |
| 22 | VH2 (based on AAY23326) (V_{H} of EMF602) | EVQLVESGPGLVKPSETLSLTCTVSGFSLTSYGVTWIRQP PGKGLEWIGVIWGDGSTNYHSGLRSRVTISVDTSKNQF SLKLSSVTAADTAVYYCAKPIYYDFDDDAMDYWGQGTL VTVSS |
| 23 | VH3 (based on AEX28400) | QVQLVESGGGVVQPGRSLRLSCVASGFSLTSYGVTWVR QAPGKGLEWVAVIWGDGSTNYHSGLRSRFTISRDNSK NTMFLQMNSLRADDTAVYYCAKPIYYDFDDDAMDYW GQGTLVTVSS |
| 24 | VH4 (based on AAD31716) | QVQLVESGGGVVQPGRSLRLSCAASGFSLTSYGVTWVR QSPGKGLEWVAVIWGDGSTNYHSGLRSRLTISRDNSKN TLYLEMNSLRTEDTATYYCAKPIYYDFDDDAMDYWGQ GTKVTVSS |
| 25 | VH5 (based on IGHV4-4) | QVQLQESGPGLVKPSETLSLTCTVSGFSLTSYGVTWIRQ PPGKGLEWIGVIWGDGSTNYHSGLRSRVTISVDTSKNQ |
| | | FSLKLSSVTAADTAVYYCAKPIYYDFDDDAMDYWGQGT SVTVSS |
| 26 | VL1^{G, CDR2 CS} (VL1 with aa4-12 adapted to aa4-12 of Homo sapiens IGKV1-16 [germlining', changed aa are in bold], and C in CDR2 substituted [X is A, R, N, D, Q, E, G, H, I, L, K, M, F, S, T, W, Y, or V]) | EIVMTQSPSSLSASVGDRVTITCKASQNVGTYVAWFQQ KPGKAPKSLIYSASYRXSGVPSKFSGSGSGTDFTLTISSLQ PEDFATYYCHQYNNYPLTFGQGTKLEIK |
| 27 | VL3^{G, CDR2 CS} (VL3 with aa4-12 in framework I adapted to aa4-12 of Homo sapiens IGKV1-16 [germlining', changed aa are in bold]), and C in CDR2 substituted [X is A, R, N, D, Q, E, G, H, I, L, K, M, F, S, T, W, Y, or V]) | DIQMTQSPSSLSASVGDRVTITCKASQNVGTYVAWYQ QKPGKAPKLLIYSASYRXSGVPSRFSGSGSGTEFTLTISSL QPDDFTTYYCHQYNNYPLTFGQGTKVEIK |
| 28 | VL4^{G, CDR2 CS} (VL4 with aa4-12 in framework I adapted to aa4-12 of Homo sapiens IGKV1-16 [germlining', changed aa are in bold]), and C in CDR2 substituted [X is A, R, N, D, Q, E, G, H, I, L, K, M, F, S, T, W, Y, or V]) | DIVMTQSPSSLSLSPGDRATLSCKASQNVGTYVAWYQQ KPGQAPRLLIYSASYRXSGIPARFSGSGSGTDFTLTISSLQ SEDFAVYYCHQYNNYPLTFGGGTKVEIK |
| 29 | VL1^{G} (VL1 with aa4-12 in framework I adapted to aa4-12 of Homo sapiens IGKV1-16 [germlining', changed aa are in bold]) | EIVMTQSPSSLSASVGDRVTITCKASQNVGTYVAWFQQ KPGKAPKSLIYSASYRCSGVPSKFSGSGSGTDFTLTISSLQ PEDFATYYCHQYNNYPLTFGQGTKLEIK |
| 30 | VL3^{G} (VL3 with aa4-12 in framework I adapted to aa4-12 of Homo sapiens IGKV1-16 [germlining', changed aa are in bold]) | DIQMTQSPSSLSASVGDRVTITCKASQNVGTYVAWYQ QKPGKAPKLLIYSASYRCSGVPSRFSGSGSGTEFTLTISSL QPDDFTTYYCHQYNNYPLTFGQGTKVEIK |
| 31 | VL4^{G} (VL4 with aa4-12 in framework I adapted to aa4-12 of Homo sapiens IGKV1-16 [germlining', changed aa are in bold]) | DIVMTQSPSSLSLSPGDRATLSCKASQNVGTYVAWYQQ KPGQAPRLLIYSASYRCSGIPARFSGSGSGTDFTLTISSLQ SEDFAVYYCHQYNNYPLTFGGGTKVEIK |
| 32 | VL1^{CDR2 CS} (based on BAH04725, with C in CDR2 substituted [X is A, R, N, D, Q, E, G, H, I, L, K, M, F, S, T, W, Y, or V]) | EIVLTQSPSSLSASVGDRVTITCKASQNVGTYVAWFQQK PGKAPKSLIYSASYRXSGVPSKFSGSGSGTDFTLTISSLQP EDFATYYCHQYNNYPLTFGQGTKLEIK |
| 33 | VL2^{CDR2 CS} (based on AIT38570, [V_{L} of EMF601 and EMF602], with C in CDR2 substituted [X is A, R, N, D, Q, E, G, H, I, L, K, M, F, S, T, W, Y, or V]) | DIQMTQSPSSLSASVGDRVTITCKASQNVGTYVAWFQ QRPGKAPKSLIYSASYRXSGVPSKFSGSGSGTDFTLTINSL QPEDFATYYCHQYNNYPLTFGQGTKVEIK |
| 34 | VL3^{CDR2 CS} (based on AMK70118, with C in CDR2 substituted [X is A, R, N, D, Q, E, G, H, I, L, K, M, F, S, T, W, Y, or V]) | DIQLTQSPSTLSASVGDRVTITCKASQNVGTYVAWYQQ KPG KAP KLLIYSASYRXSGVPSRFSGSGSGTEFTLTISSLQ PDDFTTYYCHQYNNYPLTFGQGTKVEIK |
| 35 | VL4^{CDR2 CS} (based on APZ85375, [V_{L} of EMF601 and EMF602], with C in CDR2 substituted [X is A, R, N, D, Q, E, G, H, I, L, K, M, F, S, T, W, Y, or V]) | DIVMTQSPATLSLSPGDRATLSCKASQNVGTYVAWYQ QKPGQAPRLLIYSASYRXSGIPARFSGSGSGTDFTLTISSL QSEDFAVYYCHQYNNYPLTFGGGTKVEIK |
| 36 | human_GPVI-ectodomain (residues_24-267) | PLPKPSLQALPSSLVPLEKPVTLRCQGPPGVDLYRLEKLSS SRYQDQAVLFIPAMKRSLAGRYRCSYQNGSLWSLPSDQ LELVATGVFAKPSLSAQPGPAVSSGGDVTLQCQTRYGF DQFALYKEGDPAPYKNPERWYRASFPIITVTAAHSGTYR CYSFSSRDPYLWSAPSDPLELVVTGTSVTPSRLPTEPPSP VAEFSEATAELTVSFTNEVFTTETSRSITASPKESDSPAG PARQYYTK |
| 37 | LCD (light chain 1: light chain signal peptide+VL0+ IgK LC constant domain [allotype Km3]) | MVSSAQFLGLLLLCFQGTRCDIVMTQSQKFMSTSVGDR VSVTCKASQNVGTYVAWFQQKPGQSPKTLIYSASYRCS GVP DRFTGSGSGTDFTLTISNVQSDDLAEYFCHQYNNY PLTFGAGTKLELRRTVAAPSVFIFPPSDEQLKSGTASVVC LLNNFYP REAKVQWKVDNALQSGNSQESVTEQDSKDS TYSLSSTLTLSKADYEKHKVYACEVTHQGLSSPVTKSFNR GEC |
| 38 | LC1 (light chain 1: heavy chain signal peptide+VL1+ IgK LC constant domain [allotype Km3]) | MVSSAQFLGLLLLCFQGTRCEIVLTQSPSSLSASVGDRVT ITCKASQNVGTYVAWFQQKPGKAPKSLIYSASYRCSGVP SKFSGSGSGTDFTLTISSLQPEDFATYYCHQYNNYPLTFG QGTKLEIKRTVAAPSVFIFPPSDEQLKSGTASVVCLLNNF YPREAKVQWKVDNALQSGNSQESVTEQDSKDSTYSLSS TLTLSKADYEKHKVYACEVTHQGLSSPVTKSFNRGEC |
| 39 | LC2 (light chain 2: light chain signal peptide+VL2+ IgK LC constant domain [allotype Km3]) | MVSSAQFLGLLLLCFQGTRCDIQMTQSPSSLSASVGDR VTITCKASQNVGTYVAWFQQRPGKAPKSLIYSASYRCSG VPSKFSGSGSGTDFTLTINSLQPEDFATYYCHQYNNYPLT FGQGTKVEIKRTVAAPSVFIFPPSDEQLKSGTASVVCLLN NFYPREAKVQWKVDNALQSGNSQESVTEQDSKDSTYS LSSTLTLSKADYEKHKVYACEVTHQGLSSPVTKSFNRGEC |
| 40 | LC3 (light chain 3: light chain signal peptide+VL3+ IgK LC constant domain [allotype Km3]) | MVSSAQFLGLLLLCFQGTRCDIQLTQSPSTLSASVGDRV TITCKASQNVGTYVAWYQQKPGKAPKLLIYSASYRCSGV PSRFSGSGSGTEFTLTISSLQPDDFTTYYCHQYNNYPLTF GQGTKVEIKRTVAAPSVFIFPPSDEQLKSGTASVVCLLNN FYPREAKVQWKVDNALQSGNSQESVTEQDSKDSTYSLS STLTLSKADYEKHKVYACEVTHQGLSSPVTKSFNRGEC |
| 41 | LC4 (light chain 4: light chain signal peptide+VL4+ IgK LC constant domain [allotype Km3]) | MVSSAQFLGLLLLCFQGTRCDIVMTQSPATLSLSPGDRA TLSCKASQNVGTYVAWYQQKPGQAPRLLIYSASYRCSGI PARFSGSGSGTDFTLTISSLQSEDFAVYYCHQYNNYPLTF GGGTKVEIKRTVAAPSVFIFPPSDEQLKSGTASVVCLLNN FYPREAKVQWKVDNALQSGNSQESVTEQDSKDSTYSLS STLTLSKADYEKHKVYACEVTHQGLSSPVTKSFNRGEC |
| 42 | LC2 w/o signal peptide (VL2+ IgK LC constant domain [allotype Km3]) | DIQMTQSPSSLSASVGDRVTITCKASQNVGTYVAWFQ QRPGKAPKSLIYSASYRCSGVPSKFSGSGSGTDFTLTINSL QPEDFATYYCHQYNNYPLTFGQGTKVEIKRTVAAPSVFI FPPSDEQLKSGTASVVCLLNNFYPREAKVQWKVDNALQ SGNSQESVTEQDSKDSTYSLSSTLTLSKADYEKHKVYACE VTHQGLSSPVTKSFNRGEC |
| 43 | HCD (heavy chain 0: heavy chain signal peptide+VH0+IgG1 HC constant domain [allotype G1m17,1]) | MGWTLVFLFLLSVTAGVHSQVQLKESGPGLVQPSQSLSI TCTVSGFSLTSYGVTWVRQPPGKGLEWLGVIWGDGST NYHSGLRSRLSISRDDSKSQVFLKLNSLQTDDTATYYCAK PIYYDFDDDAMDYWGQGTSVTVSSASTKGPSVFPLAPS SKSTSGGTAALGCLVKDYFPEPVTVSWNSGALTSGVHTF PAVLQSSGLYSLSSVVTVPSSSLGTQTYICNVNHKPSNTK VDKKVEPKSCDKTH |
| 44 | HC1 (heavy chain 1: heavy chain signal peptide+VH1+IgG1 HC constant domain [allotype G1m17,1]) | MGWTLVFLFLLSVTAGVHSQVQLQESGPGLVKPSETLS LTCTASGFSLTSYGVTWIRQPPGKGLEWIGVIWGDGST NYHSGLRSRVTISVDTSKSQFSLKLSSVTAADTAVYYCAK PIYYDFDDDAMDYWGQGTLVTVSSASTKGPSVFPLAPS SKSTSGGTAALGCLVKDYFPEPVTVSWNSGALTSGVHTF PAVLQSSGLYSLSSVVTVPSSSLGTQTYICNVNHKPSNTK VDKKVEPKSCDKTH |
| 45 | HC2 (heavy chain 2: heavy chain signal peptide+VH2+IgG1 HC constant domain [allotype G1m17,1]) | MGWTLVFLFLLSVTAGVHSEVQLVESGPGLVKPSETLSL TCTVSGFSLTSYGVTWIRQPPGKGLEWIGVIWGDGSTN YHSGLRSRVTISVDTSKNQFSLKLSSVTAADTAVYYCAKP IYYDFDDDAMDYWGQGTLVTVSSASTKGPSVFPLAPSS KSTSGGTAALGCLVKDYFPEPVTVSWNSGALTSGVHTF PAVLQSSGLYSLSSVVTVPSSSLGTQTYICNVNHKPSNTK VDKKVEPKSCDKTH |
| 46 | HC3 (heavy chain 3: heavy chain signal peptide+VH3+IgG1 HC constant domain [allotype G1m17,1]) | MGWTLVFLFLLSVTAGVHSQVQLVESGGGVVQPGRSL RLSCVASGFSLTSYGVTWVRQAPGKGLEWVAVIWGDG STNYHSGLRSRFTISRDNSKNTMFLQMNSLRADDTAVY YCAKPIYYDFDDDAMDYWGQGTLVTVSSASTKGPSVFP LAPSSKSTSGGTAALGCLVKDYFPEPVTVSWNSGALTSG VHTFPAVLQSSGLYSLSSVVTVPSSSLGTQTYICNVNHKP SNTKVDKKVEPKSCDKTH |
| 47 | HC4 (heavy chain 4: heavy chain signal peptide+VH4+IgG1 HC constant domain [allotype G1m17,1]) | MGWTLVFLFLLSVTAGVHSQVQLVESGGGVVQPGRSL RLSCAASGFSLTSYGVTWVRQSPGKGLEWVAVIWGDG STNYHSGLRSRLTISRDNSKNTLYLEMNSLRTEDTATYYC AKPIYYDFDDDAMDYWGQGTKVTVSSASTKGPSVFPLA PSSKSTSGGTAALGCLVKDYFPEPVTVSWNSGALTSGV HTFPAVLQSSGLYSLSSVVTVPSSSLGTQTYICNVNHKPS NTKVDKKVEPKSCDKTH |
| 48 | HC5 (heavy chain 5: heavy chain signal peptide+VH5+IgG1 HC constant domain [allotype G1m17,1]) | MGWTLVFLFLLSVTAGVHSQVQLQESGPGLVKPSETLS LTCTVSGFSLTSYGVTWIRQPPGKGLEWIGVIWGDGST NYHSGLRSRVTISVDTSKNQFSLKLSSVTAADTAVYYCAK PIYYDFDDDAMDYWGQGTSVTVSSASTKGPSVFPLAPS SKSTSGGTAALGCLVKDYFPEPVTVSWNSGALTSGVHTF PAVLQSSGLYSLSSVVTVPSSSLGTQTYICNVNHKPSNTK VDKKVEPKSCDKTH |
| 49 | LC1^{G} (HC signal peptide + VL1 with aa4-12 adapted to aa4-12 of Homo sapiens IGKV1-16 [germlining', changed aa are in bold] + IgK LC constant domain allotype Km3) | MVSSAQFLGLLLLCFQGTRCEIVMTQSPSSLSASVGDRV TITCKASQNVGTYVAWFQQKPGKAPKSLIYSASYRCSGV PSKFSGSGSGTDFTLTISSLQPEDFATYYCHQYNNYPLTF GQGTKLEIKRTVAAPSVFIFPPSDEQLKSGTASVVCLLNN FYPREAKVQWKVDNALQSGNSQESVTEQDSKDSTYSLS STLTLSKADYEKHKVYACEVTHQGLSSPVTKSFNRGEC |
| 50 | LC3^{G} (LC signal peptide + VL3 with aa4-12 adapted to aa4-12 of Homo sapiens IGKV1-16 [germlining', changed aa are in bold] + IgK LC constant domain allotype Km3) | MVSSAQFLGLLLLCFQGTRCDIQMTQSPSSLSASVGDR VTITCKASQNVGTYVAWYQQKPGKAPKLLIYSASYRCSG VPSRFSGSGSGTEFTLTISSLQPDDFTTYYCHQYNNYPLT FGQGTKVEIKRTVAAPSVFIFPPSDEQLKSGTASVVCLLN NFYPREAKVQWKVDNALQSGNSQESVTEQDSKDSTYS LSSTLTLSKADYEKHKVYACEVTHQGLSSPVTKSFNRGEC |
| 51 | LC4^{G} (light chain signal peptide + VL4 with aa4-12 adapted to aa4-12 of Homo sapiens IGKV1-16 [germlining', changed aa are in bold] + IgK LC constant domain allotype Km3) | MVSSAQFLGLLLLCFQGTRCDIVMTQSPSSLSLSPGDRA TLSCKASQNVGTYVAWYQQKPGQAPRLLIYSASYRCSGI PARFSGSGSGTDFTLTISSLQSEDFAVYYCHQYNNYPLTF GGGTKVEIKRTVAAPSVFIFPPSDEQLKSGTASVVCLLNN FYPREAKVQWKVDNALQSGNSQESVTEQDSKDSTYSLS STLTLSKADYEKHKVYACEVTHQGLSSPVTKSFNRGEC |
| 52 | LC1^{G, CDR2 CS} (HC signal peptide + VL1 with aa4-12 adapted to aa4-12 of Homo sapiens IGKV1-16 [germlining', changed aa are in bold], and C in CDR2 substituted [X is A, R, N, D, Q, E, G, H, I, L, K, M, F, S, T, W, Y, or V] + IgK LC constant domain allotype Km3) | MVSSAQFLGLLLLCFQGTRCEIVMTQSPSSLSASVGDRV TITCKASQNVGTYVAWFQQKPGKAPKSLIYSASYRXSGV PSKFSGSGSGTDFTLTISSLQPEDFATYYCHQYNNYPLTF GQGTKLEIKRTVAAPSVFIFPPSDEQLKSGTASVVCLLNN FYPREAKVQWKVDNALQSGNSQESVTEQDSKDSTYSLS STLTLSKADYEKHKVYACEVTHQGLSSPVTKSFNRGEC |
| 53 | LC3^{G, CDR2 CS} (LC signal peptide+VL3 with aa4-12 adapted to aa4-12 of Homo sapiens IGKV1-16 [germlining', changed aa are in bold], and C in CDR2 substituted [X is A, R, N, D, Q, E, G, H, I, L, K, M, F, S, T, W, Y, or V] + IgK LC constant domain allotype Km3) | MVSSAQFLGLLLLCFQGTRCDIQMTQSPSSLSASVGDR VTITCKASQNVGTYVAWYQQKPGKAPKLLIYSASYRXSG VPSRFSGSGSGTEFTLTISSLQPDDFTTYYCHQYNNYPLT FGQGTKVEIKRTVAAPSVFIFPPSDEQLKSGTASVVCLLN NFYPREAKVQWKVDNALQSGNSQESVTEQDSKDSTYS LSSTLTLSKADYEKHKVYACEVTHQGLSSPVTKSFNRGEC |
| 54 | LC4^{G, CDR2 CS} (light chain signal peptide+VL4 with aa4-12 adapted to aa4-12 of Homo sapiens IGKV1-16 [germlining', changed aa are in bold], and C in CDR2 substituted [X is A, R, N, D, Q, E, G, H, I, L, K, M, F, S, T, W, Y, or V] + IgK LC constant domain allotype Km3) | MVSSAQFLGLLLLCFQGTRCDIVMTQSPSSLSLSPGDRA TLSCKASQNVGTYVAWYQQKPGQAPRLLIYSASYRXSGI PARFSGSGSGTDFTLTISSLQSEDFAVYYCHQYNNYPLTF GGGTKVEIKRTVAAPSVFIFPPSDEQLKSGTASVVCLLNN FYPREAKVQWKVDNALQSGNSQESVTEQDSKDSTYSLS STLTLSKADYEKHKVYACEVTHQGLSSPVTKSFNRGEC |
| 55 | LC1^{CDR2 CS} (light chain signal peptide + VL1 with C in CDR2 substituted [X is A, R, N, D, Q, E, G, H, I, L, K, M, F, S, T, W, Y, or V] + IgK LC constant domain allotype Km3) | MVSSAQFLGLLLLCFQGTRCEIVLTQSPSSLSASVGDRVT ITCKASQNVGTYVAWFQQKPGKAPKSLIYSASYRXSGVP SKFSGSGSGTDFTLTISSLQPEDFATYYCHQYNNYPLTFG QGTKLEIKRTVAAPSVFIFPPSDEQLKSGTASVVCLLNNF YPREAKVQWKVDNALQSGNSQESVTEQDSKDSTYSLSS TLTLSKADYEKHKVYACEVTHQGLSSPVTKSFNRGEC |
| 56 | LC2^{CDR2 CS} (light chain signal peptide + VL2 with C in CDR2 substituted [X is A, R, N, D, Q, E, G, H, I, L, K, M, F, S, T, W, Y, or V] + IgK LC constant domain allotype Km3) | MVSSAQFLGLLLLCFQGTRCDIQMTQSPSSLSASVGDR VTITCKASQNVGTYVAWFQQRPGKAPKSLIYSASYRXSG VPSKFSGSGSGTDFTLTINSLQPEDFATYYCHQYNNYPLT FGQGTKVEIKRTVAAPSVFIFPPSDEQLKSGTASVVCLLN NFYPREAKVQWKVDNALQSGNSQESVTEQDSKDSTYS LSSTLTLSKADYEKHKVYACEVTHQGLSSPVTKSFNRGEC |
| 57 | LC3^{CDR2 CS} (light chain signal peptide + VL3 with C in CDR2 substituted [X is A, R, N, D, Q, E, G, H, I, L, K, M, F, S, T, W, Y, or V] + IgK LC constant domain allotype Km3) | MVSSAQFLGLLLLCFQGTRCDIQLTQSPSTLSASVGDRV TITCKASQNVGTYVAWYQQKPGKAPKLLIYSASYRXSGV PSRFSGSGSGTEFTLTISSLQPDDFTTYYCHQYNNYPLTF GQGTKVEIKRTVAAPSVFIFPPSDEQLKSGTASVVCLLNN FYPREAKVQWKVDNALQSGNSQESVTEQDSKDSTYSLS STLTLSKADYEKHKVYACEVTHQGLSSPVTKSFNRGEC |
| 58 | LC4^{CDR2 CS} (light chain signal peptide + VL4 with C in CDR2 substituted [X is A, R, N, D, Q, E, G, H, I, L, K, M, F, S, T, W, Y, or V] + IgK LC constant domain allotype Km3) | MVSSAQFLGLLLLCFQGTRCDIVMTQSPATLSLSPGDRA TLSCKASQNVGTYVAWYQQKPGQAPRLLIYSASYRXSGI PARFSGSGSGTDFTLTISSLQSEDFAVYYCHQYNNYPLTF GGGTKVEIKRTVAAPSVFIFPPSDEQLKSGTASVVCLLNN FYPREAKVQWKVDNALQSGNSQESVTEQDSKDSTYSLS STLTLSKADYEKHKVYACEVTHQGLSSPVTKSFNRGEC |
| 59 | HC1 w/o signal peptide (VH1+IgG1 HC constant domain [allotype G1m17,1]) | QVQLQESGPGLVKPSETLSLTCTASGFSLTSYGVTWIRQ PPGKGLEWIGVIWGDGSTNYHSGLRSRVTISVDTSKSQF SLKLSSVTAADTAVYYCAKPIYYDFDDDAMDYWGQGTL VTVSSASTKGPSVFPLAPSSKSTSGGTAALGCLVKDYFPE |
| | | PVTVSWNSGALTSGVHTFPAVLQSSGLYSLSSVVTVPSS SLGTQTYICNVNHKPSNTKVDKKVEPKSCDKTH |
| 60 | human IgK light chain constant domain (allotype Km3) | |
| 61 | human IgG1 heavy chain constant domain (allotype G1m17,1) | ASTKGPSVFPLAPSSKSTSGGTAALGCLVKDYFPEPVTVS WNSGALTSGVHTFPAVLQSSGLYSLSSVVTVPSSSLGTQ TYICNVNHKPSNTKVDKKVEPKSCDKTH |
| 62 | N-terminal light chain signal peptide | MVSSAQFLGLLLLCFQGTRC |
| 63 | N-terminal heavy chain signal peptide | MGWTLVFLFLLSVTAGVHS |

| | | |
|---|---|---|
| *Amino acids designated "X" have the meaning as defined in the accompanying sequence listing. Underlined and grey residues in SEQ ID NO:15-35 correspond to CDR residues based on Kabat and IMGT, respectively. Bold residues in SEQ ID NO:36 are binding residues on human GPVI. Underlined residues in SEQ ID NO:37-58 are V_{L} or V_{H} regions. Bold residues in SEQ ID NO:26-35 and 49-58 indicate amino acid substitutions. | | |

### Examples

### 1 Materials and Methods

### 1.1 Antibodies and Reagents

Horm collagen was purchased from Takeda (Linz, Austria); The collagen-related peptide (CRP) was purchased from Cambridge Research Biochemicals (Cambridge, UK); ADP, apyrase, prostacyclin(PGI2), fibrinogen and haematoxylin were from Sigma Aldrich (Steinheim, Germany); thrombin was purchased from Roche Diagnostic (Mannheim, Germany); convulxin was purchased from Enzo Life Sciences (New York, NY, USA rabbit anti-GAPDH and rat anti-mouse IgG-HRP antibodies were purchased from Sigma-Aldrich (Steinheim, Germany); U46619 was purchased from AlexisBiochemicals (Enzo Life sciences, New York, NY, USA); anti-rabbit IgG-HRP was purchased from Jackson Immuno (Suffolk, UK); goat anti-rat IgG-HRP was purchased from Dianova (Hamburg, Germany). The micro-cuvettes for aggregometry were purchased from LABITec (Ahrensburg, Germany). For the collection of human blood, S-monovettes 3.2% citrate and Safety-Fly-Needle 21G were purchased from Sarstedt (Nümbrecht, Germany). Heparin was purchased from Ratiopharm (Ulm, Germany); the 5 mL Polystyrene Round-Bottom Tubes for flow cytometry were purchased from Corning Inc. (New York, NY, USA). Emf6 was generated in parallel to the other Emf antibodies.²⁵ After repeated subclonings, a highly productive monoclonal subclone (Emf6.1) was isolated and further characterized (Emfret Analytics, unpublished). The Emf1³², Emf2³², Emf3, Emf6.1, JON/A³⁴ and WUG 1.9³⁴ were produced, purified and derivatized in-house.

The humanized anti-human GPVI Fab Glenzocimab (Code: PX-TA1552-1000, also referred to as ACT017) was purchased from ProteoGenix (Schiltigheim, France).

### 1.2 Blood Donors and Blood Collection

Blood was collected from healthy volunteers free from anticoagulant or anti-platelet therapy for at least 4 weeks. Blood samples were obtained after written informed consent in accordance with the Declaration of Helsinki and approval by the Institutional Review Boards of the University of Würzburg. Blood was drawn by venipuncture using butterfly needles and collected into 9 mL tubes containing 3.2% trisodium citrate. For all studies, the blood was kept at room temperature and used within 4 h. All methods were performed in accordance with the relevant guidelines and regulations. Blood was drawn by venipuncture using butterfly needles and collected into 9 mL tubes containing 3.2% trisodium citrate. For all studies, the blood was kept at room temperature and used within 4 h.

### 1.3 Animals

Animal experiments were approved by the district government of Lower Franconia (Regierung von Unterfranken) and performed in accordance with current Animal Research: reporting *of in vivo* experiments guidelines (https://arriveguidelines.org/). Mice used were matched for age, sex, and genetic background. All experiments with animals described in the study have been carried out using the previously described mouse line humanized for GPVI (*hGP6*^{*tg*/*tg*})*.*³²

### 1.4 Washed human and murine platelets

Human washed platelets were obtained as follows: Citrated blood was collected in a 10 ml S-monovette and implemented with 2 ml of ACD PH 4.5. The sample was centrifuged for 20 min at 300 g at room temperature. Platelet-Rich-Plasma (PRP) was collected in new 15 ml falcons and supplemented with 1/10 ACD, 2 µL of apyrase/ml (0.02 U ml-1; A6410, Sigma-Aldrich) and 5 µl PGI₂/µL (0.1 µg ml-1; P6188, Sigma-Aldrich). Platelets were pelleted by centrifugation for 10 min at 500 g, washed twice with Tyrode's buffer (N-2-hydroxyethyl-piperazine-N02-ethanesulphonic acid; 134 mM NaCl, 0.34 mM NaH₂PO₄, 2.9 mM KCI, 12 mM NaHCOs, 5 mM HEPES, 5 mM glucose, 0.35% BSA, pH 7.4) containing 2 µL apyrase/ml and 5 µL PGI₂/ml and finally resuspended at a concentration of 500.000/µL in Tyrode's buffer and kept for 30 min at 37°C before use. For the experiments, platelets were recalcified using calcified Tyrode's buffer.

Murine washed platelets were obtained as follows: Whole blood was collected in heparin via retro-orbital bleeding following isoflurane anesthesia. Heparinized blood was centrifuged for 6 min at 300 g. The obtained PRP was supplemented with 2 µL apyrase and 5 µL PGI₂ as for human platelets. Next, platelets were pelleted by centrifugation for 5 min at 800 g, washed twice with Tyrode's buffer (implemented with 2 µL apyrase and 5 µL PGI₂). Finally, the platelets were resuspended as for human platelets and kept for 30 minutes at 37°C before use.

### 1.5 Flow adhesion assay (whole blood perfusion assay)

Assessment of platelet adhesion and aggregate formation (thrombi formation) on Horm collagen under flow in heparinized human blood treated with 1, 2, 5 or 10 µg/mL anti-GPVI antibody or Fab, or control antibody or Fab. 200 µg/mL Horm collagen was coated on coverslips for 1 hour at 37°C and then blocked using 1% BSA In PBS1x. Heparinized human or murine blood was diluted 1:2 in Tyrode's buffer and supplemented with 2 mM Ca²⁺. Murine platelets were labelled with an anti GPIX-Dylight 488 conjugated antibody, while human platelets were labelled using the anti-GPlbβ antibody p0p1³⁵ conjugated to Dylight 488. Blood was perfused over the coverslips at a shear rate of 1000 s⁻¹ for 4 min and subsequently washed for 4 more minutes with Tyrode's buffer supplemented with calcium. After the washing step, 8 representative fields of view were imaged using a Leica DMI6000B microscope with a 63x objective (Leica Biosystems Technologies, Frankfurt, Germany). Finally, images were analyzed for overall platelet surface coverage and relative thrombus volume using (fluorescent integrated density) using Fiji.17.

### 1.6 Coagulation flow chamber

Assessment of platelet adhesion, thrombus formation, phosphatidylserine-exposure and fibrin deposition, on Horm collagen plus tissue factor, under flow in recalcified human blood treated with e.g. 10 µg/mL anti-GPVI antibody or Fab, or control antibody or Fab. In order to analyze *in vitro* the generation of thrombi under coagulating condition under flow, coverslips were freshly coated with 50 µg/mL Horm collagen for 1 h at 37°C, followed by a second incubation with 500 pM tissue factor for 1 h in a humid chamber. The slides were then blocked for 30 min at RT with PBS1x 1%BSA. For in situ blood recalcification, a Y-shaped silicone tube was used to pump citrated blood and recalcification buffer (32 mM MgCl₂ and 63 mM CaCl2 in Hepes bufer pH 7.45) in the chamber containing the blocked coverslips. Blood was perfused over the coverslips at a shear rate of 1000 s⁻¹. Images were taken every 30 seconds with a LEICA DMI6000B microscope with a 63x objective (Leica Biosystems Technologies, Frankfurt, Germany). Human platelets were labelled using the anti-GPlbβ antibody p0p1-A647³⁵, PSexposure was observed using annexin 5-A546 produced in-house, while the fibrin deposition was observed by adding fibrinogen-A488.

### 1.7 Light-transmission aggregometry (turbidimetric aggregometry)

Aggregation responses of washed human platelets treated with 1, 2, 5 or 10 µg/mL anti-GPVI antibody or Fab, or control antibody or Fab, in light-transmission aggregometry at 37°C. Washed human or murine platelets were diluted (recalcified) in Tyrode's buffer supplemented with 2 mM Ca²⁺ and 100 µg/ml human fibrinogen. When thrombin was used as agonist, Tyrode's buffer was not supplemented with fibrinogen. Light-transmission aggregometry was performed to follow platelet aggregation over time using a 4-channel APACT aggregometer (LABITec Ahrensburg, Germany) under stirring conditions for 10 min and recording transmission, after pre-incubation with the antibodies and the addition of the specified agonists (CRP at 0.5 µg/mL, collagen at 2, 5, 10 or 20 µg/mL).

### 1.8 Spreading assay

Washed platelets were pre-incubated with the Fab fragments (with e.g. 10 µg/mL anti-GPVI Fab, or control Fab) and subsequently further diluted to 100.000/µL and then pipetted onto a 100 µg/mL fibrinogen-coated surface. The platelets were allowed to spread for 45 min at 37°C. Next, the coverslips were fixated using 4% PF4 for 10 min. The spread platelets were visualized using a ZEISS Axiovert (Zeiss group, Oberkochen, Germany) microscope with a 100x objective. Images were analyzed using Fiji.17 cell counter tool and phase abundance was determined by discriminating platelets based on 4 phases of spreading. Phase 1: adhesion, phase 2: filopodia formation; phase 3: lamellipodia formation; phase 4: fully spread platelet

### 1.9 Measurement of platelet count and size

For determining platelet count and size, *hGP6*^{*tg*/*tg*} mice received a dose of 4 mg/kg of anti-GPVI Fab or control Fab intravenously and their peripheral platelets were monitored for 5 days automated cell analyses. To assess platelet count and size, mice were bled into EDTA-coated tubes at specific timepoints after administration of anti-GPVI Fab or control Fab; platelet parameters were measured using an automated cell counter (ScilVet, scil animal care company GmbH, Viernheim, Germany). Platelet count and size values from anti-GPVI Fab treated mice were normalized using values from samples of control Fab not specific to GPVI treated mice of same timepoint.

### 1.10 Flow cytometric analysis of GPVI surface levels on platelets, binding of anti-GPVI antibody or Fab of the invention on GPVI on platelets and platelet activation

For determining *in vivo* GPVI surface levels on circulating platelets, anti-GPVI Fab binding and platelet activation, *hGP6*^{*tg*/*tg*} mice received a dose of 4 mg/kg anti-GPVI Fab (Emf6.1^{Fab}), or control Fab, intravenously and their peripheral platelets were monitored for 5 or 6 days by flow cytometry and automated cell analyses. For the detection with Emf-2^{FITC} (used to test for GPVI exposure i.e. GPVI surface levels on circulating platelets) and Emf-3^{FITC} (used to assess epitope saturation of anti-GPVI antibody or Fab of the invention on circulating platelets), murine blood diluted 1:20 in Tyrode's buffer without Ca²⁺ was pre-incubated 10 min with the antibodies. In another set of experiments, *hGP6*^{*tg*/*tg*} mice received 4 mg/kg EMA601 (humanized Fab of the invention) or Glenzocimab (ACT017) intravenously and their peripheral platelets were monitored for 24 hours by flow cytometry and automated cell analyses. For the detection with Emf-2^{FITC} (used to test for GPVI surface expression) and Emf-3^{FITC} or JAQ1^{FITC} (used to assess epitope saturation of EMA601 and Glenzocimab, respectively, on circulating platelets), murine blood diluted 1:20 in Tyrode's buffer without Ca²⁺ was pre-incubated 10 min with the antibodies. To detect surface-bound EMA601 or Glenzocimab, an anti-human IgG (Fab specific)-FITC antibody (Sigma Aldrich, F5512) was used.

For the platelet activation analysis, the murine blood was diluted in Tyrode's buffer with 2 mM Ca²⁺. JON/A-PE (Emfret Analytics, Eibelstadt, Germany) was used to detect the activated integrin αllbβ3 whilst P-selectin exposure was used as marker for platelet degranulation and detected with a FITC-conjugated specific anti-mPselectin antibody, WUG 1.9.³⁴ The murine blood was drawn (as described in section 1.2) at specific timepoints after administration of anti-GPVI Fab or control Fab, was diluted and then incubated with either CRP (0.5 µg/mL), thrombin (0.1 U/mL) or vehicle solution, together with JON/A-PE and anti-Psel^{FITC} for 12 min (6 min at 37°C and 6 min at RT). Finally, the blood was further diluted in 500 µL PBS to allow the measurement of the MFI using a FACSCelesta (BD Biosciences, Franklin Lakes, New Jersey, USA). To determine GPVI surface levels on circulating platelets / binding of anti-GPVI antibody or Fab of the invention on GPVI on platelets / platelet activation (activated integrin αllbβ3, P-selectin exposure), respective MFI values of samples from anti-GPVI Fab treated mice were normalized using samples of control Fab not specific to GPVI treated mice of same timepoint.

### 1.11 In vivo model of thrombosis

The abdominal aorta of anaesthetized mice treated mice (e.g. 1 h after injection of 4 mg/kg anti-GPVI Fab, or control Fab) was exposed via accessing the abdominal cavity. The aorta was separated from the vena cava via removal of fat layers and an ultrasonic flow probe (0.5PSB699; Transonic Systems, USA) was placed around the abdominal aorta. Thrombus formation was induced by one compression of the aorta with forceps (Ultra Fine Hemostats clams, Fine Science Tools, Vancouver, Canada) for 5 s (buckle setting 1) upstream of the flow probe. Blood flow was monitored for 30 min or until vessel occlusion occurred (interruption of the blood flow for > 5 min). Significance of occluded and non-occluded vessels were statistically assessed using Fisher's exact test.

### 1.12 Tail bleeding assay

Following anesthesia of the animals, a scalpel was used to remove 2 mm tip of the tail. Tail bleeding was monitored by gently absorbing blood on filter paper at 20 s intervals without directly contacting the wound site. When no blood was observed on the paper, bleeding was determined to have ceased. The experiment was manually stopped after 20 min by cauterization. Differences between occluded and non-occluded wounds was statistically assessed using the Fisher's exact test the mean bleeding time by Mann-U-Whitney test.

### 1.13 Automated uSPOT Synthesis

The GPVI ectodomain domain (residues 24-267 of UniProtKB: Q9HCN6-1; corresponding to SEQ ID NO:36) was displayed in microarray format as 15mer overlapping peptides shifted by 3 residues. Peptide arrays were synthesized using MultiPep RSi robot (CEM GmbH, Kamp-Lintford, Germany) on cellulose discs containing 9-fluorenylmethyloxycarbonyl-β-alanine (Fmoc-β-Ala) linkers (average loading: 130 nmol/disc-4 mm diameter).³⁶ Synthesis was performed by deprotecting the Fmoc-group using 20% piperidine in dimethylformamide (DMF). Peptide chains was elongated using a coupling solution consisting of amino acids (0.5 M) with oxyma (1 M) and diisopropylmethanediimine (1 M) in DMF (1:1:1). Coupling steps were carried out for 3 times (30 min each), followed by capping (4% acetic anhydride in DMF). Cleavable peptides were coupled with acid labile linker (Fmoc-rink-amide) to ensure the cleave off the cellulose support.

Cellulose discs were transferred into 96 deep-well plates for the peptides work-up. First, side chains groups were deprotected using 90% trifluoracetic acid (TFA), 2% dichloromethane (DCM), 5% H₂O and 3% triisopropylsilane (150 µL/well) for 1 h at room temperature (RT). Afterwards, the deprotection solution was discarded and the discs were solubilized overnight (O/N) at rt, using a solvation mixture containing 88.5% TFA, 4% trifluoromethanesulfonic acid (TFMSA), 5% H₂O and 2.5% TIPS (250 µL/well). The resulting peptide-cellulose conjugates (PCCs) were precipitated in ice-cold ether (700 µL/well) and spinned down at 2000× g for 10 min at 4°C, followed by two additional washes of the formed pellet with ice-cold ether. The resulting pellets were dissolved in DMSO (250 µL/well). PCCs solutions were mixed in 2:1 ratio with saline-sodium citrate buffer (150 mM NaCl, 15 mM trisodium citrate, pH 7.0) and transferred to a 384-well plate. For transfer of the PCC solutions to white-coated CelluSpot blank slides (76 × 26 mm, Intavis AG Peptide Services GmbH and CO. KG), a SlideSpotter (CEM GmbH) was used. After completion of the printing procedure, slides were left to dry for at least 3 h. Cleavable peptides were worked-up from the supernatant of the cleavage mixture and precipitated in the same manner. Peptide pellets were resuspended in water.

### 1.14 Microarray binding assay

The microarray slides were blocked for 60 min with 5% (w/v) skimmed milk powder (Carl Roth) in phosphate-buffered saline (PBS; 137 mM NaCl, 2.7 mM KCI, 10 mM Na₂HPO₄, 1.8 mM KH₂PO₄, pH 7.4). After blocking, the slides were incubated for 30 min with Emf6.1 (5 µg/mL) in the blocking buffer, then washed 3× with PBS for 1 min. Antibody binding was detected using Goat anti-mouse IgG-HRP (Thermo Fisher Cat. No. 31430, 1:5000). The chemiluminescent readout was detected with an Azure imaging system c400 (lowest sensitivity) using SuperSignal West Femto maximum sensitive substrate (Thermo Scientific GmbH, Schwerte, Germany). Epitope neutralization was carried out by pre-incubating Emf6.1 with cleavable peptides for 30 min and then applied on the blocked slide.

Microarray binding intensities were quantified with FIJI using the "microarray profile" plugin (OptiNav Inc, Bellevue, WA, USA). After background subtraction of the mean greyscale value of the microarray surface surrounding the spots, the raw grayscale intensities for each position were obtained for the left and right sides of the internal duplicate on each microarray slide. The standard deviation (STDEV) between both sides was calculated.

### 1.15 Antibody humanization

Emf6.1 murine variable domains were sequenced, complementary-determining regions (CDRs) were identified and grafted into suitable human acceptor frameworks as described in section 2.2.1 below. DNA coding for the amino acid sequences of the variants were cloned into the mammalian transient expression plasmid pETE V2. The variants were expressed using a CHO based transient expression system and purified by affinity chromatography using AKTA chromatography equipment as described in section 2.2.2 below.

### 1.16 Kinetic analysis (K_{D})

Dissociation equilibrium constant (K_{D}) was determined using bio-layer interferometry (BLI). A series of tests were initially performed to optimize assay parameters, resulting in the following parameters:

**Table 2: Summary of optimized kinetic screening parameters for the characterization of IgG:antigen interactions.**

| | |
|---|---|
| **Antigen loading concentration** | 1.2 µg/mL |
| **Time for association /dissociation** (s) | 900 / 1200 |
| **Kinetic screening protocol required** | modified |
| **Antibody screening range** (seven concentrations, 3-fold serial dilution) | 10 - 0.014 nM |
| **Interaction** / **fitting model** | 1:1 |

K_{D} measurement was carried by immobilizing human GPVI (1.2 µg) on biosensor tips using suitable capture surfaces and monitoring binding of Emf6.1 variants (that is, the humanized Fab fragments and the chimeric Fab fragment, HC0 LCD, comprising the Emf6.1 murine variable domains and the human Ig constant domains) by BLI using an Octet instrument. The resulting sensograms were analyzed using the supplied software (Fortebio). Time (in s) for association / dissociation: 900 / 1200; antibody screening range (seven concentrations using a 3-fold serial dilution): 10 - 0.014 nM; interaction/fitting model 1:1. All samples were diluted in freshly prepared running buffer.

K_{D} measurements were performed using human GPVI protein antigen captured on biosensors. The antigen captured biosensors (that is the tips) were submerged in wells containing different concentrations of Emf6.1 variants (association stage) followed by a dissociation step in running buffer. To allow for reference correction, antigen captured sensors were dipped into wells containing only buffer (reference well). This referencing provided a means of compensating for the natural dissociation of the capture antigen. Steps were performed at 25°C and a constant orbital flow rate of 1000 rpm (generated by moving samples in wells of a multiwellplate in an orbital motion relative to the biosensor). New sensors were used for each sample. The dilution series of the Emf6.1 variants were used in the association step, in order to fit results globally and get the best values for ka, kd, and K_{D}. Dissociation equilibrium constants (K_{D}) were calculated using the ForteBio Data Analysis software, the response data for the binding were fitted to a 1:1 interaction/binding model. All consumables used were those recommended by ForteBio.

### 2. Results

### 2.1 Example 1: Generation and testing of mouse IgG antibodies against human GPVI

### 2.1.1 Emf6.1 binds to human GPVI and blocks its function

A series of monoclonal mouse IgG antibodies against human GPVI (Emf antibodies) was generated by standard hybridoma technology and 16 clones were isolated based on their specific binding to the human GPVI ectodomain (Emf1 - Emf16). Flow cytometric analysis showed that only 9 of them also bound to human platelets, i.e. membrane expressed GPVI, among them Emf1, Emf2, Emf3 and Emf6.1. These clones were further characterized for their ability to inhibit GPVI function. Indeed, 3 of these monoclonal antibodies (mAbs) inhibited CRPand collagen-induced responses of human platelets (Emf1²⁵, Emf2³² and Emf6.1) with Emf6.1 (mouse IgG2a κ) consistently showing the most potent inhibitory effect. Emf6.1 IgG (10 µg/mL) abolished CRP- and collagen-induced aggregation of human platelets whereas it had no effect on responses to other agonists, including thrombin and the stable thromboxane A2 analog U46619 (Fig. 1A). Furthermore, Emf6.1 potently blocked aggregate formation of human platelets in heparinized blood on collagen under flow (1000s⁻¹) when used at 10, 5, 2, and 1 µg/mL (Fig. 1B, C). To circumvent possible Fc-mediated effects of Emf6.1 IgG through the platelet expressed Fc gamma receptor IIA (FcyRIIA, CD32), but also to exclude Fc-dependent thrombocytopenia and/or GPVI depletion¹⁹, Fab fragments of Emf6.1 were generated and used for all further studies.

### 2.1.2 Emf6. 1^{Fab} inhibits GPVI-dependent activation of human platelets

To determine *in vitro* efficacy of Emf6.1^{Fab} in reducing GPVI-dependent platelet adhesion and aggregate formation, anti-coagulated human blood was tested under flow on a collagen-coated surface. At 10 µg/mL Emf6.1^{Fab} almost completely abrogated human platelet adhesion and aggregate formation at a shear rate of 1000 s⁻¹ (Figure 9A). Notably, also at lower concentrations of Emf6.1^{Fab} (5, 2 and 1 µg/mL) aggregate formation was significantly inhibited, with a 75% reduction in thrombus volume compared to control Fab at 1 µg/mL (Figure 9A). Next, citrated human blood was preincubated with 10 µg/mL Emf6.1^{Fab} or control Fab and then tested for thrombus formation in the flow chamber under conditions of active coagulation. Markedly, under these conditions Emf6.1^{Fab} potently inhibited platelet deposition and thrombus formation on a collagen/TF-coated surface (Figure 1D-F) and completely abolished phosphatidylserine (PS)-exposure as well as fibrin deposition (Figure 1 D,G-H).

Next, washed human platelets were treated with 10 µg/mL Emf6.1^{Fab} and then tested in standard aggregometry with different agonists. Emf6.1^{Fab} abolished CRP- and collagen-induced aggregation, while thrombin-induced responses remained unaltered, confirming the GPVI-specific inhibitory effect of the Fab (Figure 1I). Similar to the results in flow adhesion, significant inhibition was observed in aggregometry at 5, 2 and 1 µg/mL Emf6.1^{Fab} (Figure 9B). Human GPVI has been shown to facilitate platelet activation and spreading on fibrinogen.^{40, 41} To test whether Emf6.1^{Fab} interfered with this GPVI function, human platelets were allowed to adhere to immobilized fibrinogen in the presence or absence of the Fab. Indeed, Emf6.1^{Fab} (10 µg/mL) potently inhibited full spreading of the platelets as revealed by a drastic reduction of phase 4 (fully spread) platelets and a marked increase in phase 2 (filopodia-forming platelets) (Figure 1J-K).

Taken together, this data show that Emf6.1^{Fab} potently inhibits GPVI-dependent activation, aggregation and spreading of human platelets *in vitro.*

### 2.1.3 Emf6. 1^{Fab} inhibits GPVI-mediated activation of hGP6^{tg/}^{tg} platelets

In order to study the effect of Emf6.1^{Fab} *in vivo,* a recently generated mouse line humanized for GPVI (*hGP6*^{*tg*/*tg*})³² was used. First, the effect of Emf6.1^{Fab} on agonistinduced activation of *hGP6*^{*tg*/*tg*} platelets was tested in whole blood flow cytometry. Emf6.1^{Fab} (10 µg/mL) abolished CRP-induced activation but had no effect on the responses to other agonists such as thrombin, ADP and U46619 (Figure 10A-B). Similar to the results with human platelets, Emf6.1^{Fab} completely inhibited aggregate formation of *hGP6*^{*tg*/*tg*} platelets on collagen in the whole blood perfusion assay (Figure 2A-C) and the same effect was seen at a concentration of 2 µg/mL Emf6.1 ^{Fab} (Figure 10D). Furthermore, Emf6.1^{Fab} (10 or 2 µg/mL) almost completely inhibited GPVI-mediated aggregation of *hGP6*^{*tg*/*tg*} platelets (Figure 2D and Figure 10C). It has previously been shown that mouse platelets expressing human GPVI, but not WT platelets, can fully spread on fibrinogen.^{32, 42} Therefore it was also tested whether Emf6.1^{Fab} interfered with this response. Indeed, *hGP6*^{*tg*/*tg*} platelets successfully spread on the fibrinogen-coated surface and, similar to the observation with human platelets, this process was abolished in the presence of Emf6.1^{Fab} (Figure 2E-F).

Taken together, these results show comparable *in vitro* effects of Emf6.1^{Fab} on human and *hGP6*^{*tg*/*tg*} mouse platelets, indicating that the mouse line is a suitable model system to study the *in vivo* effects of Emf6.1^{Fab}.

### 2.1.4 Emf6. 1^{Fab} efficiently blocks GPVI function in hGP6^{tg/}^{tg} mice ex vivo

To study the *in vivo* effects of Emf6.1^{Fab}, *hGP6*^{*tg*/*tg*} mice received a dose of 4 mg/kg intravenously and their peripheral platelets were monitored for 5 days by flow cytometry and automated cell analyses. Platelet count and size were unaltered compared to control at all tested time intervals (Figure 3A-B) and no alteration of GPVI surface levels on circulating platelets were detectable (Figure 3C). Next, GPVI occupancy by Emf6.1^{Fab} on circulating platelets was assessed over time using a FITC-labelled anti-GPVI mAb competing for the Emf6.1 binding site (Emf3). Significant GPVI occupancy was detected for up to 96 hours after injection of a single dose of 4 mg/kg b.w. Emf6.1 ^{Fab} (Figure 3D). In detail, 1 hour after injection, ~90% of the binding epitopes on GPVI were occupied by Emf6.1^{Fab} and the percentage slowly declined to ~76% at 24 h, -50% at 48 h, ~36% at 72 h, ~19% at 96 h and ~13% at 120 h. This sustained GPVI blockade was confirmed by flow cytometric analysis of CRP-induced platelet activation in diluted blood ex *vivo.* A significantly impaired response to CRP-, but not thrombin, in Emf6.1^{Fab} treated mice compared to the control group for up to 72 h after injection of 4 mg/kg b.w. Emf6.1 ^{Fab} was detected (Figure 3E-H).

In accordance with these results, a marked inhibition of CRP-induced platelet aggregation ex *vivo* for up to 72 h after injection of 4 mg/kg b.w. Emf6.1^{Fab}, but not control Fab, was found (Figure 4A). Also, collagen-induced aggregation was potently inhibited by the treatment with Emf6.1 ^{Fab}, showing marked reduction after 1 and 24 hours after the treatment, though substantial GPVI-inhibition was still observable at 48 h which was further decreased after 72 h (Figure 4B). As expected, thrombin-induced aggregation was unaltered at all tested times (Figure 4C). Next, the effect of Emf6.1^{Fab} treatment on GPVI dependent aggregate formation on a collagen-coated surface was assessed in the whole blood flow adhesion assay *ex vivo.* Also here, a significant reduction of surface coverage and thrombus volume was found in Emf6.1^{Fab} treated compared to control Fab-treated *hGP6*^{*tg*/*tg*} mice for up to 48 hours after injection (Figure 4D-E).

In conclusion, these data show that treatment of human GPVI expressing mice with Emf6.1^{Fab} results in profound and sustained inhibition of GPVI-dependent platelet activation *ex vivo.*

### 2.1.5 Emf6. 1^{Fab} is highly protective in vivo in a model of arterial thrombosis

To assess the antithrombotic potential of GPVI blockade by Emf6.1^{Fab}, a model of arterial thrombosis, in which the abdominal aorta is mechanically injured and blood flow/occlusive thrombus formation is monitored by an ultrasonic flow probe was utilized.^{43, 44} Remarkably, Emf6.1 ^{Fab}-treated mice (1 h after injection of 4 mg/kg b.w.) were strongly protected from thrombotic vessel occlusion (Figure 5A) with 91,7% (11 of 12) not forming a stable thrombus within the observation period of 30 min, whereas 100% of the controls occluded. In most of the Emf6.1^{Fab} treated mice a transient reduction of the blood flow occurred, indicative of initial aggregate formation followed by rapid disaggregation or embolization of the formed thrombus (Figure 5B).

Finally, the effect of Emf6.1^{Fab} treatment on trauma-induced hemostasis in a tail bleeding model was assessed. No significant differences in tail bleeding times were detected in Emf6.1^{Fab}-treated *hGP6*^{*tg*/*tg*} mice compared to control Fab-treated *hGP6*^{*tg*/*tg*} as well as control Fab-treated WT mice (Figure 5C). Together, these data demonstrate that treatment with Emf6.1^{Fab} potently protects *hGP6*^{*tg*/*tg*} mice from arterial thrombosis while not significantly affecting tail bleeding times, i.e. normal hemostasis.

### 2.1.6 Emf6.1 binds to the proposed GPVI-dimerization site

To determine the epitope of Emf6.1 in human GPVI, a 15-mer overlapping peptide library covering the hGPVI ectodomain (residues 24 to 267 of UniProtKB Q9HCN6-1) was synthetized and printed in microarray format. GPVI peptide microarray binding of Emf6.1-IgG was detected by HRP-labeled an anti-mouse IgG antibody. The epitope mapping revealed a clear discontinuous binding epitope located between the D2 and the transmembrane helix (Figure 6A), more precisely on residues Val201 to Glu215 and Ser246 to Pro260 (Figure 6A). The critical contribution of both binding surfaces to Emf6.1 binding was validated by neutralization of Emf6.1 using the corresponding soluble peptides (Figure 6B). Notably, the Emf6.1 binding epitope is positioned C-terminal of all so far reported GPVI epitopes (residues 58 to 187) and hence also the structurally resolved collagen binding site (Trp76, Arg38 and Glu40) (Figure 6C). The mapping therefore suggests that Emf6.1 inhibits GPVI function via a non-canonical mechanism possibly related to the dimerization of GPVI, that was structurally resolved earlier and mapped to an overlapping surface (Asp196-Thr203)¹⁸ (Figure 6C).¹⁷

Thus, peptide microarray-based mapping and neutralization⁴⁹⁻⁵¹ confirm a non-canonical discontinuous binding site of Emf6.1 in GPVI (Va!201 to Glu215 and Ser246 to Pro260), between D2 and the transmembrane helix that does not overlap with the collagen binding interface in D117 but the proposed GPVI dimerization residues (Val201-Thr203).¹⁸ Based on this finding, it can be speculated that Emf6.1^{Fab}/EMA601 may block GPVI function not primarily by interfering with ligand binding as previously described for other GPVI antagonists,^{21, 52, 53} but possibly by modulating its clustering/signaling capacity.

### 2.2 Example 2: Generation and characterization of humanized Emf6.1 Fab variants

### 2.2.1 Generation of a fully humanized Emf6. 1^{Fab} variants

Given the profound GPVI-inhibitory activity of Emf6.1^{Fab}, a set of humanized Fab fragments deriving from the Emf6.1 sequence was designed, with the aim of creating non-immunogenic monovalent antibody fragments suitable for injection in humans and with the ability to preserve specific GPVI binding activity. Emf6.1 murine variable domains were sequenced and canonical class and sub-class complementary-determining regions (CDRs) were identified.^{37, 38} The CDR residues in the murine V_{H} and V_{L} domains were identified using a combination of the IMGT and Kabat numbering systems.^{37, 38}

The murine Emf6.1 V_{L} and V_{H} domains had the sequence below, which does not include the murine signal peptide sequence:
>original murine Emf6.1 V_{L} (VLO) 
DIVMTQSQKFMSTSVGDRVSVTCKASQNVGTYVAWFQQKPGQSPKTLIYSASYRCSGVPDRFT
GSGSGTDFTLTISNVQSDDLAEYFCHQYNNYPLTFGAGTKLELR
> original murine EMF6.1 V_{H} (VHO)

QVQLKESGPGLVQPSQSLSITCTVSGFSLTSYGVTWVRQPPGKGLEWLGVIWGDGSTNYHSGL
RSRLSISRDDSKSQVFLKLNSLQTDDTATYYCAKPIYYDFDDDAMDYWGQGTSVTVSS

The CDR residues highlighted in grey were identified using the IMGT numbering system and the underlined CDR residues were identified using the Kabat numbering system. The identified CDR sequences are summarized in Table 3 below.

**Table 3: The following sequence data regarding the Emf6.1 CDRs were obtained:**

| **CDR** | **Antibody numbering system** | **Sequence¹** | **SEQ ID NO**: |
|---|---|---|---|
| CDR L1 | Kabat (+IMBT) | KASQNVGTYVA | 1 |
| | IMGT | QNVGTY | 7 |
| CDR L2 | Kabat (+IMBT) | SASYRCS | 2 |
| | Kabat short (-2 aa) | SASYR | 8 |
| | CS | SASYRXS | 9 |
| CDR L3 | Kabat = IMGT | HQYNNYPLT | 3 |
| CDR H1 | Kabat + IMGT | GFSLTSYGVT | 4 |
| | Kabat | SYGVT | 10 |
| | IMGT | GFSLTSYG | 11 |
| CDR H2 | Kabat (+IMBT) | VIWGDGSTNYHSGLRS | 5 |
| | IMGT | IWGDGST | 12 |
| | CS | VIWGXXSTNYHSGLRS | 13 |
| CDR H3 | IMGT (+Kabat) | AKPIYYDFDDDAMDY | 6 |
| | Kabat | PIYYDFDDDAMDY | 14 |

| | | | |
|---|---|---|---|
| ¹Amino acids designated "X" have the meaning as defined in the accompanying sequence listing. | | | |

The closest human germline gene V_{H}-region was Homo sapiens IGHV4-4. Data bases of Human IgG and Human IgK sequences were searched for comparison to the murine V_{H} and V_{L} domains, respectively, using BLAST search algorithms, and suitable candidates each based on a combination of framework homology, maintaining key framework residues and canonical loop structure were selected. The humanized variants were created by grafting the CDRs of the murine V_{H} and V_{L} into these acceptor frameworks.
>VL1 (based on BAH04725) 
EIVLTQSPSSLSASVGDRVTITCKASQNVGTYVAWFQQKPGKAPKSLIYSASYRCSGVPSKFSGS
GSGTDFTLTISSLQPEDFATYYCHQYNNYPLTFGQGTKLEIK
>VL2 (based on AIT38570) 
DIQMTQSPSSLSASVGDRVTITCKASQNVGTYVAWFQQRPGKAPKSLIYSASYRCSGVPSKFSG
SGSGTDFTLTINSLQPEDFATYYCHQYNNYPLTFGQGTKVEIK
>VL3 (based on AMK70118) 
DIQLTQSPSTLSASVGDRVTITCKASQNVGTYVAWYQQKPGKAPKLLIYSASYRCSGVPSRFSG
SGSGTEFTLTISSLQPDDFTTYYCHQYNNYPLTFGQGTKVEIK
>VL4 (based on APZ85375) 
DIVMTQSPATLSLSPGDRATLSCKASQNVGTYVAWYQQKPGQAPRLLIYSASYRCSGIPARFSG
SGSGTDFTLTISSLQSEDFAVYYCHQYNNYPLTFGGGTKVEIK
>VH1 (based on AAV40414) 
QVQLQESGPGLVKPSETLSLTCTASGFSLTSYGVTWIRQPPGKGLEWIGVIWGDGSTNYHSGLR
SRVTISVDTSKSQFSLKLSSVTAADTAVYYCAKPIYYDFDDDAMDYWGQGTLVTVSS
>VH2 (based on AAY23326) 
EVQLVESGPGLVKPSETLSLTCTVSGFSLTSYGVTWIRQPPGKGLEWIGVIWGDGSTNYHSGLR
SRVTISVDTSKNQFSLKLSSVTAADTAVYYCAKPIYYDFDDDAMDYWGQGTLVTVSS
>VH3 (based on >AEX28400) 
QVQLVESGGGVVQPGRSLRLSCVASGFSLTSYGVTWVRQAPGKGLEWVAVIWGDGSTNYHSG
LRSRFTISRDNSKNTMFLQMNSLRADDTAVYYCAKPIYYDFDDDAMDYWGQGTLVTVSS
>VH4based on AAD31716) 
QVQLVESGGGVVQPGRSLRLSCAASGFSLTSYGVTWVRQSPGKGLEWVAVIWGDGSTNYHSG
LRSRLTISRDNSKNTLYLEMNSLRTEDTATYYCAKPIYYDFDDDAMDYWGQGTKVTVSS
>VH5 (based on IGHV4-4)

QVQLQESGPGLVKPSETLSLTCTVSGFSLTSYGVTWIRQPPGKGLEWIGVIWGDGSTNYHSGLR
SRVTISVDTSKNQFSLKLSSVTAADTAVYYCAKPIYYDFDDDAMDYWGQGTSVTVSS

In Figure 7A and B the murine EMF6.1 V_{L} and V_{H} sequences (VL0 and VH0) have been aligned with the humanized V_{L} variants V_{H} variants, respectively. In rank order of homology, the humanized variants are VL1>VL4>VL2>VL3. In rank order of homology, the humanized variants are VH5>VH1>VH2>VH4>VH3.

Each of the V_{H} and V_{L} domains were synthesized in-frame with human IgG1 and human IgK isotype constant domain sequences, respectively, and cloned into the mammalian transient expression plasmid pETE V2. Humanized variants were checked to determine whether they had been humanized in accordance with WHO's definition of humanized antibodies: The variable domain of a humanized chain has a V region amino acid sequence which, analyzed as a whole, is closer to human than to other species (assessed using Immunogenetics Information System^{®} (IMGTO) DomainGapAlign tool, Table 4). ³⁹ The original murine antibody V_{H} and V_{L} and the humanized variant sequences were screened for MHC Class II binding peptides to determine that the humanization process had removed peptide sequences with high affinity using in silico algorithms. Moreover, Fab variants were tested for motifs prone to deamidation of asparagine to aspartic acid. No such motifs are present in the murine or humanized Fab variants. Using the Schrodinger software, the structures of the variable domain binding sites were modelled. Based on the analysis by RMSD, compared to the murine VH0 VL0 domains, the combinations of VH2:VL3 and VH1:VL3 had the lowest scores. This ranked them as having the closest structural similarity to the murine variable domains. All humanized variable domain combinations had an RMSD value of less than 2 Å and were thus predicted to be good matches to the murine VH0 VL0 domain structure (see Table 5).

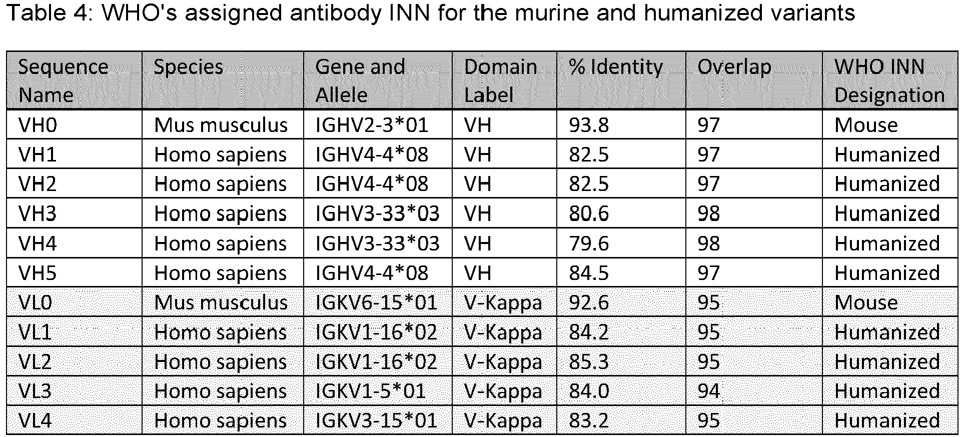

**Table 5: Predicted structure similarity using Schrodinger software.**

| **Humanized Variant compared to VH0 : VL0** | **RMSD (Å)** |
|---|---|
| VH1:VL1 | 1.278 |
| VH1:VL2 | 1.262 |
| VH1:VL3 | 1.212 |
| VH1:VL4 | 1.219 |
| VH2:VL1 | 1.226 |
| VH2:VL2 | 1.216 |
| VH2:VL3 | 1.186 |
| VH2:VL4 | 1.225 |
| VH3:VL1 | 1.450 |
| VH3:VL2 | 1.435 |
| VH3:VL3 | 1.440 |
| VH3:VL4 | 1.279 |
| VH4:VL1 | 1.507 |
| VH4:VL2 | 1.461 |
| VH4:VL3 | 1.478 |
| VH4:VL4 | 1.325 |
| VH5:VL1 | 1.282 |
| VH5:VL2 | 1.275 |
| VH5:VL3 | 1.217 |
| VH5:VL4 | 1.223 |

An N-terminal signal peptide was added to each heavy and light chain for higher levels of expression in CHO cells (heavy chain signal peptide: MGWTLVFLFLLSVTAGVH = SEQ ID NO: 63; light chain signal peptide: MVSSAQFLGLLLLCFQGTRC= SEQ ID NO:62). Each of the V_{H} domains was synthesised in-frame with the N-terminal signal peptide (SEQ ID NO:63) and a C-terminal human IgG1 isotype constant domain sequence (allotype G1m17,1) corresponding to SEQ ID NO:61. Each of the V_{L} domains was synthesised in-frame with the N-terminal signal peptide (SEQ ID NO:62) and a C-terminal human IgK isotype constant domain sequence (allotype Km3) corresponding to SEQ ID NO:60. The sequences were codon optimised (ATUM, USA) and each of the variant chains was verified by DNA sequencing analysis. The full amino acid sequence of each heavy and light chain is shown in Table 1 below.

### 2.2.2 Transient expression and purification of humanized Fab variants

The next stage was the transient transfection and expression of each of the humanized Fab fragments. There was one chimeric Fab fragment expressed for use as a positive control, having the murine variable domains and the human Ig constant domains, and 20 humanized variants having humanized variable domains and human Ig constant domains. Emf6.1 humanized variants are 50 kilodalton (kDa) monovalent Fab fragments, each composed of a heavy chain (missing the entire Fc region) and a light chain, complexed together via disulfide bonds. A mammalian expression vector (pETE V2) encoding each variant was transfected into Chinese Hamster Ovary (CHO) cells and batch cultures of each variant were grown for up to seven days.

The expressed Fab fragments were purified from cell culture supernatant via affinity chromatography. For that Fab fragment containing cell culture supernatants were clarified by centrifugation and filtration. Emf6.1 variants were purified (using state-of-the-art AKA chromatography equipment) from cell culture supernatants via affinity chromatography. Purified Fab fragment was dialyzed/buffer exchanged into phosphate buffered saline solution. quality control experiments were carried out, including determination concentration and purity of purified Fab products to ensure specified criteria were met. The purity of the Fab fragments was determined to be >95%, as judged by reducing and denaturing Sodium Dodecyl Sulfate Polyacrylamide gels. Fab concentration was determined by measuring absorbance at 280 nm and calculated using the calculated extinction coefficient where 1.0 mg/ml = A280 of 1.49 (assuming a MW = 50 kDa for a Fab fragment).

All Fab variants were successfully expressed and purified in accordance with the above criteria. From the SDS-PAGE analysis, all Fab fragments exhibited adequate levels of purity under reducing conditions. Under reducing conditions, two bands were visible at a molecular weight of about 25 and 30 kDa, respectively. Under non-reducing conditions, two bands are visible at a molecular weight of about 20 and 40 kDa. The additional bands (impurities) are likely the result of unpaired heavy and/or light chain.

### 2.2.3 Kinetic analysis of humanized Fab variants

For kinetic analysis, human GPVI-Fc fusion protein (1.2 µg was immobilized on the biosensor using suitable capture surfaces and binding of Emf6.1 variants was monitored by bio-layer interferometry (BLI) using an Octet instrument. The resulting sensograms were analyzed using the supplied software (Fortebio). For the experimental details see section 1.16 above.

**Table 6: Kinetic parameters for antibody variants and antigen interaction**

| **Antibody** | **Capture level (nm)** | **kₐ (m⁻¹s⁻¹)** | **k_{d} (s⁻¹)** | **K_{D} (M)** | **K_{D} (pM)** | **R²** | **X²** | **Mean Rₘₐₓ** |
|---|---|---|---|---|---|---|---|---|
| *HC0 LC0* | *0.731* | *4,96E*+*05* | *2.12E-04* | *4.27E-10* | *427* | *0.9957* | *0.0635* | *0.125* |
| HC1 LC1 | 0.709 | 8.04E+05 | 3.32E-04 | 4.13E-10 | 413 | 0.9945 | 0.887 | 0.130 |
| HC1 LC2 | 0.858 | 6.28E+05 | 1.23E-04 | 1.95E-10 | 195 | 0.9947 | 0.5873 | 0.159 |
| HC1 LC3 | 0.675 | 7.77E+05 | 2.21E-04 | 2.84E-10 | 284 | 0.9977 | 0.0635 | 0.133 |
| HC1 LC4 | 0.708 | 6.71E+05 | 3.33E-04 | 4.96E-10 | 496 | 0.9958 | 0.087 | 0.202 |
| HC2 LC1 | 0.707 | 7.03E+05 | 1.89E-04 | 2.69E-10 | 269 | 0.9966 | 0.863 | 0.131 |
| HC2 LC2 | 0.681 | 9.31E+05 | 1.62E-04 | 1.75E-10 | 175 | 0.9969 | 0.0776 | 0.131 |
| HC2 LC3 | 0.726 | 5.76E+05 | 1.44E-04 | 2.50E-10 | 250 | 0.9854 | 0.1888 | 0.106 |
| HC2 LC4 | 0.722 | 1.59E+06 | 1.10E-03 | 6.93E-10 | 693 | 0.9775 | 0.2152 | 0.086 |
| HC3 LC1 | 0.690 | 1.66E+06 | 5.87E-04 | 3.53E-10 | 353 | 0.9964 | 0.1022 | 0.122 |
| HC3 LC2 | 0.692 | 1.68E+06 | 3.62E-04 | 2.15E-10 | 215 | 0.9943 | 0.1319 | 0.125 |
| HC3 LC3 | 0.661 | 1.87E+06 | 5.61E-04 | 2.99E-10 | 299 | 0.9902 | 0.1019 | 0.135 |
| HC3 LC4 | 0.702 | 1.43E+06 | 6.18E-04 | 4.31E-10 | 431 | 0.9942 | 0.1227 | 0.115 |
| HC4 LC1 | 0.708 | 7.97E+05 | 3.39E-04 | 4.25E-10 | 425 | 0.9933 | 0.1647 | 0.140 |
| HC4 LC2 | 0.671 | 1.29E+06 | 5.08E-04 | 3.95E-10 | 395 | 0.9962 | 0.0559 | 0.146 |
| HC4 LC3 | 0.725 | 1.10E+06 | 6.55E-04 | 5.93E-10 | 593 | 0.9761 | 0.3477 | 0.373 |
| HC4 LC4 | 0.711 | 7.20E+05 | 4.12E-04 | 5.72E-10 | 572 | 0.9947 | 0.0667 | 0.113 |
| HC5 LC1 | 0.706 | 7.82E+05 | 2.61E-04 | 3.33E-10 | 333 | 0.9977 | 0.0314 | 0.114 |
| HC5 LC2 | 0.730 | 6.55E+05 | 2.40E-04 | 3.67E-10 | 367 | 0.9905 | 0.112 | 0.224 |
| HC5 LC3 | 0.704 | 6.86E+05 | 4.37E-04 | 6.37E-10 | 637 | 0.9934 | 0.087 | 0.186 |
| HC5 LC4 | 0.750 | 5.76E+05 | 3.19E-04 | 5.54E-10 | 554 | 0.9946 | 0.1149 | 0.378 |

| | | | | | | | | |
|---|---|---|---|---|---|---|---|---|
| R2 values indicate how well the fit and experimental data correlate and above 0.95 are considered a good fit; X2 is the sum of the squared deviation should be generally below 3. X2 is the measure of error between the experimental data and the fitted line. Italic letters: chimeric control antibody, HC0 LC0. | | | | | | | | |

Kinetic (Octet) analysis showed that in most instances the experimental data fits a 1:1 binding model. Under the experimental conditions used, a number of Fab fragments, including HC1 LC2 (KD: 195 pM), HC1 LC3 (KD: 284 pM), HC2 LC2 (KD: 175 pM) and HC2 LC3 (KD: 250 pM), exhibited affinities higher than that of the chimeric HC0 LC0 control antibody (KD: 427 pM) (see Table 6).

### 2.2.3 Further characterization of HC1 LC2 variant (EMA601)

The HC1 LC2 variant (termed EMA601) was selected for further characterization as its GPVI-blocking effect was most potent. Firstly, EMA601 at a concentration of 5 µg/mL potently inhibited aggregate formation of human platelets on collagen in the whole blood perfusion system (Figure 8A-C). Of note, also at lower concentration (1 µg/mL) EMA601 was also effective in this assay (Figure 11A).

Furthermore, EMA601 (5 µg/mL) abolished CRP- and collagen-induced aggregation of human platelets, whereas it had no effect on thrombin-induced responses (Figure 8D). This effect was also achieved at EMA601 concentrations as low as 1 µg/mL (Figure 11B) and dose response experiments further revealed that the humanized EMA601 potently inhibited activation/aggregation at high collagen concentrations (10 and 20 µg/mL). Furthermore, it was found that EMA601 (10 µg/mL) potently inhibited GPVI-dependent spreading of human platelets on fibrinogen (Figure 8E-F). Finally, EMA601 also potently inhibited platelet deposition and thrombus formation on a collagen/TF-coated surface (Figure 8G-I) and completely abolished phosphatidylserine (PS)-exposure as well as fibrin deposition (Figure 8G, J-K). Overall, these data establish EMA601 as promising lead to target human GPVI in pathological conditions.

### 2.3 Example 3: Head-to-head comparison of EMA601 and ACT017 (Glenzocimab)

### 2.3.1 In vitro binding to GPVI on platelets

To directly compare the GPVI inhibitory potencies of EMA601 and ACT017 (Glenzocimab), *in vitro* and *in vivo* studies were performed. In a first set of experiments, diluted blood of *hGP6*^{*tg*/*tg*} mice was pre-incubated with different concentrations (ranging from 50 to 0.1 µg/mL) of EMA601 or ACT017 and epitope saturation on GPVI was tested by flow cytometry. Emf3^{FITC} and JAQ1^{FITC} (each at 5 µg/mL) were used to detect epitope occupancy by EMA601 and ACT017, respectively. While Emf3^{FITC} binding was potently inhibited by EMA601, even at a concentration of 0.5 µg/mL EMA601, ACT017 had no effect on Emf3^{FITC} binding, even at very high concentrations (n=4)(Fig. 12A,B), confirming that EMA601 and ACT017 bind to different epitopes on human GPVI. In contrast, binding of JAQ1^{FITC} was inhibited by ACT017 at high concentrations (50, 20, 10 µg/mL) but the effect was gradually lost at concentrations ≤ 5 µg/mL, suggesting a comparably low affinity of ACT017 for GPVI. Of note, EMA601 inhibited JAQ1^{FITC} binding at >10-fold lower concentrations (≤ 0.5 µg/mL). Next, diluted blood of *hGP6*^{*tg*/*tg*} mice was pre-incubated with different concentrations (ranging from 50 to 0.1 µg/mL) of EMA601 or ACT017 and the bound Fabs were detected using a fluorescently labelled anti-human IgG-Fab antibody (n=4) (Fig. 12C). At all tested concentrations, EMA601 yielded significantly higher signals than ACT017 indicating a higher binding affinity of EMA601 compared to ACT017. This was further corroborated by the rapid decline in surface bound ACT017 at concentrations ≤ 2 µg/mL. Specifically, at the lowest concentrations of 0.2 and 0.1 µg/mL ACT017 was virtually absent from the platelet surface, whereas EMA601 still occupied 86.2 and 57.3% of the epitopes compared to the highest tested concentration (50 µg/mL) (Figure 12C).

### 2.3.2 Standard light-transmission aggregometry and flow adhesion assay

Next, washed human platelets were incubated with different concentrations of EMA601, ACT017 or control Fab and their response to collagen (10 µg/mL) and CRP (0.5 µg/mL) was tested by standard light-transmission aggregometry. EMA601 abolished collagen-induced aggregation at 5 µg/mL and still reduced it by -50% at 1 µg/mL. In sharp contrast, ACT017, at concentrations of up to 10 µg/mL had no significant inhibitory effect under these conditions. Of note, even at the highest tested concentration (50 µg/mL) ACT017 was unable to completely inhibit the response, demonstrating an overall limited GPVI blocking potency of ACT017 (Figure 12D). Furthermore, CRP-induced aggregation was abolished at all tested concentrations of EMA601 (5, 1 and 0.5 µg/mL), whereas ACT017 only showed partial inhibition at 1 µg/mL and no inhibitory effect at 0.5 µg/mL (Figure 12D). Next, using the flow adhesion assay the *in vitro* efficacy of EMA601 and ACT017 in reducing GPVI-dependent adhesion and aggregate formation of human platelets on collagen was tested in the whole blood flow chamber under arterial shear stress (1000 s-1). Markedly, EMA601 significantly reduced both platelet adhesion and aggregate formation at all tested concentrations, whereas ACT017 did not affect adhesion on collagen, but only reduced thrombus volume. Interestingly, EMA601 exhibited profoundly stronger reduction of the thrombus volume compared to ACT017 at all tested concentrations (Figure 12F-H).

### 2.3.3 Ex vivo testing after intravenous administration

Finally, to compare the potency of EMA601 or ACT017 to block hGPVI *in vivo, hGP6*^{*tg*/*tg*} mice received 4 mg/kg of either humanized Fab intravenously and GPVI epitope occupancy as well as platelet activation were tested after 1 hour ex *vivo.* Remarkably, ~1.8-fold higher amounts of bound EMA601 were detected compared to ACT017 (Figure 12 H). In agreement with this, CRP-induced platelet activation (JON/A-PE binding) was abolished in the blood of EMA601 treated mice, whereas no significant inhibition was detected in the blood of ACT017 treated mice (Figure 12 I).

Together, these results showed that EMA601 exerts a markedly higher GPVI inhibitory effect compared to ACT017 on human platelets *in vitro* and on *hGP6*^{*tg*/*tg*} mouse platelets *in vitro* and *in vivo.* Furthermore, the data revealed that ACT017 at concentrations of up to 50 µg/mL is unable to fully block GPVI function *in vitro* and the same is seen *in vivo* / *ex vivo* in *hGP6*^{*tg*/*tg*} at high doses (4 mg/kg).

### 2.4 Conclusions

It has been shown that the monovalent antibody Emf6.1^{Fab} and its humanized variant EMA601 potently inhibit GPVI function in human and *hGP6*^{*tg*/*tg*} mouse platelets *in vitro.* Emf6.1^{Fab} treatment of the transgenic mice resulted in sustained GPVI blockade and profound protection from occlusive arterial thrombosis, while not affecting bleeding time. Its high affinity binding to GPVI and favorable pharmacokinetics in *hGP6*^{*tg*/*tg*} mice on the one hand and its unexpected binding site on the receptor on the other hand highlight Emf6.1^{Fab}/EMA601 as novel lead to achieve potent, yet safe, GPVI inhibition in clinical settings.

The present data show a markedly prolonged persistence of Emf6.1^{Fab} on circulating platelets in *hGP6*^{*tg*/*tg*} mice, compared to what is reported for 9O12^{Fab} and ACT017 (Glenzocimab)^{23, 29, 30}, with >50% and >35% receptor occupancy at 48 h and 72 h, respectively, after injection of a single dose (4 mg/kg) which resulted in sustained inhibition of GPVI function ex *vivo.* One possible explanation for this markedly extended *in vivo* activity of Emf6.1^{Fab} may be its high affinity (kD: 0.46 nM) for human GPVI which is approximately 10-fold higher than the reported affinity of ACT017 (kD: 4.1 nM).²⁶ Of note, the affinity of Emf6.1^{Fab} was further increased ~2.3-fold during the humanization process with EMA601 displaying a kD of 0.195 nM (thus ~21-fold higher compared to ACT017). The direct head-to-head comparison of EMA601 and ACT017 confirmed that EMA601 shows >10-fold higher potency to inhibit GPVI function in human and *hGP6*^{*tg*/*tg*} platelets *in vitro* and also efficiently blocked GPVI function in *hGP6*^{*tg*/*tg*} mice, whereas no significant inhibitory effect was seen under the same conditions with ACT017 (4 mg/kg b.w.) (Fig. 12). Based on these results, it is expected that EMA601 achieves the desired levels of GPVI inhibition in humans at comparably lower doses and/or for longer time periods compared to ACT017.

The present data show that *in vitro* application of Emf6.1^{Fab} on whole recalcified blood strongly inhibits stable thrombus formation, characterized by the abolishment of PSexposure, followed by a reduction in fibrin deposition on a collagen/TF-coated surface. Emf6.1^{Fab} *in vivo* profoundly inhibited the formation of stable thrombi at sites of arterial injury, suggesting that its strong antithrombotic effect may be based on two major activities, i.e. reduced platelet activation at the exposed extracellular matrix / thrombus surface and indirectly by potent inhibition of local platelet-dependent coagulation.

Peptide microarray-based mapping and neutralization⁴⁹⁻⁵¹ confirmed a non-canonical discontinuous binding site of Emf6.1 in GPVI (Va!201 to Glu215 and Ser246 to Pro260), between D2 and the trans-membrane helix that does not overlap with the collagen binding interface in D117 but the GPVI dimerization residues (Val201-Thr203).¹⁸ Based on this finding, it is probable that Emf6.1^{Fab}/EMA601 blocks GPVI function not primarily by interfering with ligand binding as previously described for other GPVI antagonists,^{21, 52, 53} but possibly by modulating its clustering/signaling capacity. This mechanism of action could explain how Emf6.1^{Fab} profoundly inhibits GPVI-induced activation of platelets while leaving the initial adhesive functions of the receptor intact.

In conclusion, the present data provide a pre-clinical characterization of a function blocking Fab fragment with very high affinity for human GPVI, allowing sustained and safe inhibition of GPVI function in circulating platelets, presumably through a non-canonical mechanism of action.

### References

1. Alan Michelson, M.C., et al. Platelets 4th edition. (Elsevier, 2019).
2. Periayah, M.H.et al. Mechanism Action of Platelets and Crucial Blood Coagulation Pathways in Hemostasis. Int J Hematol Oncol Stem Cell Res 11, 319-327 (2017).
3. Gupta, S. et al. Hemostasis vs. homeostasis: Platelets are essential for preserving vascular barrier function in the absence of injury or inflammation. Proc Natl Acad Sci U S A 117, 24316-24325 (2020).
4. Jackson, S.P. Arterial thrombosis: insidious, unpredictable and deadly. Nat. Med. 17, 1423-1436 (2011).
5. McFadyen, J.D. et al. Current and future antiplatelet therapies: emphasis on preserving haemostasis. Nat Rev Cardiol 15, 181-191 (2018).
6. Bergmark, B.A.et al. Acute coronary syndromes. Lancet 399, 1347-1358 (2022).
7. Rodriguez, F. et al., Management of Antithrombotic Therapy after Acute Coronary Syndromes. N Engl J Med 384, 452-460 (2021).
8. Stoll, G. & Nieswandt, B. Thrombo-inflammation in acute ischaemic stroke - implications for treatment. Nat Rev Neurol 15, 473-481 (2019).
9. Nieswandt, B. et al. Long-term antithrombotic protection by in vivo depletion of platelet glycoprotein VI in mice. J Exp Med 193, 459-469 (2001).
10.Massberg, S. et al. A crucial role of glycoprotein VI for platelet recruitment to the injured arterial wall in vivo. J Exp Med 197, 41-49 (2003).
11.Kleinschnitz, C. et al. Targeting platelets in acute experimental stroke: impact of glycoprotein Ib, VI, and Ilb/Illa blockade on infarct size, functional outcome, and intracranial bleeding. Circulation 115, 2323-2330 (2007).
12. Lockyer, S. et al. GPVI-deficient mice lack collagen responses and are protected against experimentally induced pulmonary thromboembolism. Thromb Res 118, 371-380 (2006).
13. Pachel, C. et al. Inhibition of Platelet GPVI Protects Against Myocardial Ischemia-Reperfusion Injury. Arterioscler Thromb Vasc Biol 36, 629-635 (2016).
14.Nieswandt, B. & Watson, S.P. Platelet-collagen interaction: is GPVI the central receptor? Blood 102, 449-461 (2003).
15.Rayes, et al. Functional significance of the platelet immune receptors GPVI and CLEC-2. J Clin Invest 129, 12-23 (2019).
16.Moroi, M. & Jung, S.M. Platelet glycoprotein VI: its structure and function. Thromb Res 114, 221-233 (2004).
17. Feitsma, L.J. et al. Structural insights into collagen binding by platelet receptor glycoprotein VI. Blood 139, 3087-3098 (2022).
18. Horii, K.et al. Structural basis for platelet collagen responses by the immune-type receptor glycoprotein VI. Blood 108, 936-942 (2006).
19.Stegner, D. et al. FcgammaRIIB on liver sinusoidal endothelial cells is essential for antibody-induced GPVI ectodomain shedding in mice. Blood 128, 862-865 (2016).
20.Boylan, B. et al. Activation-independent, antibody-mediated removal of GPVI from circulating human platelets: development of a novel NOD/SCID mouse model to evaluate the in vivo effectiveness of anti-human platelet agents. Blood 108, 908-914 (2006).
21.Ungerer, M. et al. Novel antiplatelet drug revacept (Dimeric Glycoprotein VI-Fc) specifically and efficiently inhibited collagen-induced platelet aggregation without affecting general hemostasis in humans. Circulation 123, 1891-1899 (2011).
22. Mayer, K. et al. Efficacy and Safety of Revacept, a Novel Lesion-Directed Competitive Antagonist to Platelet Glycoprotein VI, in Patients Undergoing Elective Percutaneous Coronary Intervention for Stable Ischemic Heart Disease: The Randomized, Double-blind, Placebo-Controlled ISAR-PLASTER Phase 2 Trial. JAMA Cardiol 6, 753-761 (2021).
23.Mangin, P.H. et al. A humanized glycoprotein VI (GPVI) mouse model to assess the antithrombotic efficacies of anti-GPVI agents. J. Pharmacol. Exp. Ther. 341, 156-163 (2012).
24.Volz, J. et al. Inhibition of platelet GPVI induces intratumor hemorrhage and increases efficacy of chemotherapy in mice. Blood 133, 2696-2706 (2019).
25.Navarro, S. et al. Temporal Roles of Platelet and Coagulation Pathways in Collagen- and Tissue Factor-Induced Thrombus Formation. Int J Mol Sci 23 (2021).
26.Lebozec, K. et al. Design, development and characterization of ACT017, a humanized Fab that blocks platelet's glycoprotein VI function without causing bleeding risks. mAbs 9, 945-958 (2017).
27.ACTICOR Presentation of positive results from the ACTIMIS Phase 1b/2a study in stroke at ESOC 2022. Press release (2022).
28. Kleinschnitz, C. et al. Targeting platelets in acute experimental stroke: impact of glycoprotein Ib, VI, and Ilb/Illa blockade on infarct size, functional outcome, and intracranial bleeding. Circulation 115, 2323-2330 (2007).
29.Ohlmann, P. et al. Ex vivo inhibition of thrombus formation by an anti-glycoprotein VI Fab fragment in non-human primates without modification of glycoprotein VI expression. J. Thromb. Haemost. 6, 1003-1011 (2008).
30.Voors-Pette, C. et al. Safety and Tolerability, Pharmacokinetics, and Pharmacodynamics of ACT017, an Antiplatelet GPVI (Glycoprotein VI) Fab. Arterioscler Thromb Vasc Biol 39, 956-964 (2019).
31.Billiald, P.a.J.-P., Martine Novel anti-human GPVI antibodies and uses thereof (EP3331553) (2018).
32.Navarro, S. et al. Targeting of a Conserved Epitope in Mouse and Human GPVI Differently Affects Receptor Function. Int J Mol Sci 23 (2022).
33.Nieswandt, B. et al. Expression and function of the mouse collagen receptor glycoprotein VI is strictly dependent on its association with the FcRgamma chain. J Biol Chem 275, 23998-24002 (2000).
34. Bergmeier, W. et al. Flow cytometric detection of activated mouse integrin alphallbbeta3 with a novel monoclonal antibody. Cytometry 48, 80-86 (2002).
35.Bergmeier, W. et al. Structural and functional characterization of the mouse von Willebrand factor receptor GPlb-IX with novel monoclonal antibodies. Blood 95, 886-893 (2000).
36.Dikmans, A. et al. SC2: A Novel Process for Manufacturing Multipurpose High-Density Chemical Microarrays. QSAR & Combinatorial Science 25, 1069-1080 (2006).
37.Elemento, O. & Lefranc, M.P. IMGT/PhyloGene: an on-line tool for comparative analysis of immunoglobulin and T cell receptor genes. Dev Comp Immunol 27, 763-779 (2003).
38.Dunbar, J. & Deane, C.M. ANARCI: antigen receptor numbering and receptor classification. Bioinformatics 32, 298-300 (2016).
39.Ehrenmann, F. et al. IMGT/3Dstructure-DB and IMGT/DomainGapAlign: a database and a tool for immunoglobulins or antibodies, T cell receptors, MHC, IgSF and MhcSF. Nucleic Acids Res 38, D301-307 (2010).
40.Slater, A. et al. Does fibrin(ogen) bind to monomeric or dimeric GPVI, or not at all? Platelets 30, 281-289 (2019).
41.Xu, R.G. et al. GPVI (Glycoprotein VI) Interaction With Fibrinogen Is Mediated by Avidity and the Fibrinogen alphaC-Region. Arterioscler Thromb Vasc Biol 41, 1092-1104 (2021).
42.Mangin, P.H. et al. Immobilized fibrinogen activates human platelets through glycoprotein VI. Haematologica 103, 898-907 (2018).
43.Bender, M., Hagedorn, I. & Nieswandt, B. Genetic and antibody-induced glycoprotein VI deficiency equally protects mice from mechanically and FeCI(3) -induced thrombosis. J Thromb Haemost 9, 1423-1426 (2011).
44.Morowski, M. et al. Only severe thrombocytopenia results in bleeding and defective thrombus formation in mice. Blood 121, 4938-4947 (2013).
45.Massberg, S. et al. Soluble glycoprotein VI dimer inhibits platelet adhesion and aggregation to the injured vessel wall in vivo. FASEB J 18, 397-399 (2004).
46.Schulz, C. et al. Platelet GPVI binds to collagenous structures in the core region of human atheromatous plaque and is critical for atheroprogression in vivo. Basic Res Cardiol 103, 356-367 (2008).
47.Gruner, S. et al. Relative antithrombotic effect of soluble GPVI dimer compared with anti-GPVI antibodies in mice. Blood 105, 1492-1499 (2005).
48.Dutting, S., Bender, M. & Nieswandt, B. Platelet GPVI: a target for antithrombotic therapy?! Trends Pharmacol Sci 33, 583-590 (2012).
49.Henkel, S., Wellhausen, R., Woitalla, D., Marcus, K. & May, C. Epitope Mapping Using Peptide Microarray in Autoantibody Profiling. Methods Mol Biol 1368, 209-224 (2016).
50.Andresen, H. et al. Development of peptide microarrays for epitope mapping of antibodies against the human TSH receptor. J Immunol Methods 315, 11-18 (2006).
51.Talucci I., M.H. Peptide Microarrays for Studying Autoantibodies in Neurological Disease. (2022).
52. Taylor, L. et al. Discovery of novel GPVI receptor antagonists by structure-based repurposing. PLoS One 9, e101209 (2014).
53.Lecut, C. et al. Identification of residues within human glycoprotein VI involved in the binding to collagen: evidence for the existence of distinct binding sites. J Biol Chem 279, 52293-52299 (2004).
54. WO2019007959 A1
55. EP1224942 A1
56.EP1228768A1
57.WO 2006118350 A1
58. WO 2011073954 A2
59.WO2006117910 A1
60.WO2008049928 A1
61.WO2017021539 A2
62.WO2005111083 A2

## Claims

1. An antibody or a functional fragment thereof capable of binding to human glycoprotein VI (GPVI), wherein the antibody or functional fragment comprises (i) a V_{L} domain comprising a CDR1 region having an amino acid sequence in accordance with the amino acid sequence as shown in SEQ ID NO:1, a CDR2 region having an amino acid sequence in accordance with the amino acid sequence as shown in SEQ ID NO:2 or SEQ ID NO:9, and a CDR3 region having an amino acid sequence in accordance with the amino acid sequence as shown in SEQ ID NO:3, and (ii) a V_{H} domain comprising a CDR1 region having an amino acid sequence in accordance with the amino acid sequence as shown in SEQ ID NO:4, a CDR2 region having an amino acid sequence in accordance with the amino acid sequence as shown in SEQ ID NO:5, and a CDR3 region having an amino acid sequence in accordance with the amino acid sequence as shown in SEQ ID NO:6.

2. The antibody or functional fragment of claim 1, which is monovalent.

3. The antibody or functional fragment of claim 1 or 2, wherein the antibody or functional fragment binds to human GPVI with a dissociation equilibrium constant (K_{D}) of less than 700 pM, preferably less than 300 pM, more preferably less than 200 pM.

4. The antibody or functional fragment of any one of the preceding claims, wherein the antibody or functional fragment comprises a V_{L} domain comprising an amino acid sequence having a sequence identity of at least 90 % to a sequence selected from the group consisting of SEQ ID NO:16, 17, 18, 19, 26, 27, 28, 29, 30, 31, 32, 33, 34, and 35; and/or a V_{H} domain comprising an amino acid sequence having a sequence identity of at least 90 % to a sequence selected from the group consisting of SEQ ID NO:21, 22, 23, 24 and 25.

5. The antibody or functional fragment of any one of the preceding claims, wherein the antibody or functional fragment comprises a V_{L} domain comprising an amino acid sequence as shown in SEQ ID NO:16, 17, 18, 19, 26, 27, 28, 29, 30, 31, 32, 33, 34, or 35; and/or a V_{H} domain comprising an amino acid sequence as shown in SEQ ID NO:21, 22, 23, 24 or 25.

6. The functional fragment of any one of the preceding claims, which is a fragment antigen-binding (Fab), a F(ab'), an Fv, a monovalent IgG, a disulfide-linked variable fragment (dsFv), or a single-chain variable fragment (scFv).

7. The antibody or functional fragment of any one of the preceding claims, wherein said antibody or functional fragment comprises a light chain comprising an N-terminal light chain signal peptide having an amino acid sequence in accordance with the sequence as shown in SEQ ID NO:62 and/or a constant domain having an amino acid sequence as shown in SEQ ID NO:60; and/or a heavy chain comprising an N-terminal light chain signal peptide having an amino acid sequence in accordance with the sequence as shown in SEQ ID NO:63 and/or a constant domain having an amino acid sequence as shown in SEQ ID NO:61.

8. The functional fragment of claim 7, wherein the functional fragment is a Fab, comprising, or consisting of, a light chain having an amino acid sequence as shown in one of SEQ ID NO: 38, 39, 40, 41, 55, 56, 57 or 58, and a heavy chain having an amino acid sequence as shown in one of SEQ ID NO:44, 45, 46, 47 or 48.

9. The functional fragment of any one of the preceding claims, wherein the functional fragment is a Fab consisting of a pair of light chain and heavy chain, having the amino acid sequence as shown SEQ ID NO:39 and 44, 39 and 45, 40 and 45, 56 and 44, 56 and 45, or 57 and 45, preferably SEQ ID NO:39 and 44, or 56 and 44.

10. The antibody or functional fragment of any one of the preceding claims, which is capable of binding human GPVI at a binding epitope corresponding to amino acids V178 to E192 and/or S223 to P237 of SEQ ID NO:36.

11. A nucleic acid encoding the antibody or functional fragment of any one of the preceding claims.

12. A cell comprising the nucleic acid of claim 11.

13. A method of preparing the antibody or functional fragment of any one of claims 1 to 10, comprising culturing the cell of claim 12 in a medium under conditions that allow expression of the nucleic acid encoding the antibody or functional fragment, and recovering the antibody or functional fragment from the cells or from the medium.

14. A pharmaceutical composition comprising the antibody or functional fragment of any one of claims 1 to 10, and optionally a pharmaceutically acceptable carrier and/or excipient.

15. The antibody or functional fragment as defined in any one of claims 1 to 10, or the pharmaceutical composition of claim 14, for use in a method of treating or preventing a GPVI-related condition in a subject; optionally wherein the GPVI-related condition is a thrombo-inflammatory disease; a cardiovascular disease, preferably selected from thrombosis and thrombotic disorders, including arterial thrombosis, venous thrombosis, atherothrombosis, stent thrombosis, venous thromboembolism diseases, thrombotic microangiopathies, cancer-associated thrombosis, immunothrombosis and thrombosis associated to infection, restenosis, acute coronary syndrome, ischemic cerebrovascular accidents, cerebral vascular diseases, vascular purpura, coronary artery diseases and cerebral artery diseases, ischemic events, acute coronary artery syndrome, myocardial infarction, stroke, percutaneous coronary intervention, ischemic restenosis, acute ischemia, chronic ischemia, diseases of the aorta and its branches, peripheral artery disease, acute phlebitis, pulmonary embolism; inflammation, preferably sustained or prolonged inflammation associated with infection, arthritis, fibrosis, acute respiratory distress syndrome (ARDS), ischemia-reperfusion injury (IRI) of various organs (liver, colon, etc.), peripheral vascular disease, antiphospholipid syndrome (APS), deep vein thrombosis, thrombophlebitis & vasculitis, transfusion-related acute lung injury (TRALI), transplant rejection, pre-eclampsia, severe burns, atherosclerosis, hypertension, antiphospholipid syndrome, preeclampsia, sickle cell disease, bacterial and viral infection ischemic restenosis, sepsis, major trauma, autoimmune diseases, and disorders in which platelets modulate cell functions including, without limitation, cancer cells proliferation and/or dissemination; cancer, preferably skin cancer, colon cancer, breast cancer, ovarian cancer, lung cancer, or metastatic cancer; and/or a disorder associated with abnormal or aberrant megakaryocyte and/or platelet proliferation, differentiation, morphology, migration, aggregation, degranulation and/or function.
